# EUROPEAN PATENT APPLICATION

(11) **EP 4 309 738 A2**
(43) Date of publication of application: **24.01.2024**
(21) Application number: 23199696.8
(22) Date of filing: 05.10.2016
(51) Int. Cl.: A61P 35/00

(54) **METHOD OF TREATING MEDULLOBLASTOMA WITH AN EZH2 INHIBITOR**

(30) Priority: 06.10.2015 US 201562238074 P; 24.02.2016 US 201662299312 P
(62) Divisional of application: 16854241.3
(71) Applicant: Epizyme Inc, Cambridge, MA 02139 (US); The Regents of the University of Colorado, a body corporate, Denver, CO 80203 (US)
(72) Inventor: KEILHACK, Heike, Belmont, 02478 (US); WATERS, Nigel, J., Belmont, 02478 (US); VIBHAKAR, Rajeev, Denver, 9187 (US)
(74) Representative: Russell, Tim

(57) **Abstract**

The disclosure provides a method of treating a medulloblastoma in a subject in need thereof comprising administering to the subject a therapeutically-effective amount of an enhancer of a zeste homolog 2 (EZH2) inhibitor. In a preferred embodiment of this method, the subject is pediatric and the EZH2 inhibitor is Tazemetostat.

## Description

### RELATED APPLICATIONS

This application claims priority to, and the benefit of U.S. Provisional Application Nos. 62/238,074 filed October 6, 2015, and 62/299,312 filed February 24, 2016, the contents of each of which are incorporated herein by reference in their entireties.

### FIELD OF THE DISCLOSURE

The disclosure is directed to the fields of small molecule therapies, cancer, and methods of treating rare cancer types.

### BACKGROUND

There is a long-felt yet unmet need for effective treatments for certain cancers caused by genetic alterations or loss of function of subunits of the SWI/SNF chromatin remodeling complex that result in EZH2-dependent oncogenesis.

### SUMMARY

The disclosure provides a method of treating a medulloblastoma in a subject in need thereof comprising administering to the subject a therapeutically-effective amount of an enhancer of a zeste homolog 2 (EZH2) inhibitor. Methods of treating medulloblastoma of the disclosure may comprise preventing and/or inhibiting proliferation of a medulloblastoma cell.

In certain embodiments of the methods of the disclosure, the EZH2 inhibitor comprises 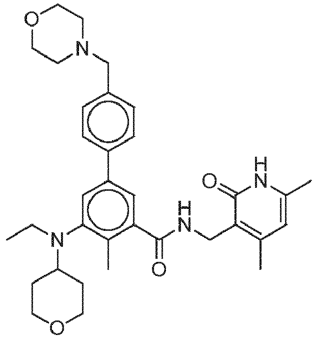 (Compound A or tazemetostat), or a pharmaceutically-acceptable salt thereof.

In certain embodiments of the methods of the disclosure, the EZH2 inhibitor comprises 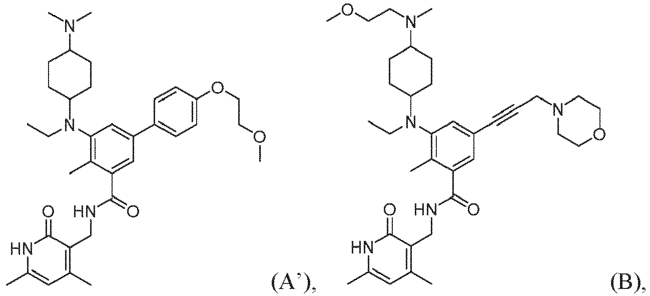 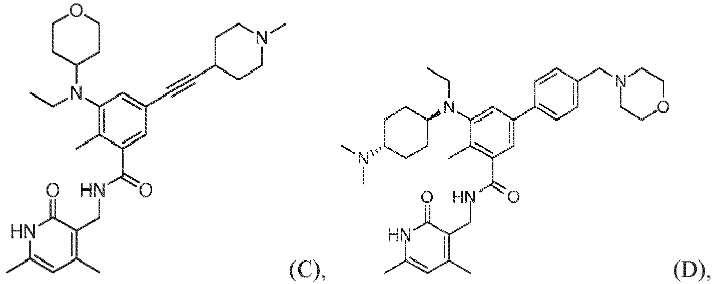 a stereoisomer, a pharmaceutically acceptable salt and/or a solvate thereof.

In certain embodiments of the methods of the disclosure, the EZH2 inhibitor comprises 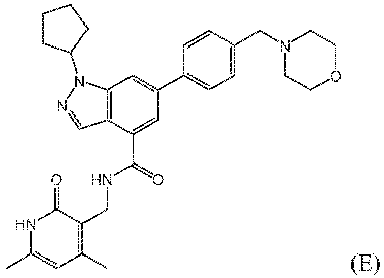 or a pharmaceutically acceptable salt thereof.

In certain embodiments of the methods of the disclosure, the EZH2 inhibitor comprises 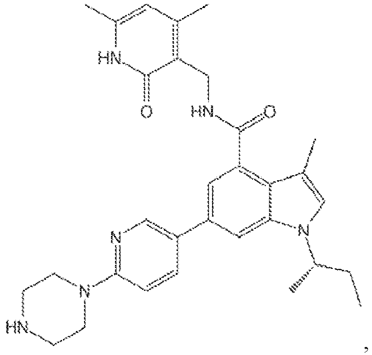 a stereoisomer, a pharmaceutically acceptable salt and/or a solvate thereof.

In certain embodiments of the methods of the disclosure, the EZH2 inhibitor comprises 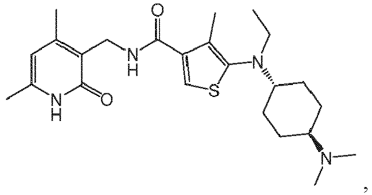 a stereoisomer, a pharmaceutically acceptable salt and/or a solvate thereof.

In certain embodiments of the methods of the disclosure, the EZH2 inhibitor comprises 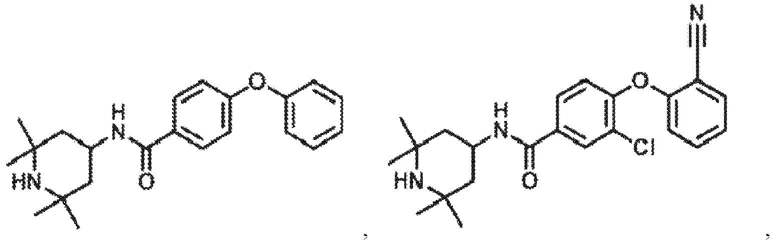 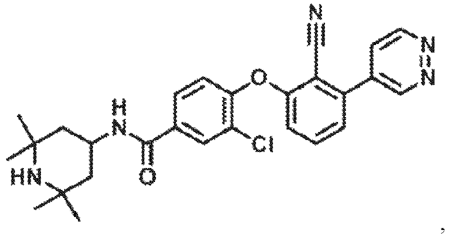 a stereoisomer, a pharmaceutically acceptable salt and/or a solvate thereof.

EZH2 inhibitors of the disclosure may be administered orally. For example, the EZH2 inhibitor may be formulated as an oral tablet or suspension.

EZH2 inhibitors of the disclosure may be formulated for administration to cerebral spinal fluid (CSF) by any route. Exemplary routes of administration to the CSF include, but are not limited to, an intraspinal, an intracranial, an intrathecal or an intranasal route.

In certain embodiments of the methods of the disclosure, including, but not limited to, those embodiments wherein the EZH2 inhibitor is formulated as an oral tablet, EZH2 inhibitors of the disclosure may be administered at a dose of between 10 mg/kg/day and 1600 mg/kg/day. EZH2 inhibitors of the disclosure may be administered at a dose of about 100, 200, 400, 800, or 1600 mg. EZH2 inhibitors of the disclosure may be administered at a dose of about 800 mg. EZH2 inhibitors of the disclosure may be administered once or twice per day (BID). For example, EZH2 inhibitors of the disclosure may be administered at a dose of between 10 mg/kg/day and 1600 mg/kg/day BID. EZH2 inhibitors of the disclosure may be administered at a dose of 800 mg BID.

In certain embodiments of the methods of the disclosure, including, but not limited to, those embodiments wherein the EZH2 inhibitor is formulated as an oral suspension and/or formulated to administration to the CSF by any route, EZH2 inhibitors of the disclosure may be administered at a dose of 50%, 60%, 70%, 80%, 90%, or any percentage in between of a value of an area under the curve (AUC) of a steady state plasma and/or CSF concentration (AUC_{SS}) of an EZH2 inhibitor, wherein the AUC_{SS} is determined following administration of the EZH2 inhibitor to an adult subject at a dose of between 10 mg/kg/day and 1600 mg/kg/day BID.

In certain embodiments of the methods of the disclosure, including, but not limited to, those embodiments wherein the EZH2 inhibitor is formulated as an oral suspension and/or formulated to administration to the CSF by any route, EZH2 inhibitors of the disclosure may be administered at a dose of between 230 mg/m² and 600 mg/m², inclusive of the endpoints. EZH2 inhibitors of the disclosure may be administered at a dose of between 300 mg/m² and 600 mg/m². EZH2 inhibitors of the disclosure may be administered at a dose of between 230 mg/m² and 305 mg/m², inclusive of the endpoints. EZH2 inhibitors of the disclosure may be administered at a dose of 240 mg/m². EZH2 inhibitors of the disclosure may be administered at a dose of 300 mg/m². EZH2 inhibitors of the disclosure may be administered once or twice per day (BID). For example, EZH2 inhibitors of the disclosure may be administered at a dose of between 230 mg/m² and 600 mg/m² BID, inclusive of the endpoints.

For example, an EZH2 inhibitor of the disclosure may be administered at a dose of about 60% of the area under the curve (AUC) at steady state (ACU_{SS}) following administration of 1600 mg twice a day to an adult subject. Accordingly, an EZH2 inhibitor of the disclosure administered at a dose of about 60% of the area under the curve (AUC) at steady state (ACU_{SS}) following administration of 1600 mg twice a day to an adult subject, is administered at a dose of about 600 mg/m² per day or at least 600 mg/m² per day. In certain aspects of this example, the subject treated with the EZH2 inhibitor is a pediatric subject.

For example, an EZH2 inhibitor of the disclosure may be administered at a dose of about 80% of the area under the curve (AUC) at steady state (ACU_{SS}) following administration of 800 mg twice a day to an adult subject. Accordingly, an EZH2 inhibitor of the disclosure administered at a dose of about 80% of the area under the curve (AUC) at steady state (ACU_{SS}) following administration of 800 mg twice a day to an adult subject, is administered at a dose of about 390 mg/m² per day or at least 390 mg/m² per day. In certain aspects of this example, the subject treated with the EZH2 inhibitor is a pediatric subject.

Subjects of the disclosure may be pediatric subjects. For example, a pediatric subject of the disclosure may be between 6 months and 21 years of age, inclusive of the endpoints. A pediatric subject of the disclosure may be between 1 year and 18 years of age, inclusive of the endpoints. A pediatric subject of the disclosure may be 10 years of age or less. A pediatric subject of the disclosure may be 5 years of age or less. A pediatric subject of the disclosure may be between 6 months and 1 year of age, inclusive of the endpoints.

The disclosure provides a method of treating medulloblastoma in a subject in need thereof comprising administering to the subject a therapeutically-effective amount of tazemetostat, wherein the therapeutically effective amount is at least 300 mg/m² twice per day (BID), and wherein the subject is between 6 months and 21 years of age, inclusive of the endpoints.

### BRIEF DESCRIPTION OF THE DRAWINGS

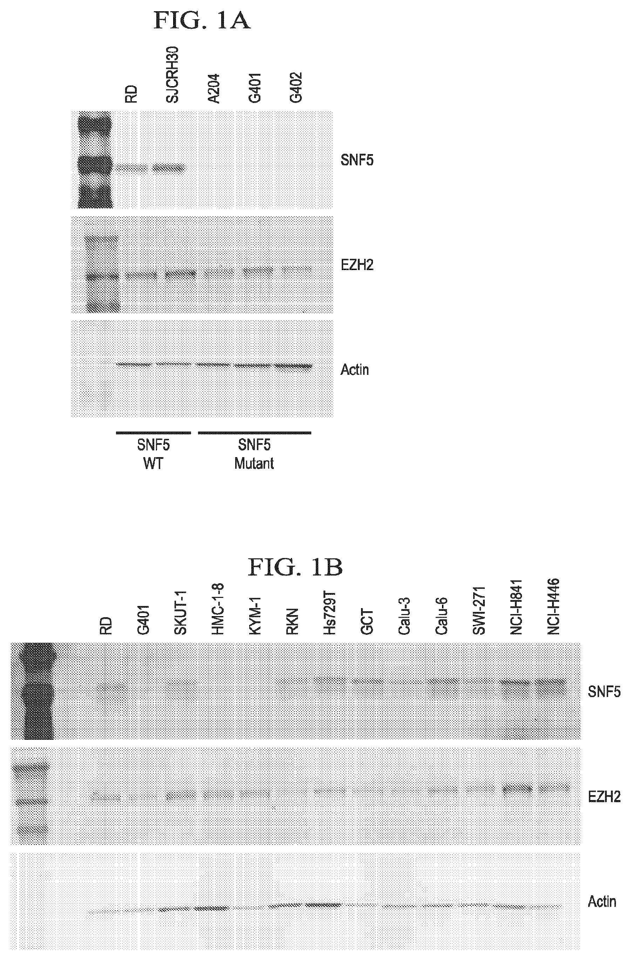

Figures 1A and 1B are a series of Western blot analyses of cell lines with wild type (RD and SJCRH30) and mutant SNF5.

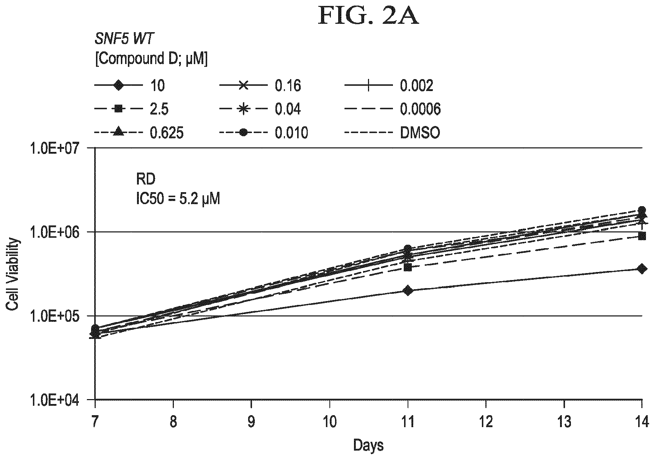

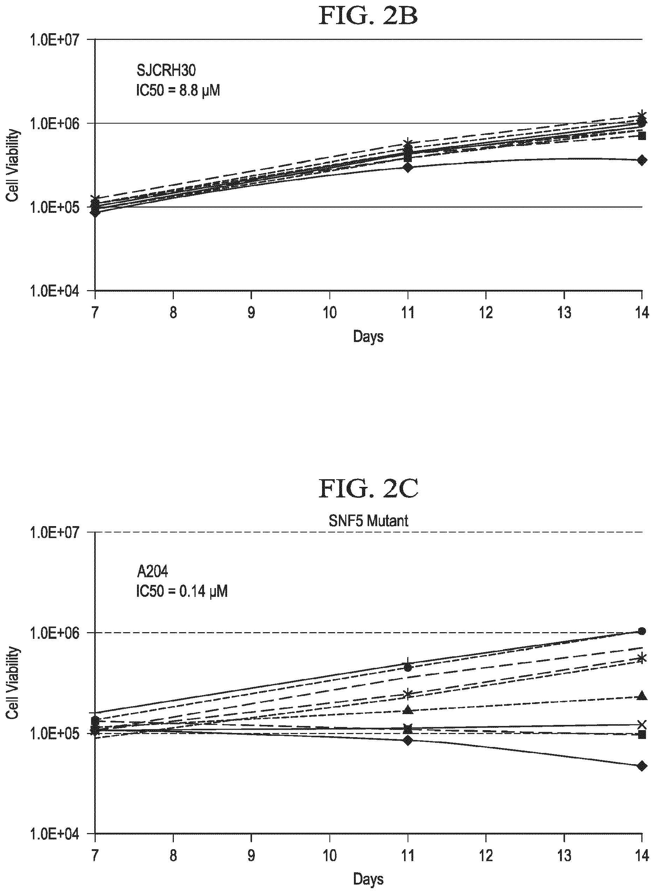

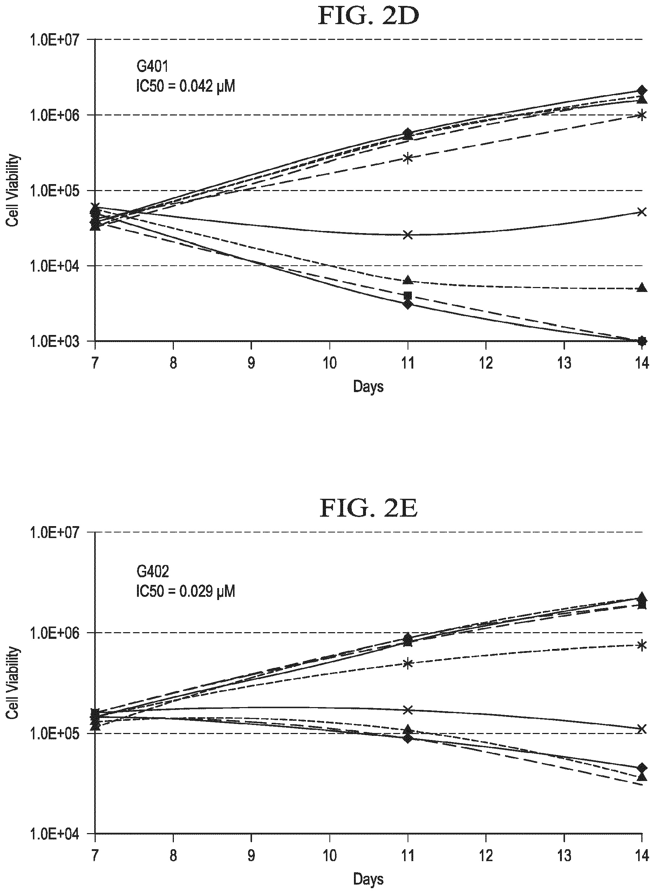

Figures 2A-2E are a series of graphs establishing that SNF5 mutant cell lines A204 (C), G401 (D) and G402 (E) selectively respond to EZH2 compound (Compound D) compared to wild type cell lines RD (A) and SJCRH30 (B).

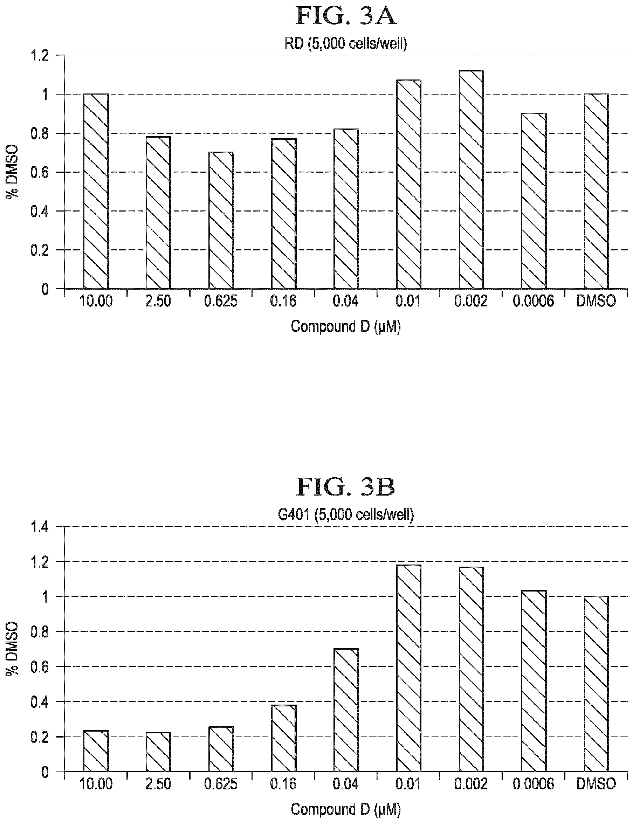

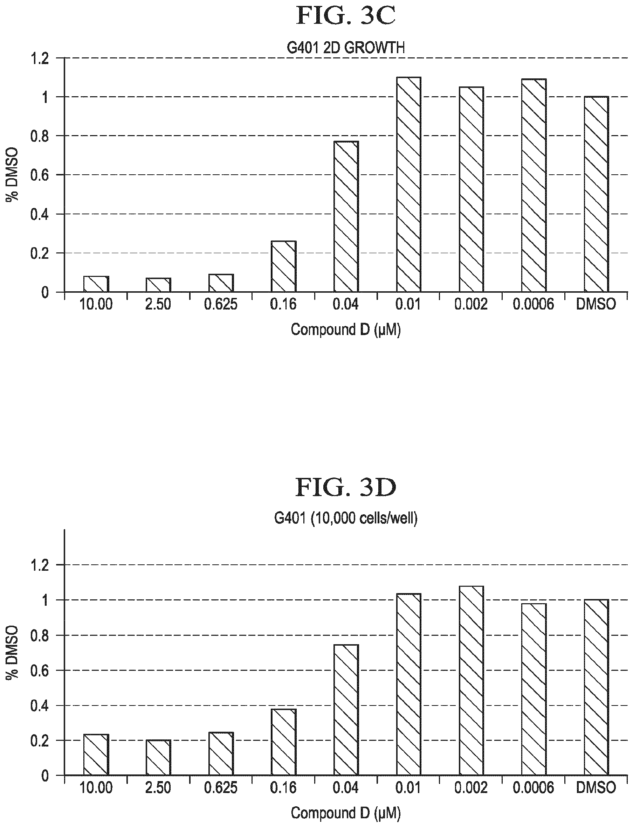

Figures 3A-3D are a series of bar graphs showing that G401 SNF mutant cell line is responding to Compound D after 7 days in soft agar compared to wild type cells RD. A shows cell line RD (5,000 cells/well). B shows G401 cells (5,000 cells/well). C shows G401 cells in 2D growth. D shows G401 cells (10,000 cells/well).

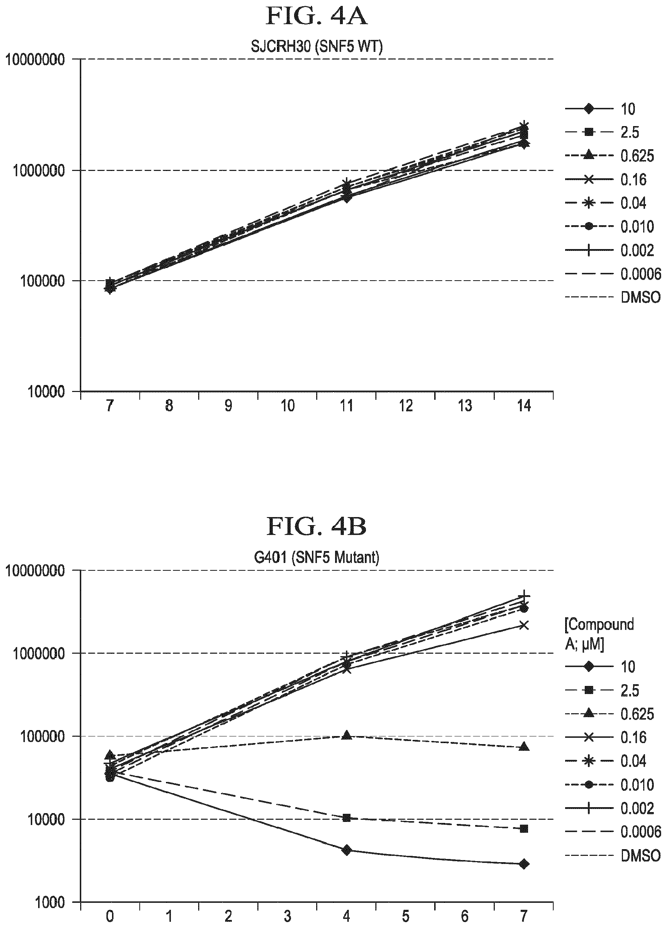

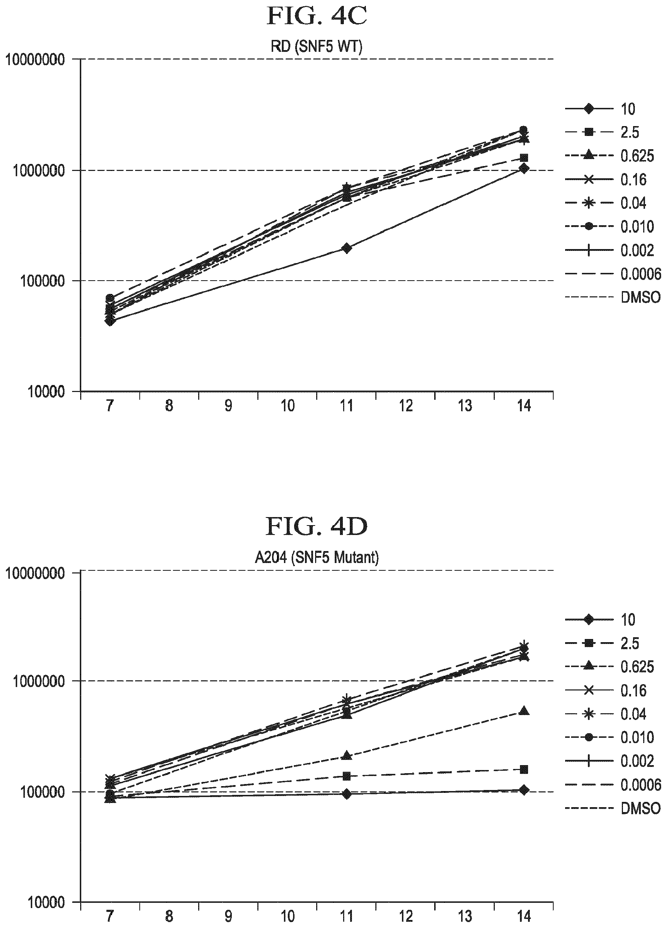

Figures 4A-4D are four graphs showing that G401 SNF5 mutant cell line is sensitive to Compound A in vitro. Wild type cell line SJCRH30 (A) and RD (C) and SNF5 mutant cell line G401 (B) and A204 (D) were pretreated for 7 days with indicated concentrations of Compound A and replated on day 0. Cell viability was determined by CellTiter-Glo^{®} Luminescent Cell Viability Assay.

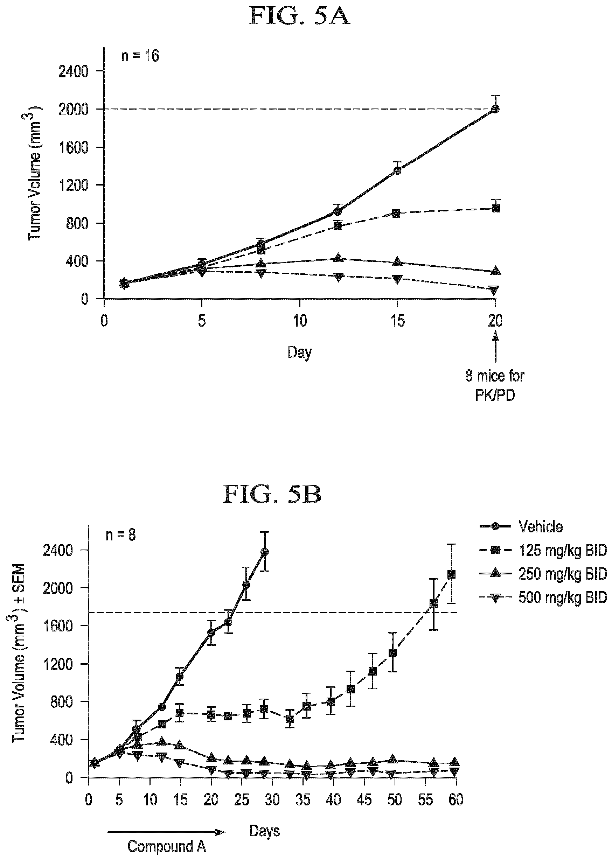

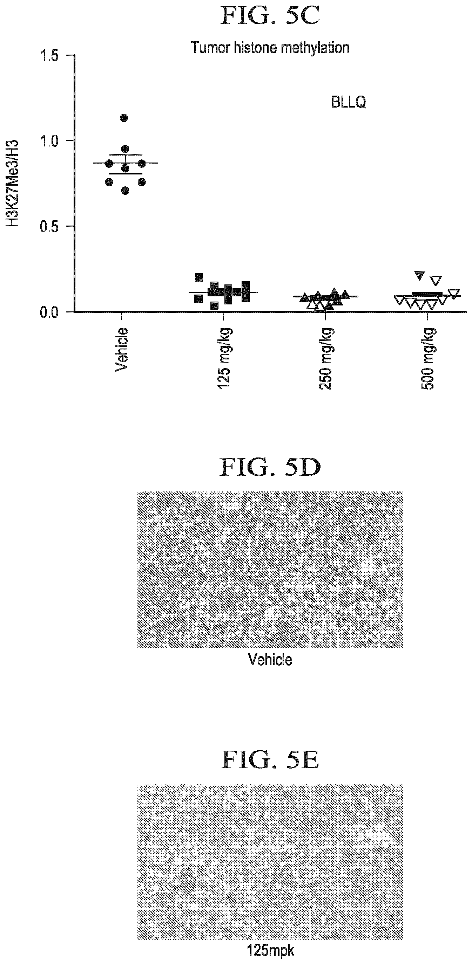

Figures 5A-5E are a series of graphs showing durable regressions in G401 xenografts (malignant rhabdoid tumor model) with Compound A treatment. (A) Tumor regressions induced by Compound A at the indicated doses. (B) Tumor regressions induced by twice daily administration of Compound A at the indicated doses. Data represent the mean values ± SEM (n=8). Compound administration was stopped on day 28. (C) EZH2 target inhibition in G401 xenograft tumor tissue collected from a parallel cohort of mice on day 21. Each point shows the ratio of H3K27Me3 to total H3. Horizontal lines represent group mean values. BLLQ = below lower limit of quantification. (D, E) Immunohistochemical staining of tumor histone methylation of tumor samples from the vehicle treated (D) and Compound A treated (E) (at 125 mg/kg) mice.

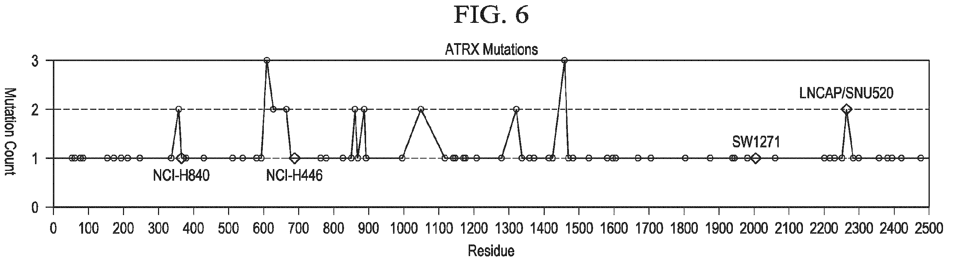

Figure 6 is a graph showing the locations of ATRX mutations identified in SCLC cell lines.

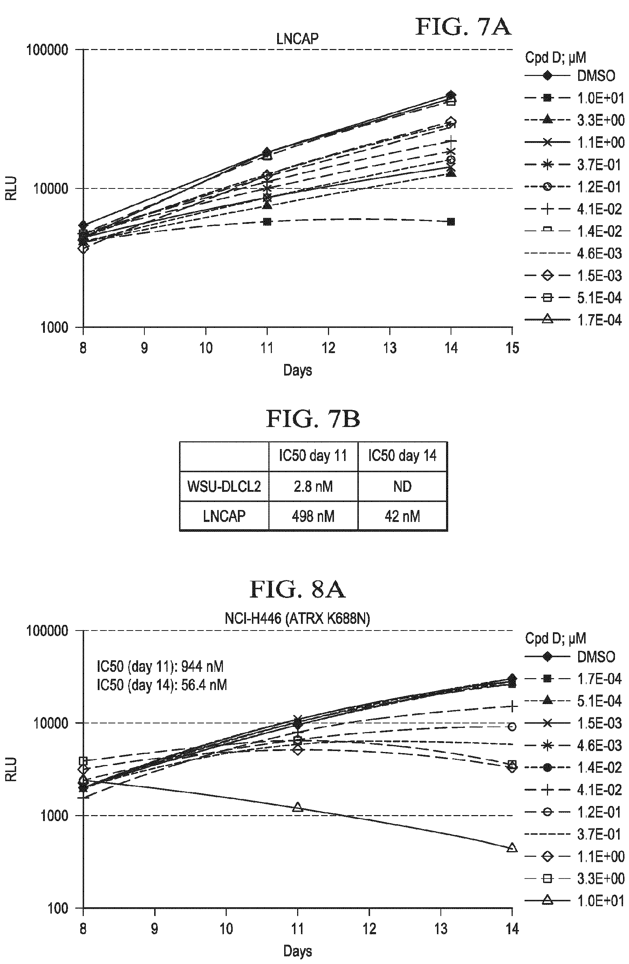

Figure 7A is a graph showing that LNCAP prostate cancer cells display dose-dependent cell growth inhibition with Compound D treatment *in vitro.*
Figure 7B is a graph showing IC50 value of Compound D at day 11 and day 14 for WSU-DLCL2 and LNCAP cells.

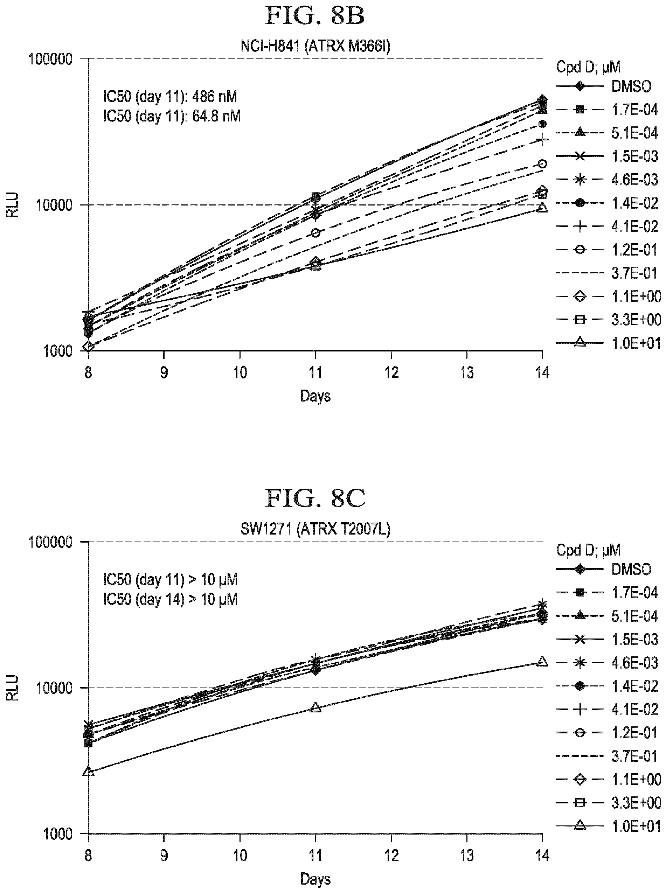

Figures 8A-8C are three graphs establishing that ATRX mutant SCLC lines NCI-H446 (A), SW1271 (B) and NCI-H841 (C) are responding to Compound D.

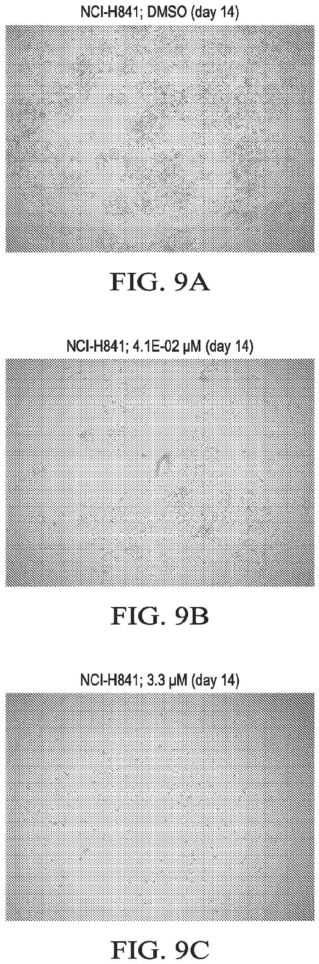

Figures 9A-9C are three microscopy images showing that SCLC line NCI-H841 changes morphology after treatment with vehicle (A) or Compound D at concentration of 4.1E-02 uM (B) or 3.3 uM (C).

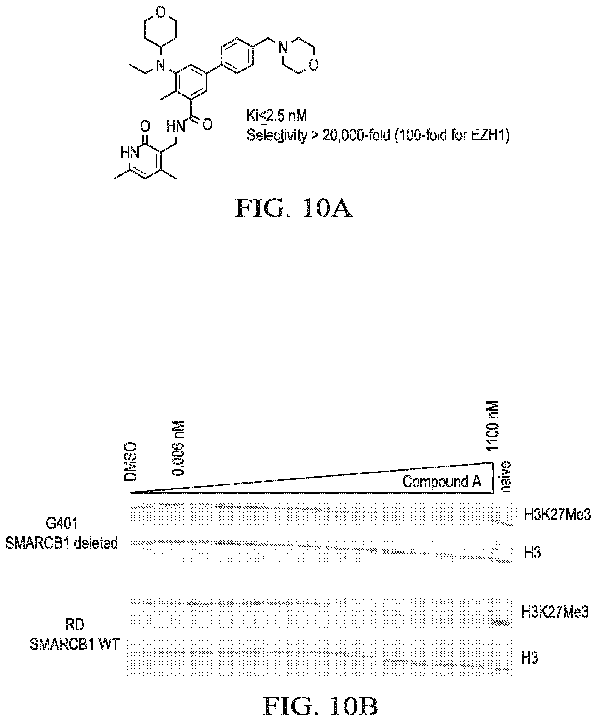

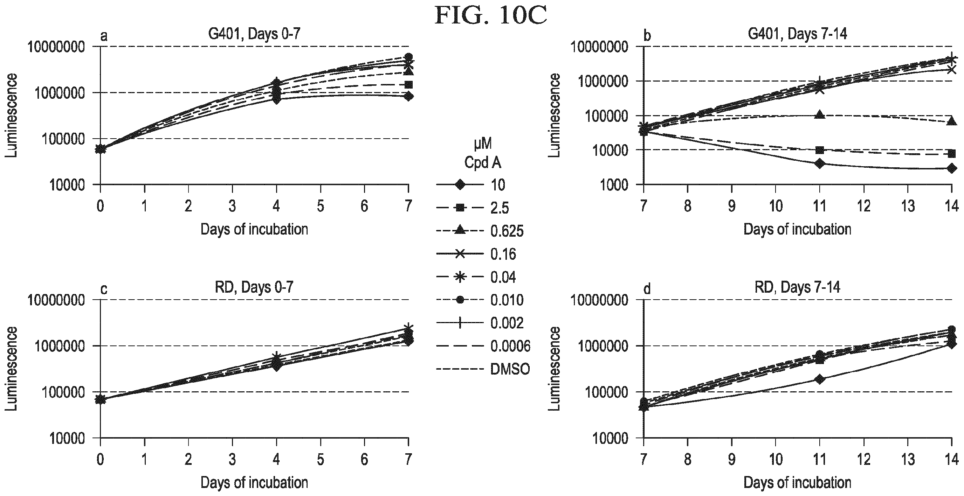

Figures 10A-10C are a series of graphs showing effects of Compound A on cellular global histone methylation and cell viability. (A) Chemical structure of Compound A (or tazemetostat). (B) Concentration-dependent inhibition of cellular H3K27Me3 levels in G401 and RD cells. (C) Selective inhibition of proliferation of *SMARCB1*-deleted G401 cells by Compound A in vitro (measured by ATP content). G401 (panels a and b) and RD cells (panels c and d) were re-plated at the original seeding densities on day 7. Each point represents the mean for each concentration (n=3).

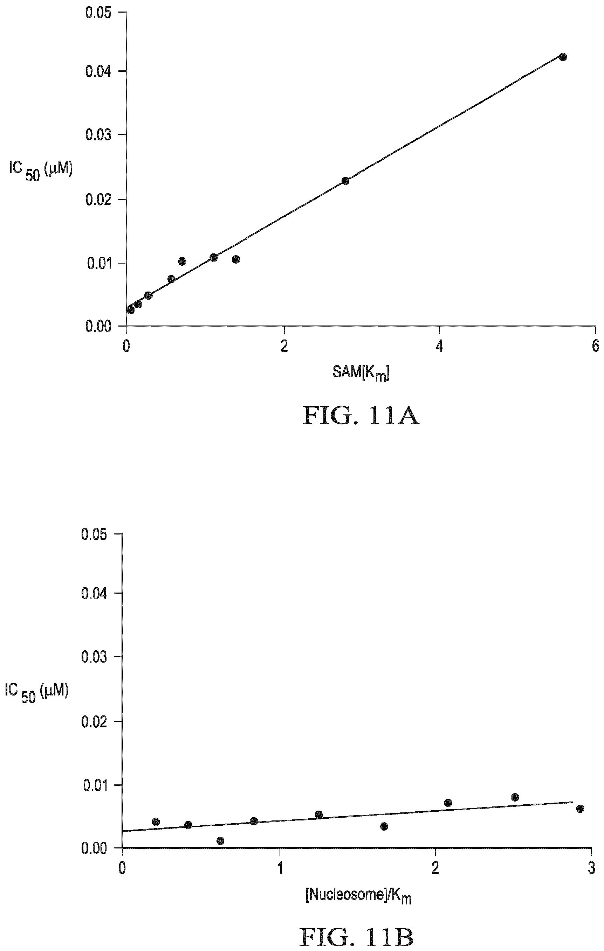

Figures 11A and 11B are a series of graphs showing biochemical mechanism of action studies. The IC₅₀ value of Compound A increases with increasing SAM concentration (A) and is minimally affected by increasing oligonucleosome concentration (B), indicating SAM-competitive and nucleosome-noncompetitive mechanism of action.

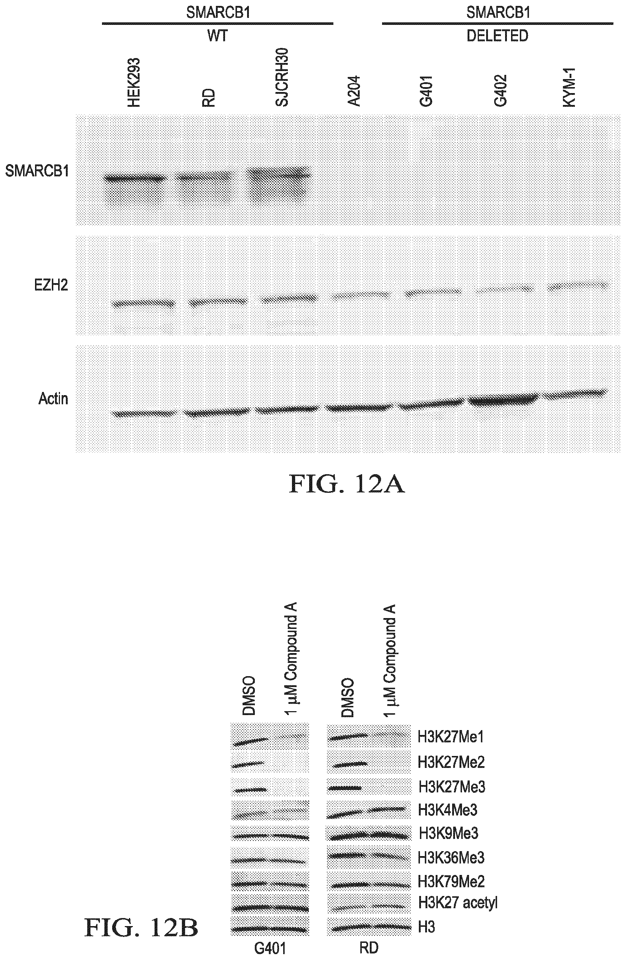

Figures 12A and 12B are a series of panels demonstrating verification of SMARCB1 and EZH2 expression in cell lines and specificity of Compound A for inhibition of cellular histone methylation. (A) Cell lysates were analyzed by immunoblot with antibodies specific to SMARCB1, EZH2 and Actin (loading control). (B) Selective inhibition of cellular H3K27 methylation in G401 and RD cells. Cells were incubated with Compound A for 4 days, and acid-extracted histones were analyzed by immunoblot.

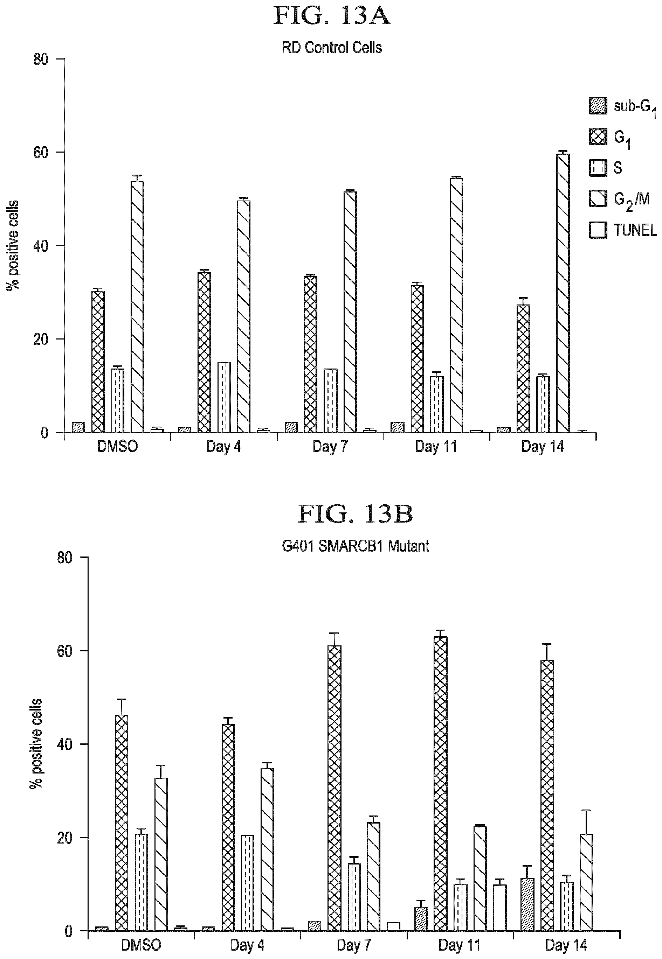

Figures 13A and 13B are a series of bar graphs demonstrating that Compound A induces G₁ arrest and apoptosis in *SMARCB1-*deleted MRT cells. Cell cycle analysis (by flow cytometry) and determination of apoptosis (by TUNEL assay) in RD (panel A) or G401 cells (panel B) during incubation with either vehicle or 1 µM Compound A for up to 14 days. G₁ arrest was observed as of day 7 and apoptosis was induced as of day 11. Data are represented as mean values ± SEM (n=2). The DMSO control values shown are the average ± SEM from each time point. Cells were split and re-plated on days 4, 7 and 11 at the original seeding density.

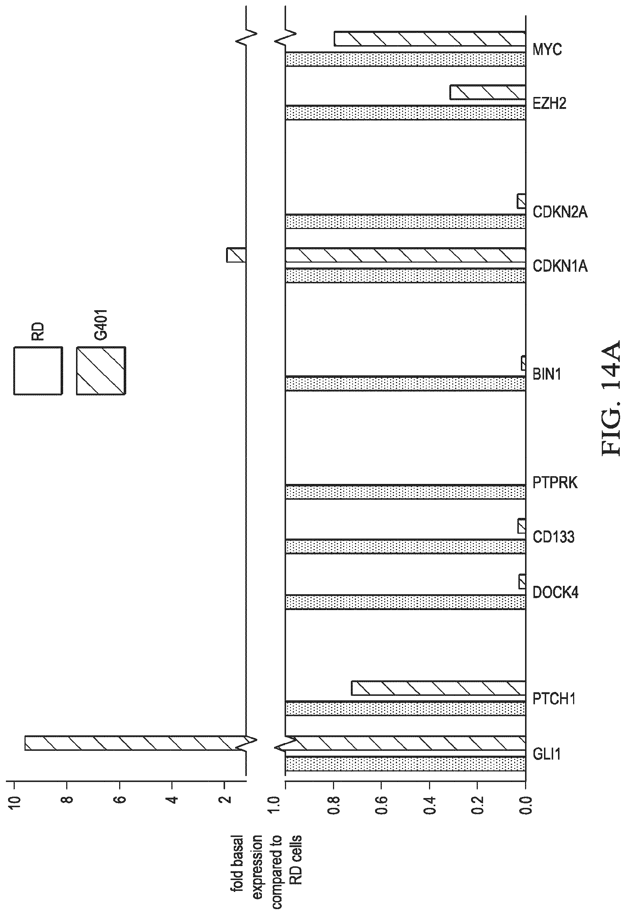

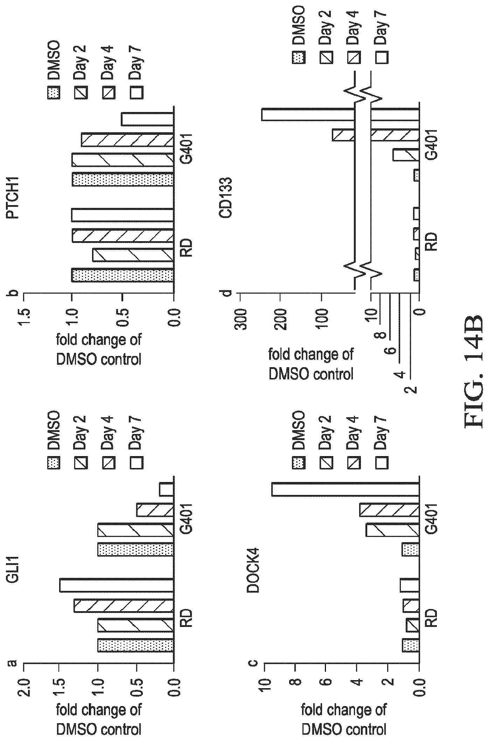

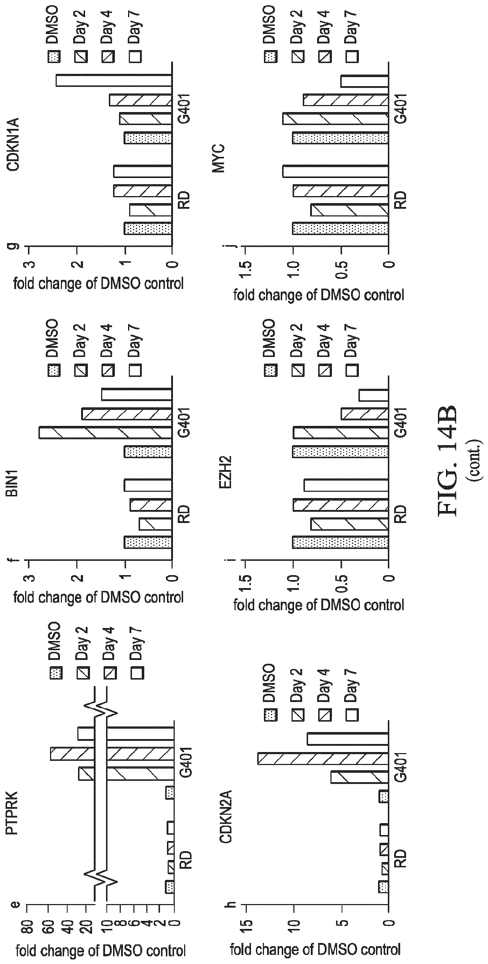

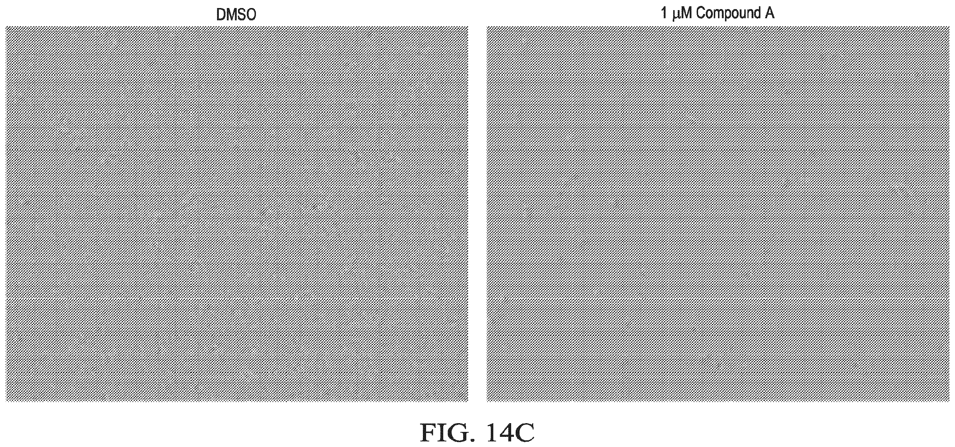

Figures 14A-14C are a series of graphs showing that Compound A induces changes in expression of SMARCB1 regulated genes and cell morphology. (A) Basal expression of SMARCB1 regulated genes in G401 *SMARCB1*-deleted cells, relative to RD control cells (measured by qPCR, n=2). (B) G401 and RD cells were incubated with either DMSO or 1 µM Compound A for 2, 4 and 7 days. Gene expression was determined by qPCR (n=2) and is expressed relative to the DMSO control of each time point. Panels a-j correspond to genes GLI1, PTCh1, DOCK4, CD133, PTPRK, BIN1, CDKN1A, CDKN2A, EZH2, and MYC, respectively. (C) G402 cells were incubated with either DMSO (left panel) or 1 µM Compound A (right panel) for 14 days. Cells were split and re-plated to the original seeding density on day 7.

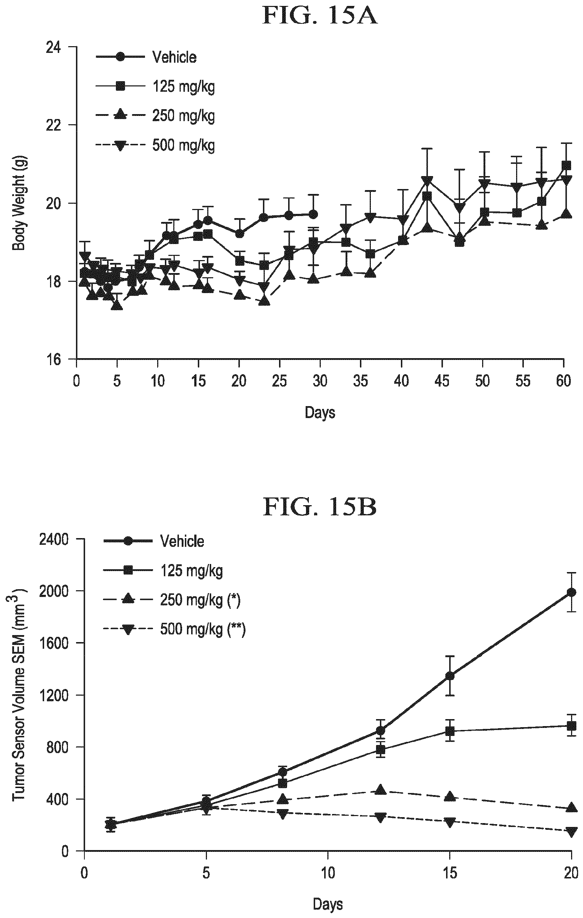

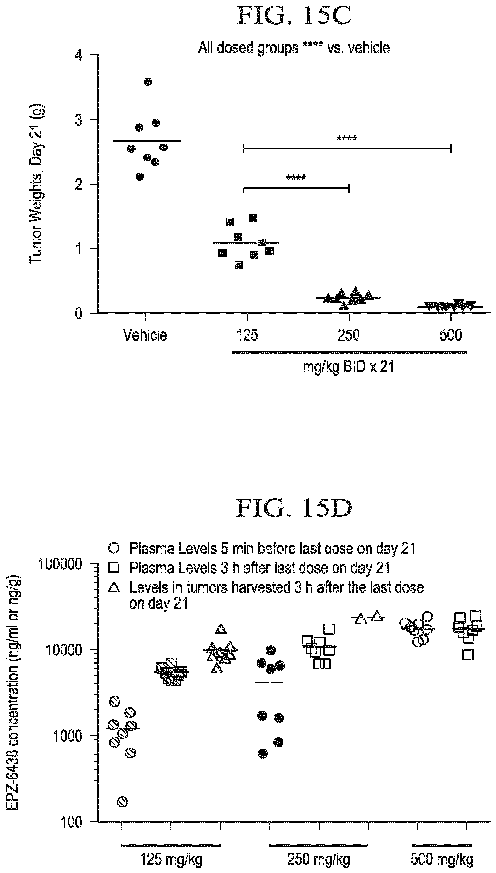

Figures 15A-15D are series of graphs demonstrating body weights, tumor regressions and plasma levels in G401 xenograft bearing mice treated with Compound A. (A) Body weights were determined twice a week for animals treated with Compound A on a BID schedule for 28 days. Data are presented as mean values ± SEM (n=16 until day 21, n=8 from day 22 to 60). (B) Tumor regressions induced by twice daily (BID) administration of Compound A for 21 days at the indicated doses (mean values ± SEM, n=16). * p <0.05, ** p < 0.01, repeated measures ANOVA, Dunnett's post-test vs. vehicle. (C) Tumor weights of 8 mice euthanized on day 21. **** p < 0.0001, Fisher's exact test. (D) Plasma was collected 5 min before and 3 h after dosing of Compound A on day 21, and compound levels were measured by LC-MS/MS. Animals were euthanized, and tumors were collected 3 h after dosing on day 21. Tumor homogenates were generated and subjected to LC-MS/MS analysis to determine Compound A concentrations. Note that tumor compound levels could not be determined from all animals especially in the higher dose groups because the xenografts were too small on day 21. Dots represent values for the individual animals; horizontal lines represent group mean values.

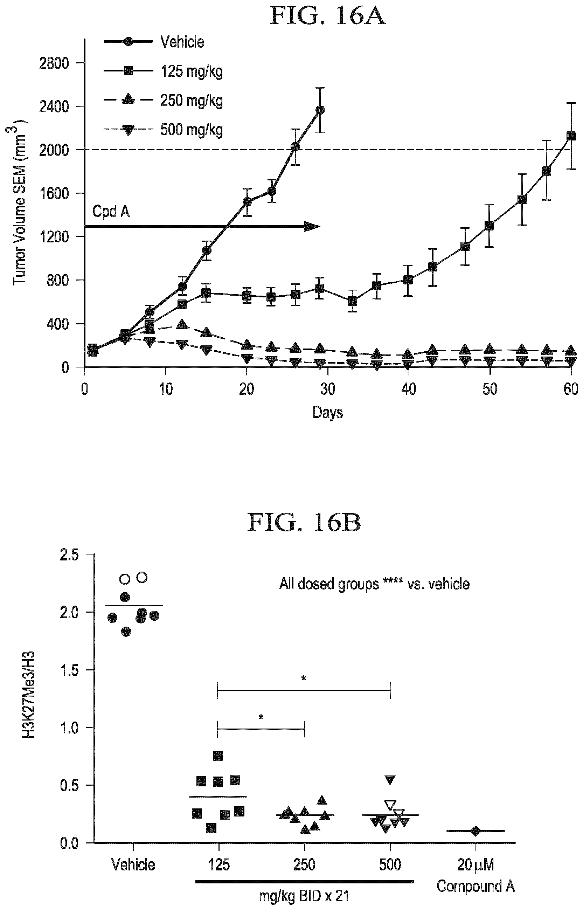

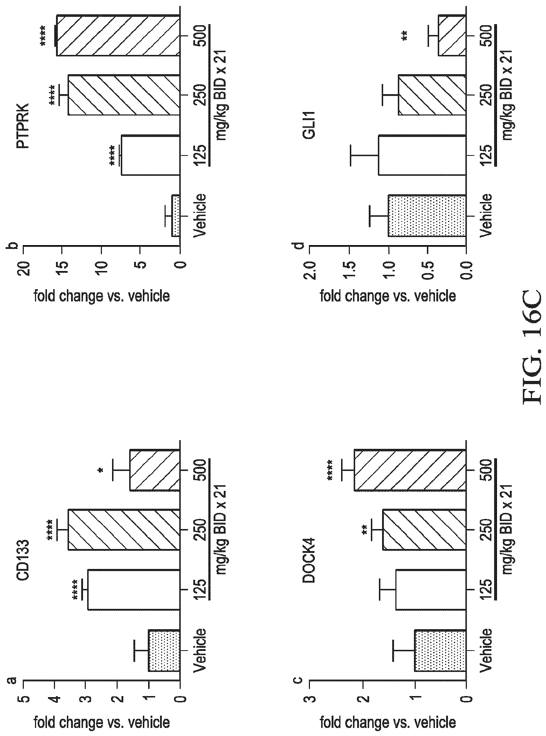

Figures 16A-16C are a series of graphs showing that Compound A eradicates *SMARCB1*-deleted MRT xenografts in SCID mice. (A) Tumor regressions induced by twice daily (BID) administration of Compound A for 28 days at the indicated doses. Compound administration was stopped on day 28 and tumors were allowed to re-grow until they reached 2000 mm³ (data shown as mean values ± SEM, n=8). (B) EZH2 target inhibition in G401 xenograft tumor tissue collected from mice euthanized on day 21. Each point shows the ratio of H3K27Me3 to total H3, measured by ELISA. Horizontal lines represent group mean values; grey symbols are values outside of the ELISA standard curve. (C) Change in gene expression in G401 xenograft tumor tissue collected from mice treated with Compound A for 21 days. Panels a-d correspond to genes CD133, PTPRK, DOCK4, and GLI1, respectively. Data are presented as fold change compared to vehicle ± SEM (n=6, n=4 for 500 mg/kg group). * p < 0.05, ** p < 0.01, **** p < 0.0001, vs. vehicle, Fisher's exact test.

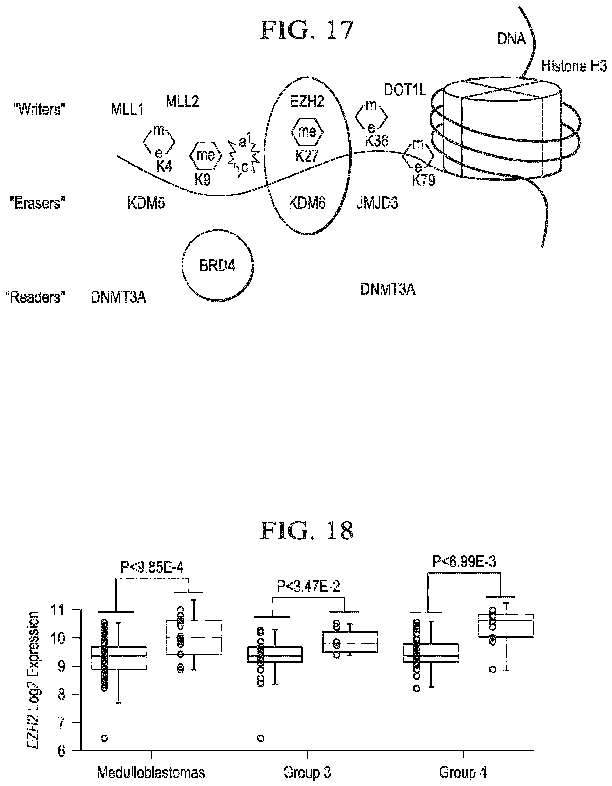

Figure 17 is a schematic diagram depicting epigenetic control of gene expression. Combinations of histone modifications encode information that governs coordinated activation or repression of genetic programs as well as developmental cell identity and fate decisions.
Figure 18 is a graph showing that EZH2 is over expressed and associated with chromosome 7 amplification in medulloblastoma. Solid bars indicate a balanced chromosome 7 whereas hatched bars indicate a chromosome 7 gain.

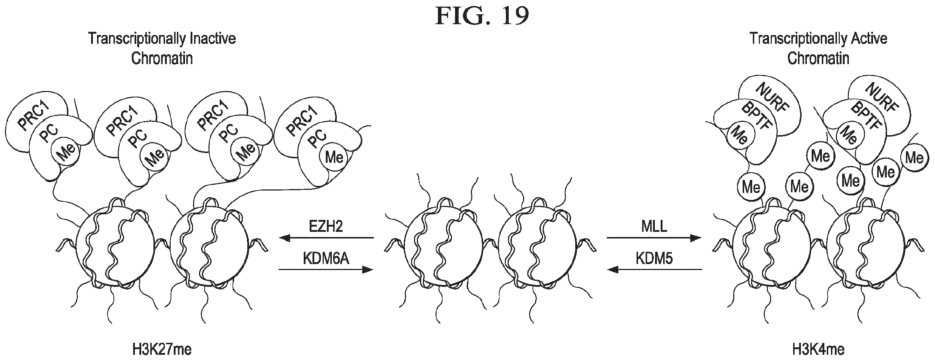

Figure 19 is a schematic diagram depicting control of histone lysine methylation by EZH2 and MLL.

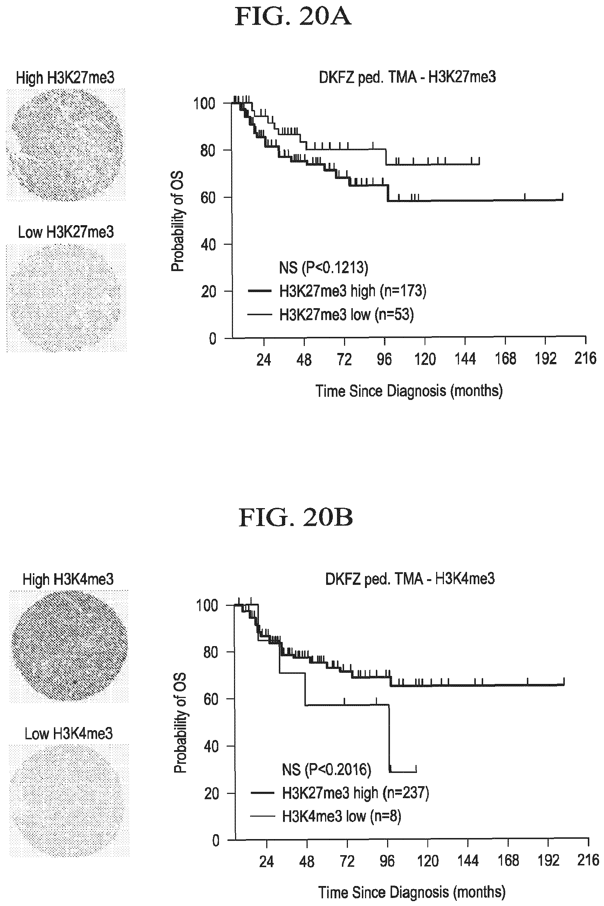

Figure 20A is a graph showing the probability of overall survival (OS) as a function of time since diagnosis (in months) with medulloblastoma. Histone lysine methylation is altered in medulloblastoma. H3K27me3 abundance is increased in medulloblastoma cells compared to control cells.
Figure 20B is a graph showing the probability of overall survival (OS) as a function of time since diagnosis (in months) with medulloblastoma. Histone lysine methylation is altered in medulloblastoma. H3K27me3 abundance is increased in medulloblastoma cells compared to control cells.

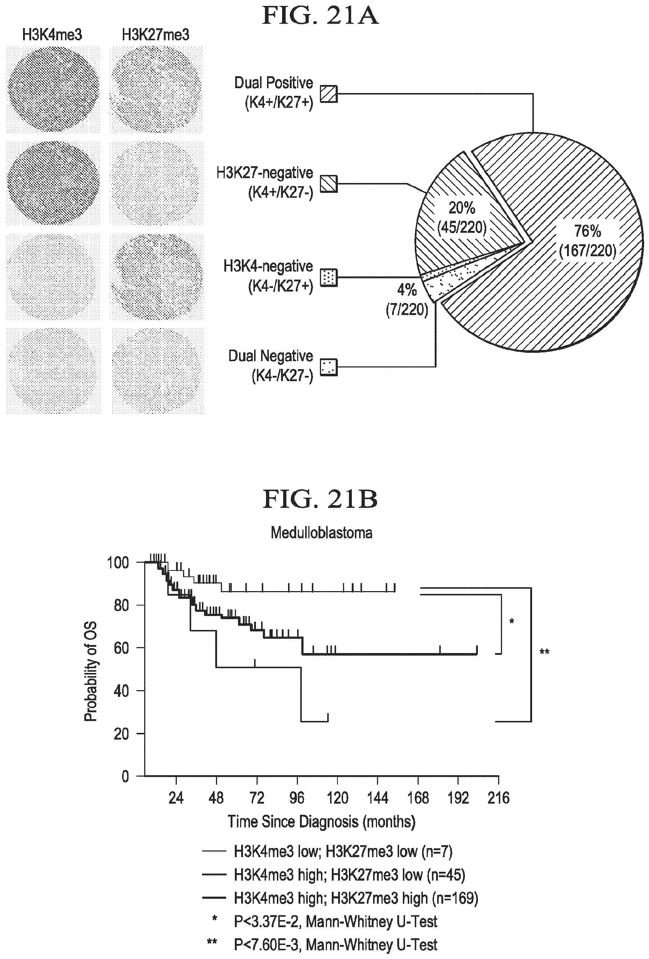

Figure 21A is a series of photographs and a graph showing the abundances of H3K4me3 and H3K27Me3 in medulloblastoma cells. The data demonstrate deregulation of the histone code in medulloblastoma.
Figure 21B is a graph depicting the probability of overall survival as a function of time since diagnosis (in months) for medulloblastoma subjects having deregulated histone methylation at H3K4me3 and/or H3K27Me3.

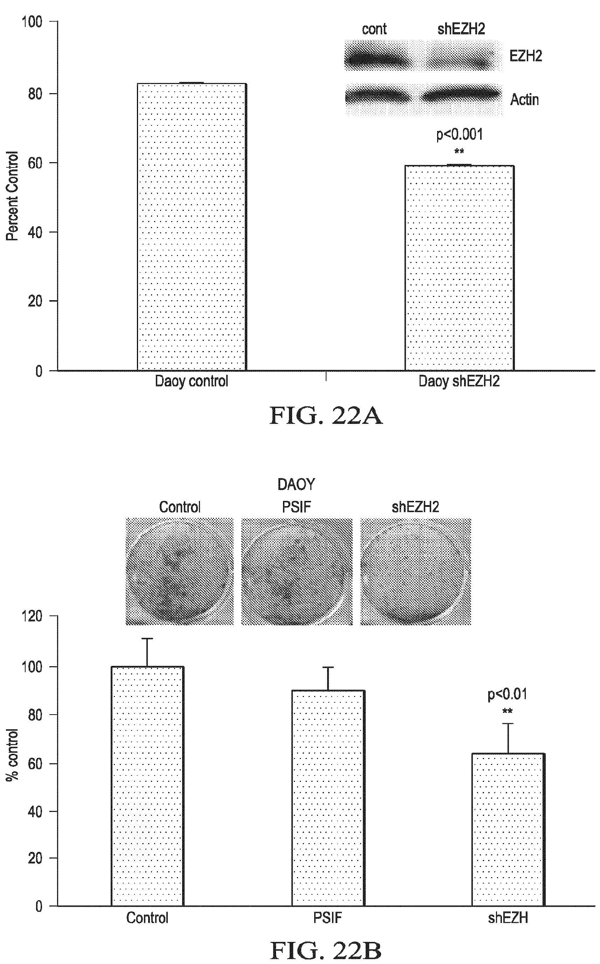

Figure 22A is a graph demonstrating that inhibition of EZH2 by a short-hairpin EZH2 (shEZH2) construct suppresses medulloblastoma cell growth (growth of the DAOY medulloblastoma cell line) compared to a negative-control construct.
Figure 22B a series of photographs and a graph demonstrating that inhibition of EZH2 by a short-hairpin EZH2 (shEZH2) construct suppresses medulloblastoma cell growth (growth of the DAOY medulloblastoma cell line) compared to a negative-control construct and/or the empty pSIF vector control.

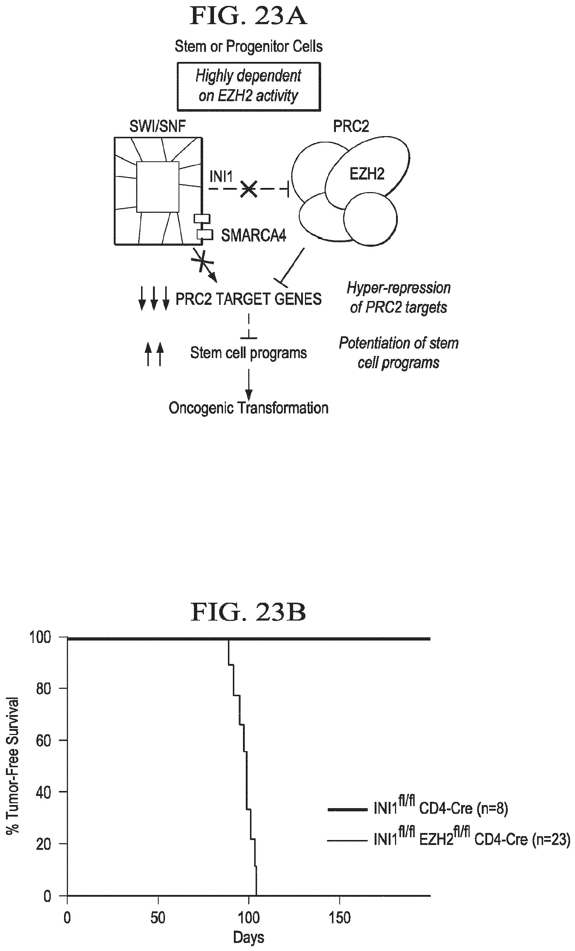

Figure 23A is a schematic diagram depicting the mechanism by which INI1 loss creates an oncogenic dependency on EZH2 in tumors.
Figure 23B is a graph showing the percent of tumor-free survival of INI1 deficient mice as a function of time (days) when EZH2 is knocked out. EZH2 knockout reverses oncogenesis induced by INI1 loss.

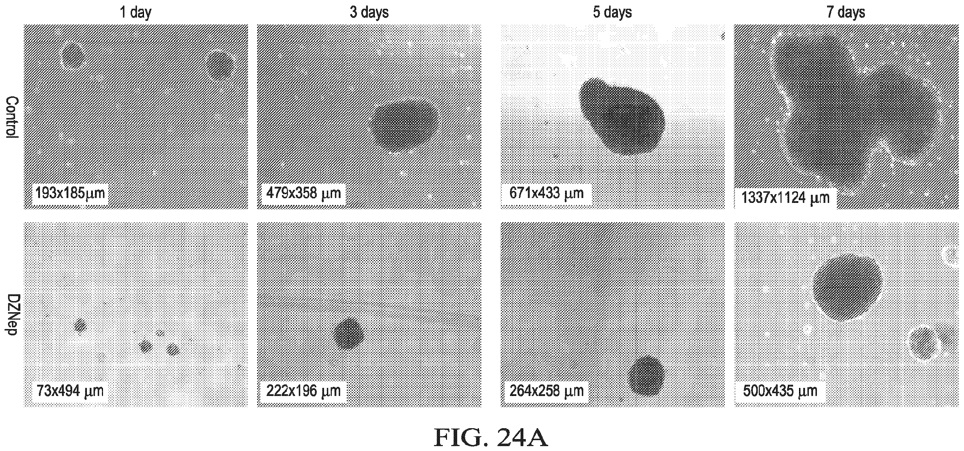

Figure 24A is a series of photographs showing control or EZH2 inhibitor-treated (DNZep-treated) atypical teratoid rhabdoid tumors (ATRTs) at 1, 3, 5, and 7 days post-treatment. Inhibition of EZH2 suppresses ATRT cell self-renewal.

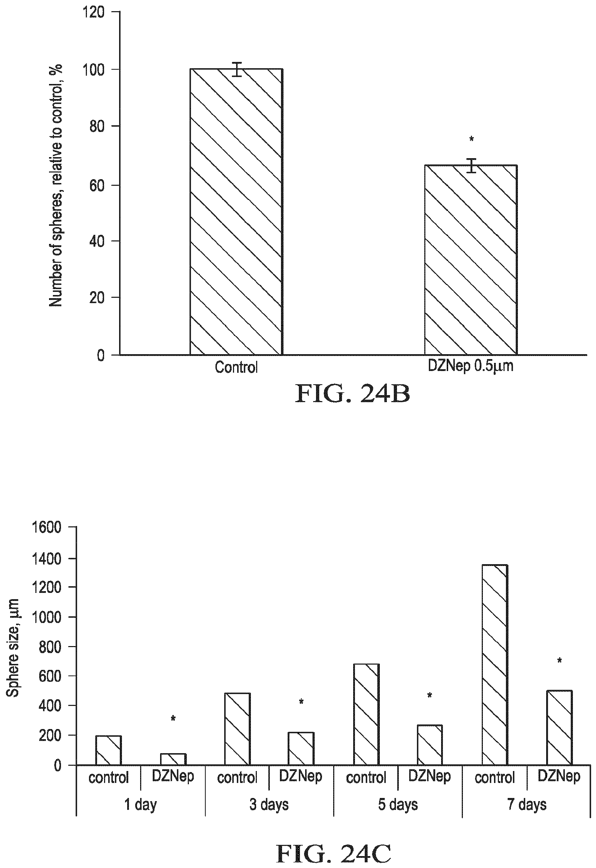

Figure 24B is a graph quantifying the results of Figure 24A.
Figure 24C is a graph quantifying the results of Figure 24A.

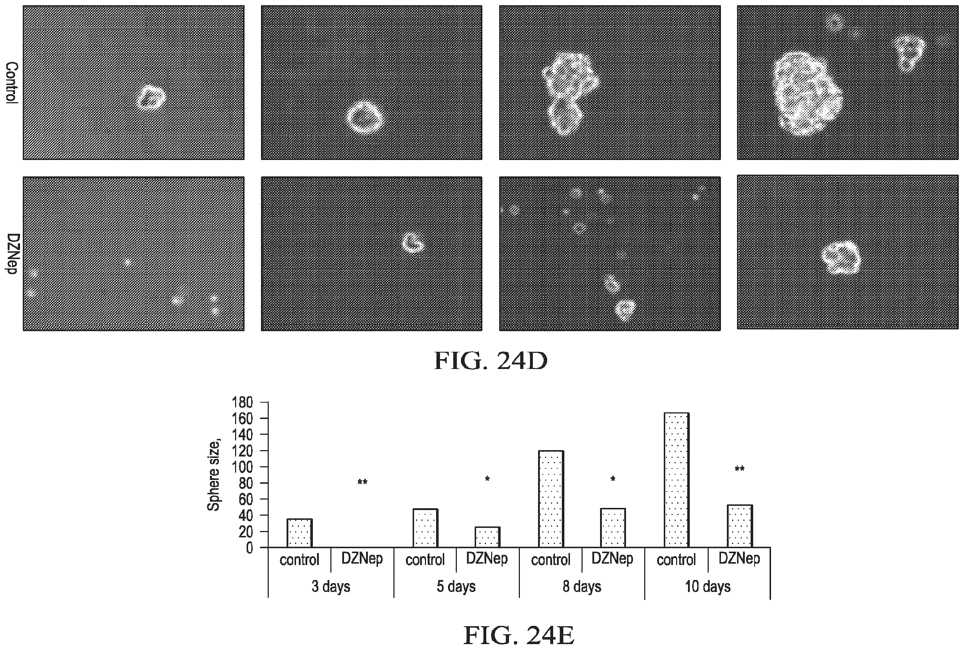

Figure 24D is a series of photographs showing control or EZH2 inhibitor-treated (DNZep-treated) atypical teratoid rhabdoid tumors (ATRTs) at 3, 5, 8 and 10 days post-treatment. Inhibition of EZH2 suppresses ATRT cell self-renewal.
Figure 24E is a graph quantifying the results of Figure 24D.

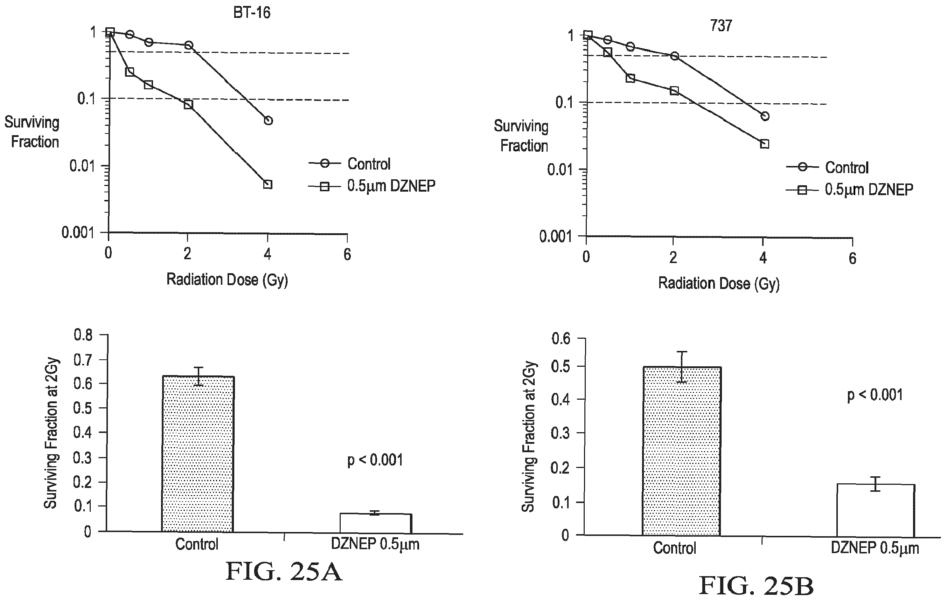

Figure 25A is a pair of graphs showing a surviving fraction of untreated or DZNEP-treated ATRT cells (from a BT-16 ATRT cell line) exposed to 2Gy radiation. Inhibition of EZH2 radio-sensitizes ATRT.
Figure 25B is a pair of graphs showing a surviving fraction of untreated or DZNEP-treated ATRT cells (from a UPN737 ATRT cell line, "737") exposed to 2Gy radiation. Inhibition of EZH2 radio-sensitizes ATRT.
Figure 26A is a graph showing the concentration of medulloblastoma cells (total cells per milliliter) as a function of time (days) following treatment with GSK-126, a small molecule inhibitor of EZH2. Small molecule inhibitors of EZH2 decrease medulloblastoma cell growth.
Figure 26B is a graph showing the concentration of medulloblastoma cells (total cells per milliliter) as a function of time (days) following treatment with UNC 1999, a small molecule inhibitor of EZH2. Small molecule inhibitors of EZH2 decrease medulloblastoma cell growth.
Figure 26C is a graph showing the concentration of medulloblastoma cells (total cells per milliliter) as a function of time (days) following treatment with tazemetostat (EPZ 6438), a small molecule inhibitor of EZH2. Small molecule inhibitors of EZH2 decrease medulloblastoma cell growth.
Figure 26D is a graph showing the concentration of medulloblastoma cells (total cells per milliliter) as a function of time (days) following treatment with GSK-126, UNC 1999, and tazemetostat (EPZ 6438). Tazemetostat has the greatest effect on medulloblastoma cell growth of the small molecule inhibitors tested.
Figure 27 is a pair of schematic diagrams depicting the relative selectivity of tazemetostat for EZH2.
Figure 28A is a schematic diagram depicting the process by which primary medulloblastoma cell growth is evaluated ex vivo.
Figure 28B is a graph depicting the relative abundances (percent of cells) of untreated or tazemetostat (EPZ 6438)-treated primary medulloblastoma cells in various cell cycle stages (sub Go/G1, Go/G1, S, or G2/M). A slice culture of medulloblastoma was freshly isolated from a 5 year old subject. The slice culture was treated with tazemetostat for 4 days before being disaggregated and analyzed by flow cytometry. Tazemetostat treatment decreases primary medulloblastoma cell growth ex vivo.
Figure 28C is a graph depicting BrdU expression of the cells analyzed in Figure 28B. Tazemetostat treatment decreases primary medulloblastoma cell growth ex vivo.

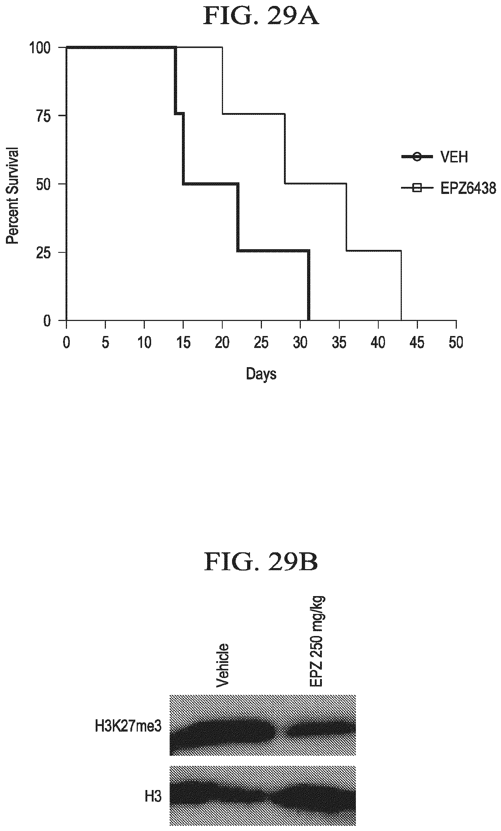

Figure 29A is a graph depicting percent survival of vehicle or tazemetostat (EPZ 6438)-treated ATRT cells in vivo as a function of time (days) post-treatment. Tazemetostat decreases ATRT in vivo.
Figure 29B is a photograph of a Western blot showing the relative amounts of H2K27me3 and H3 in vehicle or tazemetostat (EPZ 6438)-treated ATRT cells from Figure 29A.

### DETAILED DESCRIPTION

The disclosure provides a method of treating a medulloblastoma in a subject in need thereof comprising administering to the subject a therapeutically-effective amount of an enhancer of a zeste homolog 2 (EZH2) inhibitor. Methods of treating medulloblastoma of the disclosure may comprise preventing and/or inhibiting proliferation of a medulloblastoma cell.

The disclosure provides a method for treating or alleviating a symptom of a SWI/SNF-associated cancer in a subject by administering to a subject in need thereof a therapeutically effective amount of an EZH2 inhibitor. For example, the SWI/SNF-associated cancer is characterized by reduced expression and/or loss of function of the SWI/SNF complex or one or more components of the SWI/SNF complex. In a preferred embodiment, the cancer is medulloblastoma

Medulloblastoma results from reduced expression and/or loss of function of the SWI/SNF complex or one or more components of the SWI/SNF complex, including, but not limited to, SNF5, ATRX, and ARID1A. For example, the loss of function is caused by a loss of function mutation resulting from a point mutation, a deletion, and/or an insertion.

For example, the subject has a deletion of SNF5.

For example, the subject has a mutation of ATRX selected from the group consisting of a substitution of asparagine (N) for the wild type residue lysine (K) at amino acid position 688 of SEQ ID NO: 5 (K688N), and a substitution of isoleucine (I) for the wild type residue methionine (M) at amino acid position 366 of SEQ ID NO: 5 (M366I).

For example, subject has a mutation of ARID1A selected from the group consisting of a nonsense mutation for the wild type residue cysteine (C) at amino acid position 884 of SEQ ID NO: 11 (C884*), a substitution of lysine (K) for the wild type residue glutamic acid (E) at amino acid position 966 (E966K), a nonsense mutation for the wild type residue glutamine (Q) at amino acid position 1411 of SEQ ID NO: 11 (Q1411*), a frame shift mutation at the wild type residue phenylalanine (F) at amino acid position 1720 of SEQ ID NO: 11 (F1720fs), a frame shift mutation after the wild type residue glycine (G) at amino acid position 1847 of SEQ ID NO: 11 (G1847fs), a frame shift mutation at the wild type residue cysteine (C) at amino acid position 1874 of SEQ ID NO: 11 (C1874fs), a substitution of glutamic acid (E) for the wild type residue aspartic acid (D) at amino acid position 1957 (D1957E), a nonsense mutation for the wild type residue glutamine (Q) at amino acid position 1430 of SEQ ID NO: 11 (Q1430*), a frame shift mutation at the wild type residue arginine (R) at amino acid position 1721 of SEQ ID NO: 11 (R1721fs), a substitution of glutamic acid (E) for the wild type residue glycine (G) at amino acid position 1255 (G1255E), a frame shift mutation at the wild type residue glycine (G) at amino acid position 284 of SEQ ID NO: 11 (G284fs), a nonsense mutation for the wild type residue arginine (R) at amino acid position 1722 of SEQ ID NO: 11 (R1722*), a frame shift mutation at the wild type residue methionine (M) at amino acid position 274 of SEQ ID NO: 11 (M274fs), a frame shift mutation at the wild type residue glycine (G) at amino acid position 1847 of SEQ ID NO: 11 (G1847fs), a frame shift mutation at the wild type residue P at amino acid position 559 of SEQ ID NO: 11 (P559fs), a nonsense mutation for the wild type residue arginine (R) at amino acid position 1276 of SEQ ID NO: 11 (R1276*), a frame shift mutation at the wild type residue glutamine (Q) at amino acid position 2176 of SEQ ID NO: 11 (Q2176fs), a frame shift mutation at the wild type residue histidine (H) at amino acid position 203 of SEQ ID NO: 11 (H203fs), a frame shift mutation at the wild type residue alanine (A) at amino acid position 591 of SEQ ID NO: 11 (A591fs), a nonsense mutation for the wild type residue glutamine (Q) at amino acid position 1322 of SEQ ID NO: 11 (Q1322*), a nonsense mutation for the wild type residue serine (S) at amino acid position 2264 of SEQ ID NO: 11 (S2264*), a nonsense mutation for the wild type residue glutamine (Q) at amino acid position 586 of SEQ ID NO: 11 (Q586*), a frame shift mutation at the wild type residue glutamine (Q) at amino acid position 548 of SEQ ID NO: 11 (Q548fs), and a frame shift mutation at the wild type residue asparagine (N) at amino acid position 756 of SEQ ID NO: 11 (N756fs).

The disclosure also provides a method of treating or alleviating a symptom of a SWI/SNF-associated cancer in a subject in need thereof by (a) determining the expression level of at least one gene selected from the group consisting of neuronal differentiation genes, cell cycle inhibition genes and tumor suppressor genes in a sample obtained from the subject; (b) selecting the subject having a decreased expression level of at least one gene in step a; and (c) administering to the subject selected in step b an effective amount of an EZH2 inhibitor, thereby treating or alleviating a symptom of cancer in the subject. In a preferred embodiment, the cancer is medulloblastoma.

The disclosure further provides a method of treating or alleviating a symptom of a SWI/SNF-associated cancer in a subject in need thereof by (a) determining the expression level of at least one gene selected from the group consisting of hedgehog pathway genes, myc pathway genes and histone methyltransferase genes in a sample obtained from the subject; (b) selecting the subject having an increased expression level of at least one gene in step a; and (c) administering to the subject selected in step b an effective amount of an EZH2 inhibitor, thereby treating or alleviating a symptom of cancer in the subject. In a preferred embodiment, the cancer is medulloblastoma.

For example, the neuronal differentiation gene is CD133, DOCK4, or PTPRK.

For example, the cell cycle inhibition gene is CKDN1A or CDKN2A.

For example, the tumor suppressor gene is BIN1.

For example, the hedgehog pathway gene is GLI1 or PTCH1.

For example, the myc pathway gene is MYC.

For example, the histone methyltransferase gene is EZH2.

The disclosure also provides a method of inducing neuronal differentiation, cell cycle inhibition or tumor suppression by contacting a cell with an EZH2 inhibitor. The EZH2 inhibitor may be in an amount sufficient to increase expression of at least one gene selected from the group consisting of CD133, DOCK4, PTPRK, CKDN1A, CDKN2A andBIN1.

The disclosure also provides a method of inhibiting hedgehog signaling by contacting a cell with an EZH2 inhibitor. The EZH2 inhibitor can be in an amount sufficient to reduce expression of GLI1 and/or PTCH1.

The disclosure also provides a method of inducing gene expression by contacting a cell with an EZH2 inhibitor. The EZH2 inhibitor can be in an amount sufficient to induce neuronal differentiation, cell cycle inhibition and/or tumor suppression. For example, the gene can be CD133, DOCK4, PTPRK, CKDN1A, CKDN2A or BIN1.

The disclosure also provides a method of inhibiting gene expression by contacting a cell with an EZH2 inhibitor. The EZH2 inhibitor is in an amount sufficient to inhibit hedgehog signaling. For example, the gene can be GLI1 or PTCH1.

For example, the cell may have loss of function of SNF5, ARID1A, ATRX, and/or a component of the SWI/SNF complex.

For example, the loss of function is caused by a deletion of SNF5.

For example, the cell is a cancer cell. Preferably, the cancer is medulloblastoma.

For example, the EZH2 inhibitor comprises 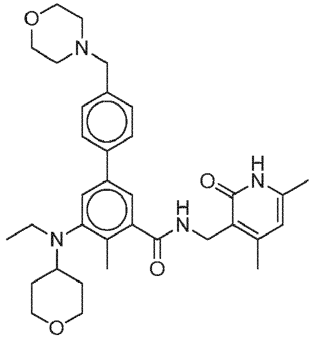 (tazemetostat), or a pharmaceutically-acceptable salt thereof.

For example, the EZH2 inhibitor comprises 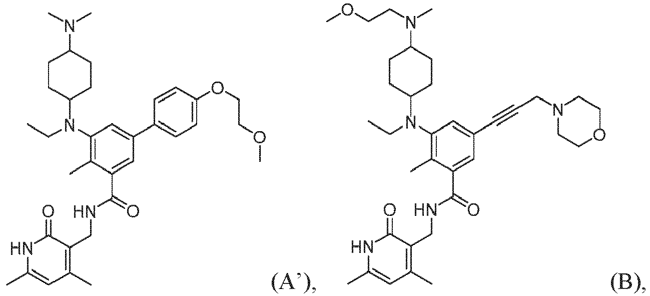 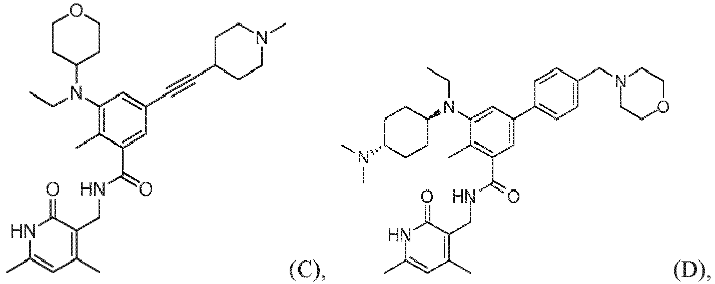 a stereoisomer, a pharmaceutically acceptable salt and/or a solvate thereof.

For example, the EZH2 inhibitor comprises 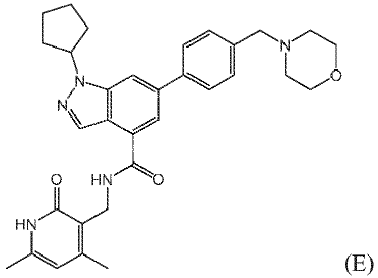 or a pharmaceutically acceptable salt thereof.

For example, the EZH2 inhibitor comprises 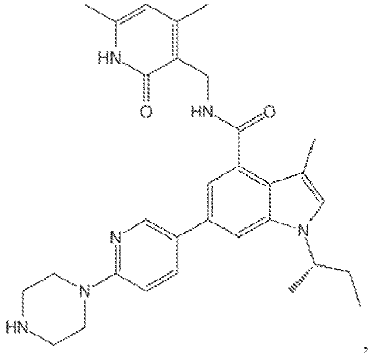 a stereoisomer, a pharmaceutically acceptable salt and/or a solvate thereof.

For example, the EZH2 inhibitor comprises 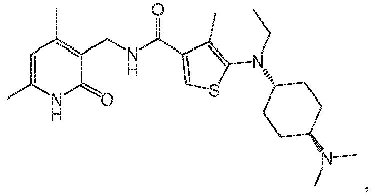 a stereoisomer, a pharmaceutically acceptable salt and/or a solvate thereof.

For example, the EZH2 inhibitor comprises 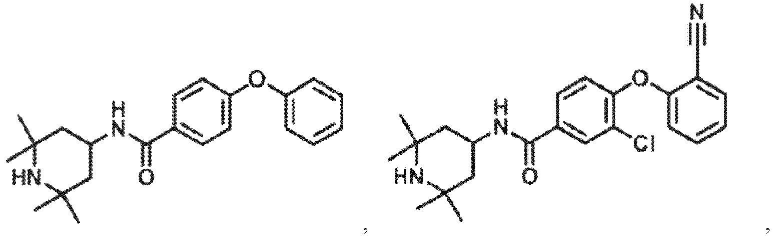 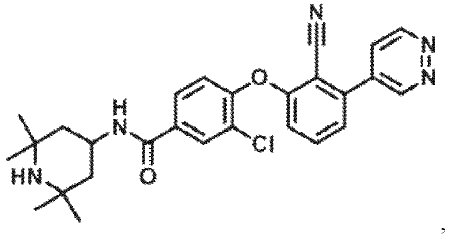 a stereoisomer, a pharmaceutically acceptable salt and/or a solvate thereof.

Human nucleic acid and amino acid sequence of components of the SWI/SNF complex have previously been described. *See, e.g.,* GenBank Accession Nos NP_003064.2, NM_003073.3, NP_001007469.1, andNM_001007468.1 for SNF5, GenBank Accession Nos NM_000489.3, NP_000480.2, NM_138270.2, andNP_612114.1 for ATRX, GenBank Accession Nos NP_006006.3, NM_006015.4, NP_624361.1, and NM_139135.2 for ARID1A, each of which is incorporated herein by reference in its entirety.

Spectrum of hSNF5 somatic mutations in human has also been described in Sevenet et al., Human Molecular Genetics, 8: 2359-2368, 1999, which is incorporated herein by reference in its entirety.

A subject in need thereof may have reduced expression, haploinsufficiency, and/or loss of function of SNF5. For example, a subject can comprise a deletion of SNF5 in SNF5 polypeptide or a nucleic acid sequence encoding a SNF5 polypeptide.

| |
|---|
| SWI/SNF-related matrix-associated actin-dependent regulator of chromatin subfamily B member 1 isoform a (SMARCB1, also called SNF5) [Homo sapiens] (SEQ ID NO: 1) |
| 1  |

| |
|---|
| Homo sapiens SWI/SNF related, matrix associated, actin dependent regulator of chromatin, subfamily b, member 1 (SMARCB1, also called SNF5), transcript variant 1, mRNA (SEQ ID NO: 2) |
| 1 aacgccagcg cctgcgcact gagggcggcc tggtcgtcgt ctgcggcggc ggcggcggct 61 gaggagcccg gctgaggcgc cagtacccgg cccggtccgc atttcgcctt ccggcttcgg 121 tttccctcgg cccagcacgc cccggccccg ccccagccct cctgatccct cgcagcccgg 181 ctccggccgc ccgcctctgc cgccgcaatg atgatgatgg cgctgagcaa gaccttcggg 241 cagaagcccg tgaagttcca gctggaggac gacggcgagt tctacatgat cggctccgag 301 gtgggaaact acctccgtat gttccgaggt tctctgtaca agagataccc ctcactctgg 361 aggcgactag ccactgtgga agagaggaag aaaatagttg catcgtcaca tggtaaaaaa 421 acaaaaccta acactaagga tcacggatac acgactctag ccaccagtgt gaccctgtta 481 aaagcctcgg aagtggaaga gattctggat ggcaacgatg agaagtacaa ggctgtgtcc 541 atcagcacag agccccccac ctacctcagg gaacagaagg ccaagaggaa cagccagtgg 601 gtacccaccc tgcccaacag ctcccaccac ttagatgccg tgccatgctc cacaaccatc 661 aacaggaacc gcatgggccg agacaagaag agaaccttcc ccctttgctt tgatgaccat 721 gacccagctg tgatccatga gaacgcatct cagcccgagg tgctggtccc catccggctg 781 gacatggaga tcgatgggca gaagctgcga gacgccttca cctggaacat gaatgagaag 841 ttgatgacgc ctgagatgtt ttcagaaatc ctctgtgacg atctggattt gaacccgctg 901 acgtttgtgc cagccatcgc ctctgccatc agacagcaga tcgagtccta ccccacggac 961 agcatcctgg aggaccagtc agaccagcgc gtcatcatca agctgaacat ccatgtggga 1021 aacatttccc tggtggacca gtttgagtgg gacatgtcag agaaggagaa ctcaccagag 1081 aagtttgccc tgaagctgtg ctcggagctg gggttggcgg gggagtttgt caccaccatc 1141 gcatacagca tccggggaca gctgagctgg catcagaaga cctacgcctt cagcgagaac 1201 cctctgccca cagtggagat tgccatccgg aacacgggcg atgcggacca gtggtgccca 1261 ctgctggaga ctctgacaga cgctgagatg gagaagaaga tccgcgacca ggacaggaac |
| 1321 acgaggcgga tgaggcgtct tgccaacacg gccccggcct ggtaaccagc ccatcagcac 1381 acggctccca cggagcatct cagaagattg ggccgcctct cctccatctt ctggcaagga 1441 cagaggcgag gggacagccc agcgccatcc tgaggatcgg gtgggggtgg agtgggggct 1501 tccaggtggc ccttcccggc acacattcca tttgttgagc cccagtcctg ccccccaccc 1561 caccctccct acccctcccc agtctctggg gtcaggaaga aaccttattt taggttgtgt 1621 tttgtttttg tataggagcc ccaggcaggg ctagtaacag tttttaaata aaaggcaaca 1681 ggtcatgttc aatttcttca acaaaaaaaa aaaaaaa |

| |
|---|
| SWI/SNF-related matrix-associated actin-dependent regulator of chromatin subfamily B member 1 isoform b [Homo sapiens] (SMARCB1, also called SNF5) (SEQ ID NO: 3) |
| 1 mmmmalsktf gqkpvkfqle ddgefymigs evgnylrmfr gslykrypsl wrrlatveer 61 kkivasshdh gyttlatsvt llkaseveei ldgndekyka vsisteppty lreqkakrns 121 qwvptlpnss hhldavpcst tinrnrmgrd kkrtfplcfd dhdpavihen asqpevlvpi 181 rldmeidgqk lrdaftwnmn eklmtpemfs eilcddldln pltfvpaias airqqiesyp 241 tdsiledqsd qrviiklnih vgnislvdqf ewdmsekens pekfalklcs elglggefvt 301 tiaysirgql swhqktyafs enplptveia irntgdadqw cplletltda emekkirdqd 361 rntrrmrrla ntapaw |

| |
|---|
| Homo sapiens SWI/SNF related, matrix associated, actin dependent regulator of chromatin, subfamily b, member 1 (SMARCB1, also called SNF5), transcript variant 2, mRNA (SEQ ID NO: 4) |
| 1 aacgccagcg cctgcgcact gagggcggcc tggtcgtcgt ctgcggcggc ggcggcggct 61 gaggagcccg gctgaggcgc cagtacccgg cccggtccgc atttcgcctt ccggcttcgg 121 tttccctcgg cccagcacgc cccggccccg ccccagccct cctgatccct cgcagcccgg 181 ctccggccgc ccgcctctgc cgccgcaatg atgatgatgg cgctgagcaa gaccttcggg 241 cagaagcccg tgaagttcca gctggaggac gacggcgagt tctacatgat cggctccgag 301 gtgggaaact acctccgtat gttccgaggt tctctgtaca agagataccc ctcactctgg 361 aggcgactag ccactgtgga agagaggaag aaaatagttg catcgtcaca tgatcacgga 421 tacacgactc tagccaccag tgtgaccctg ttaaaagcct cggaagtgga agagattctg 481 gatggcaacg atgagaagta caaggctgtg tccatcagca cagagccccc cacctacctc 541 agggaacaga aggccaagag gaacagccag tgggtaccca ccctgcccaa cagctcccac 601 cacttagatg ccgtgccatg ctccacaacc atcaacagga accgcatggg ccgagacaag 661 aagagaacct tccccctttg ctttgatgac catgacccag ctgtgatcca tgagaacgca 721 tctcagcccg aggtgctggt ccccatccgg ctggacatgg agatcgatgg gcagaagctg 781 cgagacgcct tcacctggaa catgaatgag aagttgatga cgcctgagat gttttcagaa 841 atcctctgtg acgatctgga tttgaacccg ctgacgtttg tgccagccat cgcctctgcc 901 atcagacagc agatcgagtc ctaccccacg gacagcatcc tggaggacca gtcagaccag 961 cgcgtcatca tcaagctgaa catccatgtg ggaaacattt ccctggtgga ccagtttgag 1021 tgggacatgt cagagaagga gaactcacca gagaagtttg ccctgaagct gtgctcggag 1081 ctggggttgg gcggggagtt tgtcaccacc atcgcataca gcatccgggg acagctgagc 1141 tggcatcaga agacctacgc cttcagcgag aaccctctgc ccacagtgga gattgccatc 1201 cggaacacgg gcgatgcgga ccagtggtgc ccactgctgg agactctgac agacgctgag 1261 atggagaaga agatccgcga ccaggacagg aacacgaggc ggatgaggcg tcttgccaac 1321 acggccccgg cctggtaacc agcccatcag cacacggctc ccacggagca tctcagaaga 1381 ttgggccgcc tctcctccat cttctggcaa ggacagaggc gaggggacag cccagcgcca 1441 tcctgaggat cgggtggggg tggagtgggg gcttccaggt ggcccttccc ggcacacatt 1501 ccatttgttg agccccagtc ctgcccccca ccccaccctc cctacccctc cccagtctct 1561 ggggtcagga agaaacctta ttttaggttg tgttttgttt ttgtatagga gccccaggca 1621 gggctagtaa cagtttttaa ataaaaggca acaggtcatg ttcaatttct tcaacaaaaa 1681 aaaaaaaaaa |

A subject in need thereof may have reduced expression, haploinsufficiency, and/or loss of function of ATRX. For example, a subject can comprise a mutation selected from the group consisting of a substitution of asparagine (N) for the wild type residue lysine (K) at amino acid position 688 of SEQ ID NO: 5 (K688N), and a substitution of isoleucine (I) for the wild type residue methionine (M) at amino acid position 366 of SEQ ID NO: 5 (M366I).

| |
|---|
| Homo sapiens alpha thalassemia/mental retardation syndrome X-linked (ATRX) isoform 1 (SEQ ID NO: 5) |
|    1 mtaepmsesk lntlvqklhd flahsseese etsspprlam nqntdkisgs gsnsdmmens 61 keegtsssek skssgssrsk rkpsivtkyv esddekpldd etvnedasne nsenditmqs 121 lpkgtvivqp epvlnedkdd fkgpefrsrs kmktenlkkr gedglhgivs ctacgqqvnh 181 fqkdsiyrhp slqvlicknc fkyymsddis rdsdgmdeqc rwcaeggnli ccdfchnafc 241 kkcilrnlgr kelstimden nqwycyichp eplldlvtac nsvfenleql lqqnkkkikv 301 dseksnkvye htsrfspkkt ssncngeekk lddscsgsvt ysysalivpk emikkakkli 361 ettanmnssy vkflkqatdn seissatklr qlkafksvla dikkahlale edlnsefram 421 davnkekntk ehkvidakfe tkarkgekpc alekkdisks eaklsrkqvd sehmhqnvpt 481 eeqrtnkstg gehkksdrke epqyepants edldmdivsv pssvpedife nletamevqs 541 svdhqgdgss gteqevesss vklnisskdn rggiksktta kvtkelyvkl tpvslsnspi 601 kgadcqevpq dkdgykscgl npklekcglg qensdnehlv enevslllee sdlrrsprvk 661 ttplrrptet npvtsnsdee cnetvkekqk lsvpvrkkdk rnssdsaidn pkpnklpksk 721 qsetvdqnsd sdemlailke vsrmshssss dtdineihtn hktlydlktq agkddkgkrk 781 rksstsgsdf dtkkgksaks siiskkkrqt qsessnydse lekeiksmsk igaarttkkr 841 ipntkdfdss edekhskkgm dnqghknlkt sqegssddae rkqeretfss aegtvdkdtt 901 imelrdrlpk kqqasastdg vdklsgkeqs ftslevrkva etkekskhlk tktckkvqdg 961 lsdiaekflk kdqsdetsed dkkqskkgte ekkkpsdfkk kvikmeqqye sssdgteklp 1021 ereeichfpk gikqikngtt dgekkskkir dktskkkdel sdyaekstgk gdscdssedk 1081 ksknqaygre kkrckllgks srkrqdcsss dtekysmked gcnssdkrlk rielrerrnl 1141 sskrntkeiq sgssssdaee ssednkkkkq rtsskkkavi vkekkrnslr tstkrkqadi 1201 tsssssdied ddqnsigegs sdeqkikpvt enlvlsshtg fcqssgdeal sksvpvtvdd 1261 ddddndpenr iakkmlleei kanlssdedg ssddepeegk krtgkqneen pgdeeaknqv 1321 nsesdsdsee skkpryrhrl lrhkltvsdg esgeekktkp kehkevkgrn rrkvssedse 1381 dsdfqesgvs eevsesedeq rprtrsakka eleenqrsyk qkkkrrrikv qedsssenks 1441 nseeeeeeke eeeeeeeeee eeeedendds kspgkgrkki rkilkddklr tetqnalkee 1501 eerrkriaer erereklrev ieiedasptk cpittklvld edeetkeplv qvhrnmvikl 1561 kphqvdgvqf mwdcccesvk ktkkspgsgc ilahcmglgk tlqvvsflht vllcdkldfs 1621 talvvcplnt alnwmnefek wqeglkddek levselatvk rpqersymlq rwqedggvmi 1681 igyemyrnla qgrnvksrkl keifnkalvd pgpdfvvcde ghilkneasa vskamnsirs 1741 rrriiltgtp lqnnlieyhc mvnfikenll gsikefrnrf inpiqngqca dstmvdvrvm 1801 kkrahilyem lagcvqrkdy taltkflppk heyvlavrmt siqcklyqyy ldhltgvgnn 1861 seggrgkaga klfqdfqmls riwthpwclq ldyiskenkg yfdedsmdef iasdsdetsm 1921 slssddytkk kkkgkkgkkd ssssgsgsdn dvevikvwns rsrgggegnv detgnnpsvs 1981 lkleeskats ssnpsspapd wykdfvtdad aevlehsgkm vllfeilrma eeigdkvlvf 2041 sqslisldli edflelasre ktedkdkpli ykgegkwlrn idyyrldgst taqsrkkwae 2101 efndetnvrg rlfiistkag slginlvaan rviifdaswn psydiqsifr vyrfgqtkpv 2161 yvyrflaqgt medkiydrqv tkqslsfrvv dqqqverhft mneltelytf epdllddpns 2221 ekkkkrdtpm lpkdtilael lqihkehivg yhehdslldh keeeelteee rkaawaeyea 2281 ekkgltmrfn iptgtnlppv sfnsqtpyip fnlgalsams nqqledlinq grekvveatn 2341 svtavriqpl ediisavwke nmnlseaqvq alalsrqasq eldvkrreai yndvltkqqm 2401 liscvqrilm nrrlqqqynq qqqqqmtyqq atlghlmmpk ppnlimnpsn yqqidmrgmy 2461 qpvaggmqpp plqrapppmr sknpgpsqgk sm |

| |
|---|
| Homo sapiens alpha thalassemia/mental retardation syndrome X-linked (ATRX), transcript variant 1, mRNA (SEQ ID NO: 6) |
| 1 aattctcctg cctgagcctc ggcccaacaa aatggcggcg gcagcggtgt cgctttgttt 61 ccgcggctcc tgcggcggtg gcagtggtag cggcctttga gctgtgggga ggttccagca 121 gcagctacag tgacgactaa gactccagtg catttctatc gtaaccgggc gcgggggagc 181 gcagatcggc gcccagcaat cacagaagcc gacaaggcgt tcaagcgaaa acatgaccgc 241 tgagcccatg agtgaaagca agttgaatac attggtgcag aagcttcatg acttccttgc 301 acactcatca gaagaatctg aagaaacaag ttctcctcca cgacttgcaa tgaatcaaaa 361 cacagataaa atcagtggtt ctggaagtaa ctctgatatg atggaaaaca gcaaggaaga 421 gggaactagc tcttcagaaa aatccaagtc ttcaggatcg tcacgatcaa agaggaaacc 481 ttcaattgta acaaagtatg tagaatcaga tgatgaaaaa cctttggatg atgaaactgt 541 aaatgaagat gcgtctaatg aaaattcaga aaatgatatt actatgcaga gcttgccaaa 601 aggtacagtg attgtacagc cagagccagt gctgaatgaa gacaaagatg attttaaagg 661 gcctgaattt agaagcagaa gtaaaatgaa aactgaaaat ctcaaaaaac gcggagaaga 721 tgggcttcat gggattgtga gctgcactgc ttgtggacaa caggtcaatc attttcaaaa 781 agattccatt tatagacacc cttcattgca agttcttatt tgtaagaatt gctttaagta 841 ttacatgagt gatgatatta gccgtgactc agatggaatg gatgaacaat gtaggtggtg 901 tgcggaaggt ggaaacttga tttgttgtga cttttgccat aatgctttct gcaagaaatg 961 cattctacgc aaccttggtc gaaaggagtt gtccacaata atggatgaaa acaaccaatg 1021 gtattgctac atttgtcacc cagagccttt gttggacttg gtcactgcat gtaacagcgt 1081 atttgagaat ttagaacagt tgttgcagca aaataagaag aagataaaag ttgacagtga 1141 aaagagtaat aaagtatatg aacatacatc cagattttct ccaaagaaga ctagttcaaa 1201 ttgtaatgga gaagaaaaga aattagatga ttcctgttct ggctctgtaa cctactctta 1261 ttccgcacta attgtgccca aagagatgat taagaaggca aaaaaactga ttgagaccac 1321 agccaacatg aactccagtt atgttaaatt tttaaagcag gcaacagata attcagaaat 1381 cagttctgct acaaaattac gtcagcttaa ggcttttaag tctgtgttgg ctgatattaa 1441 gaaggctcat cttgcattgg aagaagactt aaattccgag tttcgagcga tggatgctgt 1501 aaacaaagag aaaaatacca aagagcataa agtcatagat gctaagtttg aaacaaaagc 1561 acgaaaagga gaaaaacctt gtgctttgga aaagaaggat atttcaaagt cagaagctaa 1621 actttcaaga aaacaggtag atagtgagca catgcatcag aatgttccaa cagaggaaca 1681 aagaacaaat aaaagtaccg gtggtgaaca taagaaatct gatagaaaag aagaacctca 1741 atatgaacct gccaacactt ctgaagattt agacatggat attgtgtctg ttccttcctc 1801 agttccagaa gacatttttg agaatcttga gactgctatg gaagttcaga gttcagttga 1861 tcatcaaggg gatggcagca gtggaactga acaagaagtg gagagttcat ctgtaaaatt 1921 aaatatttct tcaaaagaca acagaggagg tattaaatca aaaactacag ctaaagtaac 1981 aaaagaatta tatgttaaac tcactcctgt ttccctttct aattccccaa ttaaaggtgc 2041 tgattgtcag gaagttccac aagataaaga tggctataaa agttgtggtc tgaaccccaa 2101 gttagagaaa tgtggacttg gacaggaaaa cagtgataat gagcatttgg ttgaaaatga 2161 agtttcatta cttttagagg aatctgatct tcgaagatcc ccacgtgtaa agactacacc 2221 cttgaggcga ccgacagaaa ctaaccctgt aacatctaat tcagatgaag aatgtaatga 2281 aacagttaag gagaaacaaa aactatcagt tccagtgaga aaaaaggata agcgtaattc 2341 ttctgacagt gctatagata atcctaagcc taataaattg ccaaaatcta agcaatcaga 2401 gactgtggat caaaattcag attctgatga aatgctagca atcctcaaag aggtgagcag 2461 gatgagtcac agttcttctt cagatactga tattaatgaa attcatacaa accataagac 2521 tttgtatgat ttaaagactc aggcggggaa agatgataaa ggaaaaagga aacgaaaaag 2581 ttctacatct ggctcagatt ttgatactaa aaagggcaaa tcagctaaga gctctataat 2641 ttctaaaaag aaacgacaaa cccagtctga gtcttctaat tatgactcag aattagaaaa 2701 agagataaag agcatgagta aaattggtgc tgccagaacc accaaaaaaa gaattccaaa 2761 tacaaaagat tttgactctt ctgaagatga gaaacacagc aaaaaaggaa tggataatca 2821 agggcacaaa aatttgaaga cctcacaaga aggatcatct gatgatgctg aaagaaaaca 2881 agagagagag actttctctt cagcagaagg cacagttgat aaagacacga ccatcatgga 2941 attaagagat cgacttccta agaagcagca agcaagtgct tccactgatg gtgtcgataa 3001 gctttctggg aaagagcaga gttttacttc tttggaagtt agaaaagttg ctgaaactaa 3061 agaaaagagc aagcatctca aaaccaaaac atgtaaaaaa gtacaggatg gcttatctga 3121 tattgcagag aaattcctaa agaaagacca gagcgatgaa acttctgaag atgataaaaa 3181 gcagagcaaa aagggaactg aagaaaaaaa gaaaccttca gactttaaga aaaaagtaat 3241 taaaatggaa caacagtatg aatcttcatc tgatggcact gaaaagttac ctgagcgaga 3301 agaaatttgt cattttccta agggcataaa acaaattaag aatggaacaa ctgatggaga 3361 aaagaaaagt aaaaaaataa gagataaaac ttctaaaaag aaggatgaat tatctgatta 3421 tgctgagaag tcaacaggga aaggagatag ttgtgactct tcagaggata aaaagagtaa |
| 3481 gaatggagca tatggtagag agaagaaaag gtgcaagttg cttggaaaga gttcaaggaa 3541 gagacaagat tgttcatcat ctgatactga gaaatattcc atgaaagaag atggttgtaa 3601 ctcttctgat aagagactga aaagaataga attgagggaa agaagaaatt taagttcaaa 3661 gagaaatact aaggaaatac aaagtggctc atcatcatct gatgctgagg aaagttctga 3721 agataataaa aagaagaagc aaagaacttc atctaaaaag aaggcagtca ttgtcaagga 3781 gaaaaagaga aactccctaa gaacaagcac taaaaggaag caagctgaca ttacatcctc 3841 atcttcttct gatatagaag atgatgatca gaattctata ggtgagggaa gcagcgatga 3901 acagaaaatt aagcctgtga ctgaaaattt agtgctgtct tcacatactg gattttgcca 3961 atcttcagga gatgaagcct tatctaaatc agtgcctgtc acagtggatg atgatgatga 4021 cgacaatgat cctgagaata gaattgccaa gaagatgctt ttagaagaaa ttaaagccaa 4081 tctttcctct gatgaggatg gatcttcaga tgatgagcca gaagaaggga aaaaaagaac 4141 tggaaaacaa aatgaagaaa acccaggaga tgaggaagca aaaaatcaag tcaattctga 4201 atcagattca gattctgaag aatctaagaa gccaagatac agacataggc ttttgcggca 4261 caaattgact gtgagtgacg gagaatctgg agaagaaaaa aagacaaagc ctaaagagca 4321 taaagaagtc aaaggcagaa acagaagaaa ggtgagcagt gaagattcag aagattctga 4381 ttttcaggaa tcaggagtta gtgaagaagt tagtgaatcc gaagatgaac agcggcccag 4441 aacaaggtct gcaaagaaag cagagttgga agaaaatcag cggagctata aacagaaaaa 4501 gaaaaggcga cgtattaagg ttcaagaaga ttcatccagt gaaaacaaga gtaattctga 4561 ggaagaagag gaggaaaaag aagaggagga ggaagaggag gaggaggagg aagaggagga 4621 ggaagatgaa aatgatgatt ccaagtctcc tggaaaaggc agaaagaaaa ttcggaagat 4681 tcttaaagat gataaactga gaacagaaac acaaaatgct cttaaggaag aggaagagag 4741 acgaaaacgt attgctgaga gggagcgtga gcgagaaaaa ttgagagagg tgatagaaat 4801 tgaagatgct tcacccacca agtgtccaat aacaaccaag ttggttttag atgaagatga 4861 agaaaccaaa gaacctttag tgcaggttca tagaaatatg gttatcaaat tgaaacccca 4921 tcaagtagat ggtgttcagt ttatgtggga ttgctgctgt gagtctgtga aaaaaacaaa 4981 gaaatctcca ggttcaggat gcattcttgc ccactgtatg ggccttggta agactttaca 5041 ggtggtaagt tttcttcata cagttctttt gtgtgacaaa ctggatttca gcacggcgtt 5101 agtggtttgt cctcttaata ctgctttgaa ttggatgaat gaatttgaga agtggcaaga 5161 gggattaaaa gatgatgaga agcttgaggt ttctgaatta gcaactgtga aacgtcctca 5221 ggagagaagc tacatgctgc agaggtggca agaagatggt ggtgttatga tcataggcta 5281 tgagatgtat agaaatcttg ctcaaggaag gaatgtgaag agtcggaaac ttaaagaaat 5341 atttaacaaa gctttggttg atccaggccc tgattttgtt gtttgtgatg aaggccatat 5401 tctaaaaaat gaagcatctg ctgtttctaa agctatgaat tctatacgat caaggaggag 5461 gattatttta acaggaacac cacttcaaaa taacctaatt gagtatcatt gtatggttaa 5521 ttttatcaag gaaaatttac ttggatccat taaggagttc aggaatagat ttataaatcc 5581 aattcaaaat ggtcagtgtg cagattctac catggtagat gtcagagtga tgaaaaaacg 5641 tgctcacatt ctctatgaga tgttagctgg atgtgttcag aggaaagatt atacagcatt 5701 aacaaaattc ttgcctccaa aacacgaata tgtgttagct gtgagaatga cttctattca 5761 gtgcaagctc tatcagtact acttagatca cttaacaggt gtgggcaata atagtgaagg 5821 tggaagagga aaggcaggtg caaagctttt ccaagatttt cagatgttaa gtagaatatg 5881 gactcatcct tggtgtttgc agctagacta cattagcaaa gaaaataagg gttattttga 5941 tgaagacagt atggatgaat ttatagcctc agattctgat gaaacctcca tgagtttaag 6001 ctccgatgat tatacaaaaa agaagaaaaa agggaaaaag gggaaaaaag atagtagctc 6061 aagtggaagt ggcagtgaca atgatgttga agtgattaag gtctggaatt caagatctcg 6121 gggaggtggt gaaggaaatg tggatgaaac aggaaacaat ccttctgttt ctttaaaact 6181 ggaagaaagt aaagctactt cttcttctaa tccaagcagc ccagctccag actggtacaa 6241 agattttgtt acagatgctg atgctgaggt tttagagcat tctgggaaaa tggtacttct 6301 ctttgaaatt cttcgaatgg cagaggaaat tggggataaa gtccttgttt tcagccagtc 6361 cctcatatct ctggacttga ttgaagattt tcttgaatta gctagtaggg agaagacaga 6421 agataaagat aaacccctta tttataaagg tgaggggaag tggcttcgaa acattgacta 6481 ttaccgttta gatggttcca ctactgcaca gtcaaggaag aagtgggctg aagaatttaa 6541 tgatgaaact aatgtgagag gacgattatt tatcatttct actaaagcag gatctctagg 6601 aattaatctg gtagctgcta atcgagtaat tatattcgac gcttcttgga atccatctta 6661 tgacatccag agtatattca gagtttatcg ctttggacaa actaagcctg tttatgtata 6721 taggttctta gctcaggaaa ccatggaaga taagatttat gatcggcaag taactaagca 6781 gtcactgtct tttcgagttg ttgatcagca gcaggtggag cgtcatttta ctatgaatga 6841 gcttactgaa ctttatactt ttgagccaga cttattagat gaccctaatt cagaaaagaa 6901 gaagaagagg gatactccca tgctgccaaa ggataccata cttgcagagc tccttcagat 6961 acataaagaa cacattgtag gataccatga acatgattct cttttggacc acaaagaaga 7021 agaagagttg actgaagaag aaagaaaagc agcttgggct gagtatgaag cagagaagaa |
| 7081 gggactgacc atgcgtttca acataccaac tgggaccaat ttaccccctg tcagtttcaa 7141 ctctcaaact ccttatattc ctttcaattt gggagccctg tcagcaatga gtaatcaaca 7201 gctggaggac ctcattaatc aaggaagaga aaaagttgta gaagcaacaa acagtgtgac 7261 agcagtgagg attcaacctc ttgaggatat aatttcagct gtatggaagg agaacatgaa 7321 tctctcagag gcccaagtac aggcgttagc attaagtaga caagccagcc aggagcttga 7381 tgttaaacga agagaagcaa tctacaatga tgtattgaca aaacaacaga tgttaatcag 7441 ctgtgttcag cgaatactta tgaacagaag gctccagcag cagtacaatc agcagcaaca 7501 gcaacaaatg acttatcaac aagcaacact gggtcacctc atgatgccaa agcccccaaa 7561 tttgatcatg aatccttcta actaccagca gattgatatg agaggaatgt atcagccagt 7621 ggctggtggt atgcagccac caccattaca gcgtgcacca cccccaatga gaagcaaaaa 7681 tccaggacct tcccaaggga aatcaatgtg attttgcact aaaagcttaa tggattgtta 7741 aaatcataga aagatctttt atttttttag gaatcaatga cttaacagaa ctcaactgta 7801 taaatagttt ggtcccctta aatgccaatc ttccatatta gttttacttt tttttttttt 7861 aaatagggca taccatttct tcctgacatt tgtcagtgat gttgcctaga atcttcttac 7921 acacgctgag tacagaagat atttcaaatt gttttcagtg aaaacaagtc cttccataat 7981 agtaacaact ccacagattt cctctctaaa tttttatgcc tgcttttagc aaccataaaa 8041 ttgtcataaa attaataaat ttaggaaaga ataaagattt atatattcat tctttacata 8101 taaaaacaca cagctgagtt cttagagttg attcctcaag ttatgaaata cttttgtact 8161 taatccattt cttgattaaa gtgattgaaa tggttttaat gttcttttga ctgaagtctg 8221 aaactgggct cctgctttat tgtctctgtg actgaaagtt agaaactgag ggttatcttt 8281 gacacagaat tgtgtgcaat attcttaaat actactgctc taaaagttgg agaagtcttg 8341 cagttatctt agcattgtat aaacagcctt aagtatagcc taagaagaga attccttttt 8401 cttctttagt ccttctgcca ttttttattt tcagttatat gtgctgaaat aattactggt 8461 aaaatttcag ggttgtggat tatcttccac acatgaattt tctctctcct ggcacgaata 8521 taaagcacat ctcttaactg catggtgcca gtgctaatgc ttcatcctgt tgctggcagt 8581 gggatgtgga cttagaaaat caagttctag cattttagta ggttaacact gaagttgtgg 8641 ttgttaggtt cacaccctgt tttataaaca acatcaaaat ggcagaacca ttgctgactt 8701 taggttcaca tgaggaatgt acttttaaca attcccagta ctatcagtat tgtgaaataa 8761 ttcctctgaa agataagaat cactggcttc tatgcgcttc ttttctctca tcatcatgtt 8821 cttttacccc agtttcctta cattttttta aattgtttca gagtttgttt tttttttagt 8881 ttagattgtg aggcaattat taaatcaaaa ttaattcatc caatacccct ttactagaag 8941 ttttactaga aaatgtatta cattttattt tttcttaatc cagttctgca aaaatgacct 9001 ataaatttat tcatgtacaa ttttggttac ttgaattgtt aaagaaaaca ttgtttttga 9061 ctatgggagt caactcaaca tggcagaacc atttttgaga tgatgataca acaggtagtg 9121 aaacagctta agaattccaa aaaaaaaaaa aaaaaaaaaa aaaagaaaac tgggtttggg 9181 ctttgcttta ggtatcactg gattagaatg agtttaacat tagctaaaac tgctttgagt 9241 tgtttggatg attaagagat tgccattttt atcttggaag aactagtggt aaaacatcca 9301 agagcactag gattgtgata cagaatttgt gaggtttggt ggatccacgc ccctctcccc 9361 cactttccca tgatgaaata tcactaataa atcctgtata tttagatatt atgctagcca 9421 tgtaatcaga tttatttaat tgggtggggc aggtgtgtat ttactttaga aaaaatgaaa 9481 aagacaagat ttatgagaaa tatttgaagg cagtacactc tggccaactg ttaccagttg 9541 gtatttctac aagttcagaa tattttaaac ctgatttact agacctggga attttcaaca 9601 tggtctaatt atttactcaa agacatagat gtgaaaattt taggcaacct tctaaatctt 9661 tttcaccatg gatgaaacta taacttaaag aataatactt agaaggttta attggaaatc 9721 agagtttgaa ataaaacttg gaccactttg tatacactct tctcacttga cattttagct 9781 atataatatg tactttgagt ataacatcaa gctttaacaa atatttaaag acaaaaaaat 9841 cacgtcagta aaatactaaa aggctcattt ttatatttgt tttagatgtt ttaaatagtt 9901 gcaatggatt aaaaatgatg atttaaaatg ttgcttgtaa tacagttttg cctgctaaat 9961 tctccacatt ttgtaacctg ttttatttct ttgggtgtaa agcgtttttg cttagtattg 10021 tgatattgta tatgttttgt cccagttgta tagtaatgtt tcagtccatc atccagcttt 10081 ggctgctgaa atcatacagc tgtgaagact tgcctttgtt tctgttagac tgcttttcag 10141 ttctgtattg agtatcttaa gtactgtaga aaagatgtca cttcttcctt taaggctgtt 10201 ttgtaatata tataaggact ggaattgtgt ttttaaagaa aagcattcaa gtatgacaat 10261 atactatctg tgttttcacc attcaaagtg ctgtttagta gttgaaactt aaactattta 10321 atgtcattta ataaagtgac caaaatgtgt tgtgctcttt attgtatttt cacagctttg 10381 aaaatctgtg cacatactgt ttcatagaaa atgtatagct tttgttgtcc tatataatgg 10441 tggttctttt gcacatttag ttatttaata ttgagaggtc acgaagtttg gttattgaat 10501 ctgttatata ctaaattctg taaaggagaa tctctcatct caaaaagaat ttacatacca 10561 ggaagtccat gtgtgtttgt gttagttttg gatgtctttg tgtaatccag ccccatttcc 10621 tgtttcccaa cagctgtaac actcatttta agtcaagcag ggctaccaac ccacacttga |
| 10681 tagaaaagct gcttaccatt cagaagcttc cttattacct ggcctccaaa tgagctgaat 10741 attttgtagc cttcccttag ctatgttcat tttccctcca ttatcataaa atcagatcga 10801 tatttatgtg ccccaaacaa aactttaaga gcagttacat tctgtcccag tagcccttgt 10861 ttcctttgag agtagcatgt tgtgaggcta tagagactta ttctaccagt aaaacaggtc 10921 aatcctttta catgtttatt atactaaaaa ttatgttcag ggtatttact actttatttc 10981 accagactca gtctcaagtg acttggctat ctccaaatca gatctaccct tagagaataa 11041 acatttttct accgttattt tttttcaagt ctataatctg agccagtccc aaaggagtga 11101 tcaagtttca gaaatgcttt catcttcaca acattttata tatactatta tatggggtga 11161 ataaagtttt aaatccgaaa tataaaaaaa aaaaaaaaaa aa |

| |
|---|
| Homo sapiens alpha thalassemia/mental retardation syndrome X-linked (ATRX) isoform 2 (SEQ ID NO: 7) |
| 1 mtaepmsesk lntlvqklhd flahsseese etsspprlam nqntdkisgs gsnsdmmens 61 keegtsssek skssgssrsk rkpsivtkyv esddekpldd etvnedasne nsenditmqs 121 lpkedglhgi vsctacgqqv nhfqkdsiyr hpslqvlick ncfkyymsdd isrdsdgmde 181 qcrwcaeggn liccdfchna fckkcilrnl grkelstimd ennqwycyic hpeplldlvt 241 acnsvfenle qllqqnkkki kvdseksnkv yehtsrfspk ktssncngee kklddscsgs 301 vtysysaliv pkemikkakk liettanmns syvkflkqat dnseissatk lrqlkafksv 361 ladikkahla leedlnsefr amdavnkekn tkehkvidak fetkarkgek pcalekkdis 421 kseaklsrkq vdsehmhqnv pteeqrtnks tggehkksdr keepqyepan tsedldmdiv 481 svpssvpedi fenletamev qssvdhqgdg ssgteqeves ssvklnissk dnrggikskt 541 takvtkelyv kltpvslsns pikgadcqev pqdkdgyksc glnpklekcg lgqensdneh 601 lvenevslll eesdlrrspr vkttplrrpt etnpvtsnsd eecnetvkek qklsvpvrkk 661 dkrnssdsai dnpkpnklpk skqsetvdqn sdsdemlail kevsrmshss ssdtdineih 721 tnhktlydlk tqagkddkgk rkrksstsgs dfdtkkgksa kssiiskkkr qtqsessnyd 781 selekeiksm skigaarttk kripntkdfd ssedekhskk gmdnqghknl ktsqegssdd 841 aerkqeretf ssaegtvdkd ttimelrdrl pkkqqasast dgvdklsgke qsftslevrk 901 vaetkekskh lktktckkvq dglsdiaekf lkkdqsdets eddkkqskkg teekkkpsdf 961 kkkvikmeqq yesssdgtek lpereeichf pkgikqikng ttdgekkskk irdktskkkd 1021 elsdyaekst gkgdscdsse dkksknqayg rekkrcklig kssrkrqdcs ssdtekysmk 1081 edgcnssdkr lkrielrerr nlsskrntke iqsgssssda eessednkkk kqrtsskkka 1141 vivkekkrns lrtstkrkqa ditsssssdi edddqnsige gssdeqkikp vtenlvlssh 1201 tgfcqssgde alsksvpvtv ddddddndpe nriakkmlle eikanlssde dgssddepee 1261 gkkrtgkqne enpgdeeakn qvnsesdsds eeskkpryrh rllrhkltvs dgesgeekkt 1321 kpkehkevkg rnrrkvssed sedsdfqesg vseevsesed eqrprtrsak kaeleenqrs 1381 ykqkkkrrri kvqedsssen ksnseeeeee keeeeeeeee eeeeeedend dskspgkgrk 1441 kirkilkddk lrtetqnalk eeeerrkria erererekl r evieiedasp tkcpittklv 1501 ldedeetkep lvqvhrnmvi klkphqvdgv qfmwdccces vkktkkspgs gcilahcmgl 1561 gktlqvvsfl htvllcdkld fstalvvcpl ntalnwmnef ekwqeglkdd eklevselat 1621 vkrpqersym lqrwqedggv miigyemyrn laqgrnvksr klkeifnkal vdpgpdfvvc 1681 deghilknea savskamnsi rsrrriiltg tplqnnliey hcmvnfiken llgsikefrn 1741 rfinpiqngq cadstmvdvr vmkkrahily emlagcvqrk dytaltkflp pkheyvlavr 1801 mtsiqcklyq yyldhltgvg nnseggrgka gaklfqdfqm lsriwthpwc lqldyisken 1861 kgyfdedsmd efiasdsdet smslssddyt kkkkkgkkgk kdssssgsgs dndvevikvw 1921 nsrsrgggeg nvdetgnnps vslkleeska tsssnpsspa pdwykdfvtd adaevlehsg 1981 kmvllfeilr maeeigdkvl vfsqslisld liedflelas rektedkdkp liykgegkwl 2041 rnidyyrldg sttaqsrkkw aeefndetnv rgrlfiistk agslginlva anrviifdas 2101 wnpsydiqsi frvyrfgqtk pvyvyrflaq gtmedkiydr qvtkqslsfr vvdqqqverh 2161 ftmneltely tfepdllddp nsekkkkrdt pmlpkdtila ellqihkehi vgyhehdsll 2221 dhkeeeelte eerkaawaey eaekkgltmr fniptgtnlp pvsfnsqtpy ipfnlgalsa 2281 msnqqledli nqgrekvvea tnsvtavriq plediisavw kenmnlseaq vqalalsrqa 2341 sqeldvkrre aiyndvltkq qmliscvqri lmnrrlqqqy nqqqqqqmty qqatlghlmm 2401 pkppnlimnp snyqqidmrg myqpvaggmq ppplqrappp mrsknpgpsq gksm |

| |
|---|
| Homo sapiens alpha thalassemia/mental retardation syndrome X-linked (ATRX), transcript variant 2, mRNA (SEQ ID NO: 8) |
| 1 aattctcctg cctgagcctc ggcccaacaa aatggcggcg gcagcggtgt cgctttgttt 61 ccgcggctcc tgcggcggtg gcagtggtag cggcctttga gctgtgggga ggttccagca 121 gcagctacag tgacgactaa gactccagtg catttctatc gtaaccgggc gcgggggagc 181 gcagatcggc gcccagcaat cacagaagcc gacaaggcgt tcaagcgaaa acatgaccgc 241 tgagcccatg agtgaaagca agttgaatac attggtgcag aagcttcatg acttccttgc 301 acactcatca gaagaatctg aagaaacaag ttctcctcca cgacttgcaa tgaatcaaaa 361 cacagataaa atcagtggtt ctggaagtaa ctctgatatg atggaaaaca gcaaggaaga 421 gggaactagc tcttcagaaa aatccaagtc ttcaggatcg tcacgatcaa agaggaaacc 481 ttcaattgta acaaagtatg tagaatcaga tgatgaaaaa cctttggatg atgaaactgt 541 aaatgaagat gcgtctaatg aaaattcaga aaatgatatt actatgcaga gcttgccaaa 601 agaagatggg cttcatggga ttgtgagctg cactgcttgt ggacaacagg tcaatcattt 661 tcaaaaagat tccatttata gacacccttc attgcaagtt cttatttgta agaattgctt 721 taagtattac atgagtgatg atattagccg tgactcagat ggaatggatg aacaatgtag 781 gtggtgtgcg gaaggtggaa acttgatttg ttgtgacttt tgccataatg ctttctgcaa 841 gaaatgcatt ctacgcaacc ttggtcgaaa ggagttgtcc acaataatgg atgaaaacaa 901 ccaatggtat tgctacattt gtcacccaga gcctttgttg gacttggtca ctgcatgtaa 961 cagcgtattt gagaatttag aacagttgtt gcagcaaaat aagaagaaga taaaagttga 1021 cagtgaaaag agtaataaag tatatgaaca tacatccaga ttttctccaa agaagactag 1081 ttcaaattgt aatggagaag aaaagaaatt agatgattcc tgttctggct ctgtaaccta 1141 ctcttattcc gcactaattg tgcccaaaga gatgattaag aaggcaaaaa aactgattga 1201 gaccacagcc aacatgaact ccagttatgt taaattttta aagcaggcaa cagataattc 1261 agaaatcagt tctgctacaa aattacgtca gcttaaggct tttaagtctg tgttggctga 1321 tattaagaag gctcatcttg cattggaaga agacttaaat tccgagtttc gagcgatgga 1381 tgctgtaaac aaagagaaaa ataccaaaga gcataaagtc atagatgcta agtttgaaac 1441 aaaagcacga aaaggagaaa aaccttgtgc tttggaaaag aaggatattt caaagtcaga 1501 agctaaactt tcaagaaaac aggtagatag tgagcacatg catcagaatg ttccaacaga 1561 ggaacaaaga acaaataaaa gtaccggtgg tgaacataag aaatctgata gaaaagaaga 1621 acctcaatat gaacctgcca acacttctga agatttagac atggatattg tgtctgttcc 1681 ttcctcagtt ccagaagaca tttttgagaa tcttgagact gctatggaag ttcagagttc 1741 agttgatcat caaggggatg gcagcagtgg aactgaacaa gaagtggaga gttcatctgt 1801 aaaattaaat atttcttcaa aagacaacag aggaggtatt aaatcaaaaa ctacagctaa 1861 agtaacaaaa gaattatatg ttaaactcac tcctgtttcc ctttctaatt ccccaattaa 1921 aggtgctgat tgtcaggaag ttccacaaga taaagatggc tataaaagtt gtggtctgaa 1981 ccccaagtta gagaaatgtg gacttggaca ggaaaacagt gataatgagc atttggttga 2041 aaatgaagtt tcattacttt tagaggaatc tgatcttcga agatccccac gtgtaaagac 2101 tacacccttg aggcgaccga cagaaactaa ccctgtaaca tctaattcag atgaagaatg 2161 taatgaaaca gttaaggaga aacaaaaact atcagttcca gtgagaaaaa aggataagcg 2221 taattcttct gacagtgcta tagataatcc taagcctaat aaattgccaa aatctaagca 2281 atcagagact gtggatcaaa attcagattc tgatgaaatg ctagcaatcc tcaaagaggt 2341 gagcaggatg agtcacagtt cttcttcaga tactgatatt aatgaaattc atacaaacca 2401 taagactttg tatgatttaa agactcaggc ggggaaagat gataaaggaa aaaggaaacg 2461 aaaaagttct acatctggct cagattttga tactaaaaag ggcaaatcag ctaagagctc 2521 tataatttct aaaaagaaac gacaaaccca gtctgagtct tctaattatg actcagaatt 2581 agaaaaagag ataaagagca tgagtaaaat tggtgctgcc agaaccacca aaaaaagaat 2641 tccaaataca aaagattttg actcttctga agatgagaaa cacagcaaaa aaggaatgga 2701 taatcaaggg cacaaaaatt tgaagacctc acaagaagga tcatctgatg atgctgaaag 2761 aaaacaagag agagagactt tctcttcagc agaaggcaca gttgataaag acacgaccat 2821 catggaatta agagatcgac ttcctaagaa gcagcaagca agtgcttcca ctgatggtgt 2881 cgataagctt tctgggaaag agcagagttt tacttctttg gaagttagaa aagttgctga 2941 aactaaagaa aagagcaagc atctcaaaac caaaacatgt aaaaaagtac aggatggctt 3001 atctgatatt gcagagaaat tcctaaagaa agaccagagc gatgaaactt ctgaagatga 3061 taaaaagcag agcaaaaagg gaactgaaga aaaaaagaaa ccttcagact ttaagaaaaa 3121 agtaattaaa atggaacaac agtatgaatc ttcatctgat ggcactgaaa agttacctga 3181 gcgagaagaa atttgtcatt ttcctaaggg cataaaacaa attaagaatg gaacaactga 3241 tggagaaaag aaaagtaaaa aaataagaga taaaacttct aaaaagaagg atgaattatc 3301 tgattatgct gagaagtcaa cagggaaagg agatagttgt gactcttcag aggataaaaa 3361 gagtaagaat ggagcatatg gtagagagaa gaaaaggtgc aagttgcttg gaaagagttc 3421 aaggaagaga caagattgtt catcatctga tactgagaaa tattccatga aagaagatgg |
| 3481 ttgtaactct tctgataaga gactgaaaag aatagaattg agggaaagaa gaaatttaag 3541 ttcaaagaga aatactaagg aaatacaaag tggctcatca tcatctgatg ctgaggaaag 3601 ttctgaagat aataaaaaga agaagcaaag aacttcatct aaaaagaagg cagtcattgt 3661 caaggagaaa aagagaaact ccctaagaac aagcactaaa aggaagcaag ctgacattac 3721 atcctcatct tcttctgata tagaagatga tgatcagaat tctataggtg agggaagcag 3781 cgatgaacag aaaattaagc ctgtgactga aaatttagtg ctgtcttcac atactggatt 3841 ttgccaatct tcaggagatg aagccttatc taaatcagtg cctgtcacag tggatgatga 3901 tgatgacgac aatgatcctg agaatagaat tgccaagaag atgcttttag aagaaattaa 3961 agccaatctt tcctctgatg aggatggatc ttcagatgat gagccagaag aagggaaaaa 4021 aagaactgga aaacaaaatg aagaaaaccc aggagatgag gaagcaaaaa atcaagtcaa 4081 ttctgaatca gattcagatt ctgaagaatc taagaagcca agatacagac ataggctttt 4141 gcggcacaaa ttgactgtga gtgacggaga atctggagaa gaaaaaaaga caaagcctaa 4201 agagcataaa gaagtcaaag gcagaaacag aagaaaggtg agcagtgaag attcagaaga 4261 ttctgatttt caggaatcag gagttagtga agaagttagt gaatccgaag atgaacagcg 4321 gcccagaaca aggtctgcaa agaaagcaga gttggaagaa aatcagcgga gctataaaca 4381 gaaaaagaaa aggcgacgta ttaaggttca agaagattca tccagtgaaa acaagagtaa 4441 ttctgaggaa gaagaggagg aaaaagaaga ggaggaggaa gaggaggagg aggaggaaga 4501 ggaggaggaa gatgaaaatg atgattccaa gtctcctgga aaaggcagaa agaaaattcg 4561 gaagattctt aaagatgata aactgagaac agaaacacaa aatgctctta aggaagagga 4621 agagagacga aaacgtattg ctgagaggga gcgtgagcga gaaaaattga gagaggtgat 4681 agaaattgaa gatgcttcac ccaccaagtg tccaataaca accaagttgg ttttagatga 4741 agatgaagaa accaaagaac ctttagtgca ggttcataga aatatggtta tcaaattgaa 4801 accccatcaa gtagatggtg ttcagtttat gtgggattgc tgctgtgagt ctgtgaaaaa 4861 aacaaagaaa tctccaggtt caggatgcat tcttgcccac tgtatgggcc ttggtaagac 4921 tttacaggtg gtaagttttc ttcatacagt tcttttgtgt gacaaactgg atttcagcac 4981 ggcgttagtg gtttgtcctc ttaatactgc tttgaattgg atgaatgaat ttgagaagtg 5041 gcaagaggga ttaaaagatg atgagaagct tgaggtttct gaattagcaa ctgtgaaacg 5101 tcctcaggag agaagctaca tgctgcagag gtggcaagaa gatggtggtg ttatgatcat 5161 aggctatgag atgtatagaa atcttgctca aggaaggaat gtgaagagtc ggaaacttaa 5221 agaaatattt aacaaagctt tggttgatcc aggccctgat tttgttgttt gtgatgaagg 5281 ccatattcta aaaaatgaag catctgctgt ttctaaagct atgaattcta tacgatcaag 5341 gaggaggatt attttaacag gaacaccact tcaaaataac ctaattgagt atcattgtat 5401 ggttaatttt atcaaggaaa atttacttgg atccattaag gagttcagga atagatttat 5461 aaatccaatt caaaatggtc agtgtgcaga ttctaccatg gtagatgtca gagtgatgaa 5521 aaaacgtgct cacattctct atgagatgtt agctggatgt gttcagagga aagattatac 5581 agcattaaca aaattcttgc ctccaaaaca cgaatatgtg ttagctgtga gaatgacttc 5641 tattcagtgc aagctctatc agtactactt agatcactta acaggtgtgg gcaataatag 5701 tgaaggtgga agaggaaagg caggtgcaaa gcttttccaa gattttcaga tgttaagtag 5761 aatatggact catccttggt gtttgcagct agactacatt agcaaagaaa ataagggtta 5821 ttttgatgaa gacagtatgg atgaatttat agcctcagat tctgatgaaa cctccatgag 5881 tttaagctcc gatgattata caaaaaagaa gaaaaaaggg aaaaagggga aaaaagatag 5941 tagctcaagt ggaagtggca gtgacaatga tgttgaagtg attaaggtct ggaattcaag 6001 atctcgggga ggtggtgaag gaaatgtgga tgaaacagga aacaatcctt ctgtttcttt 6061 aaaactggaa gaaagtaaag ctacttcttc ttctaatcca agcagcccag ctccagactg 6121 gtacaaagat tttgttacag atgctgatgc tgaggtttta gagcattctg ggaaaatggt 6181 acttctcttt gaaattcttc gaatggcaga ggaaattggg gataaagtcc ttgttttcag 6241 ccagtccctc atatctctgg acttgattga agattttctt gaattagcta gtagggagaa 6301 gacagaagat aaagataaac cccttattta taaaggtgag gggaagtggc ttcgaaacat 6361 tgactattac cgtttagatg gttccactac tgcacagtca aggaagaagt gggctgaaga 6421 atttaatgat gaaactaatg tgagaggacg attatttatc atttctacta aagcaggatc 6481 tctaggaatt aatctggtag ctgctaatcg agtaattata ttcgacgctt cttggaatcc 6541 atcttatgac atccagagta tattcagagt ttatcgcttt ggacaaacta agcctgttta 6601 tgtatatagg ttcttagctc agggaaccat ggaagataag atttatgatc ggcaagtaac 6661 taagcagtca ctgtcttttc gagttgttga tcagcagcag gtggagcgtc attttactat 6721 gaatgagctt actgaacttt atacttttga gccagactta ttagatgacc ctaattcaga 6781 aaagaagaag aagagggata ctcccatgct gccaaaggat accatacttg cagagctcct 6841 tcagatacat aaagaacaca ttgtaggata ccatgaacat gattctcttt tggaccacaa 6901 agaagaagaa gagttgactg aagaagaaag aaaagcagct tgggctgagt atgaagcaga 6961 gaagaaggga ctgaccatgc gtttcaacat accaactggg accaatttac cccctgtcag 7021 tttcaactct caaactcctt atattccttt caatttggga gccctgtcag caatgagtaa |
| 7081 tcaacagctg gaggacctca ttaatcaagg aagagaaaaa gttgtagaag caacaaacag 7141 tgtgacagca gtgaggattc aacctcttga ggatataatt tcagctgtat ggaaggagaa 7201 catgaatctc tcagaggccc aagtacaggc gttagcatta agtagacaag ccagccagga 7261 gcttgatgtt aaacgaagag aagcaatcta caatgatgta ttgacaaaac aacagatgtt 7321 aatcagctgt gttcagcgaa tacttatgaa cagaaggctc cagcagcagt acaatcagca 7381 gcaacagcaa caaatgactt atcaacaagc aacactgggt cacctcatga tgccaaagcc 7441 cccaaatttg atcatgaatc cttctaacta ccagcagatt gatatgagag gaatgtatca 7501 gccagtggct ggtggtatgc agccaccacc attacagcgt gcaccacccc caatgagaag 7561 caaaaatcca ggaccttccc aagggaaatc aatgtgattt tgcactaaaa gcttaatgga 7621 ttgttaaaat catagaaaga tcttttattt ttttaggaat caatgactta acagaactca 7681 actgtataaa tagtttggtc cccttaaatg ccaatcttcc atattagttt tacttttttt 7741 ttttttaaat agggcatacc atttcttcct gacatttgtc agtgatgttg cctagaatct 7801 tcttacacac gctgagtaca gaagatattt caaattgttt tcagtgaaaa caagtccttc 7861 cataatagta acaactccac agatttcctc tctaaatttt tatgcctgct tttagcaacc 7921 ataaaattgt cataaaatta ataaatttag gaaagaataa agatttatat attcattctt 7981 tacatataaa aacacacagc tgagttctta gagttgattc ctcaagttat gaaatacttt 8041 tgtacttaat ccatttcttg attaaagtga ttgaaatggt tttaatgttc ttttgactga 8101 agtctgaaac tgggctcctg ctttattgtc tctgtgactg aaagttagaa actgagggtt 8161 atctttgaca cagaattgtg tgcaatattc ttaaatacta ctgctctaaa agttggagaa 8221 gtcttgcagt tatcttagca ttgtataaac agccttaagt atagcctaag aagagaattc 8281 ctttttcttc tttagtcctt ctgccatttt ttattttcag ttatatgtgc tgaaataatt 8341 actggtaaaa tttcagggtt gtggattatc ttccacacat gaattttctc tctcctggca 8401 cgaatataaa gcacatctct taactgcatg gtgccagtgc taatgcttca tcctgttgct 8461 ggcagtggga tgtggactta gaaaatcaag ttctagcatt ttagtaggtt aacactgaag 8521 ttgtggttgt taggttcaca ccctgtttta taaacaacat caaaatggca gaaccattgc 8581 tgactttagg ttcacatgag gaatgtactt ttaacaattc ccagtactat cagtattgtg 8641 aaataattcc tctgaaagat aagaatcact ggcttctatg cgcttctttt ctctcatcat 8701 catgttcttt taccccagtt tccttacatt tttttaaatt gtttcagagt ttgttttttt 8761 tttagtttag attgtgaggc aattattaaa tcaaaattaa ttcatccaat acccctttac 8821 tagaagtttt actagaaaat gtattacatt ttattttttc ttaatccagt tctgcaaaaa 8881 tgacctataa atttattcat gtacaatttt ggttacttga attgttaaag aaaacattgt 8941 ttttgactat gggagtcaac tcaacatggc agaaccattt ttgagatgat gatacaacag 9001 gtagtgaaac agcttaagaa ttccaaaaaa aaaaaaaaaa aaaaaaaaaa gaaaactggg 9061 tttgggcttt gctttaggta tcactggatt agaatgagtt taacattagc taaaactgct 9121 ttgagttgtt tggatgatta agagattgcc atttttatct tggaagaact agtggtaaaa 9181 catccaagag cactaggatt gtgatacaga atttgtgagg tttggtggat ccacgcccct 9241 ctcccccact ttcccatgat gaaatatcac taataaatcc tgtatattta gatattatgc 9301 tagccatgta atcagattta tttaattggg tggggcaggt gtgtatttac tttagaaaaa 9361 atgaaaaaga caagatttat gagaaatatt tgaaggcagt acactctggc caactgttac 9421 cagttggtat ttctacaagt tcagaatatt ttaaacctga tttactagac ctgggaattt 9481 tcaacatggt ctaattattt actcaaagac atagatgtga aaattttagg caaccttcta 9541 aatctttttc accatggatg aaactataac ttaaagaata atacttagaa gggttaattg 9601 gaaatcagag tttgaaataa aacttggacc actttgtata cactcttctc acttgacatt 9661 ttagctatat aatatgtact ttgagtataa catcaagctt taacaaatat ttaaagacaa 9721 aaaaatcacg tcagtaaaat actaaaaggc tcatttttat atttgtttta gatgttttaa 9781 atagttgcaa tggattaaaa atgatgattt aaaatgttgc ttgtaataca gttttgcctg 9841 ctaaattctc cacattttgt aacctgtttt atttctttgg gtgtaaagcg tttttgctta 9901 gtattgtgat attgtatatg ttttgtccca gttgtatagt aatgtttcag tccatcatcc 9961 agctttggct gctgaaatca tacagctgtg aagacttgcc tttgtttctg ttagactgct 10021 tttcagttct gtattgagta tcttaagtac tgtagaaaag atgtcacttc ttcctttaag 10081 gctgttttgt aatatatata aggactggaa ttgtgttttt aaagaaaagc attcaagtat 10141 gacaatatac tatctgtgtt ttcaccattc aaagtgctgt ttagtagttg aaacttaaac 10201 tatttaatgt catttaataa agtgaccaaa atgtgttgtg ctctttattg tattttcaca 10261 gctttgaaaa tctgtgcaca tactgtttca tagaaaatgt atagcttttg ttgtcctata 10321 taatggtggt tcttttgcac atttagttat ttaatattga gaggtcacga agtttggtta 10381 ttgaatctgt tatatactaa attctgtaaa gggagatctc tcatctcaaa aagaatttac 10441 ataccaggaa gtccatgtgt gtttgtgtta gttttggatg tctttgtgta atccagcccc 10501 atttcctgtt tcccaacagc tgtaacactc attttaagtc aagcagggct accaacccac 10561 acttgataga aaagctgctt accattcaga agcttcctta ttacctggcc tccaaatgag 10621 ctgaatattt tgtagccttc ccttagctat gttcattttc cctccattat cataaaatca |
| 10681 gatcgatatt tatgtgcccc aaacaaaact ttaagagcag ttacattctg tcccagtagc 10741 ccttgtttcc tttgagagta gcatgttgtg aggctataga gacttattct accagtaaaa 10801 caggtcaatc cttttacatg tttattatac taaaaattat gttcagggta tttactactt 10861 tatttcacca gactcagtct caagtgactt ggctatctcc aaatcagatc tacccttaga 10921 gaataaacat ttttctaccg ttattttttt tcaagtctat aatctgagcc agtcccaaag 10981 gagtgatcaa gtttcagaaa tgctttcatc ttcacaacat tttatatata ctattatatg 11041 gggtgaataa agttttaaat ccgaaatata aaaaaaaaaa aaaaaaaa |

A subject in need thereof may have reduced expression, haploinsufficiency, and/or loss of function of ARID1A. For example, a subject may comprise a mutation selected from the group consisting of a nonsense mutation for the wild type residue cysteine (C) at amino acid position 884 of SEQ ID NO: 11 (C884*), a substitution of lysine (K) for the wild type residue glutamic acid (E) at amino acid position 966 (E966K), a nonsense mutation for the wild type residue glutamine (Q) at amino acid position 1411 of SEQ ID NO: 11 (Q1411*), a frame shift mutation at the wild type residue phenylalanine (F) at amino acid position 1720 of SEQ ID NO: 11 (F1720fs), a frame shift mutation after the wild type residue glycine (G) at amino acid position 1847 of SEQ ID NO: 11 (G1847fs), a frame shift mutation at the wild type residue cysteine (C) at amino acid position 1874 of SEQ ID NO: 11 (C1874fs), a substitution of glutamic acid (E) for the wild type residue aspartic acid (D) at amino acid position 1957 (D1957E), a nonsense mutation for the wild type residue glutamine (Q) at amino acid position 1430 of SEQ ID NO: 11 (Q1430*), a frame shift mutation at the wild type residue arginine (R) at amino acid position 1721 of SEQ ID NO: 11 (R1721fs), a substitution of glutamic acid (E) for the wild type residue glycine (G) at amino acid position 1255 (G1255E), a frame shift mutation at the wild type residue glycine (G) at amino acid position 284 of SEQ ID NO: 11 (G284fs), a nonsense mutation for the wild type residue arginine (R) at amino acid position 1722 of SEQ ID NO: 11 (R1722*), a frame shift mutation at the wild type residue methionine (M) at amino acid position 274 of SEQ ID NO: 11 (M274fs), a frame shift mutation at the wild type residue glycine (G) at amino acid position 1847 of SEQ ID NO: 11 (G1847fs), a frame shift mutation at the wild type residue P at amino acid position 559 of SEQ ID NO: 11 (P559fs), a nonsense mutation for the wild type residue arginine (R) at amino acid position 1276 of SEQ ID NO: 11 (R1276*), a frame shift mutation at the wild type residue glutamine (Q) at amino acid position 2176 of SEQ ID NO: 11 (Q2176fs), a frame shift mutation at the wild type residue histidine (H) at amino acid position 203 of SEQ ID NO: 11 (H203fs), a frame shift mutation at the wild type residue alanine (A) at amino acid position 591 of SEQ ID NO: 11 (A591fs), a nonsense mutation for the wild type residue glutamine (Q) at amino acid position 1322 of SEQ ID NO: 11 (Q1322*), a nonsense mutation for the wild type residue serine (S) at amino acid position 2264 of SEQ ID NO: 11 (S2264*), a nonsense mutation for the wild type residue glutamine (Q) at amino acid position 586 of SEQ ID NO: 11 (Q586*), a frame shift mutation at the wild type residue glutamine (Q) at amino acid position 548 of SEQ ID NO: 11 (Q548fs), and a frame shift mutation at the wild type residue asparagine (N) at amino acid position 756 of SEQ ID NO: 11 (N756fs). "*" used herein refers to a stop codon. "fs" used herein refers to a frame shift.

| |
|---|
| AT-rich interactive domain-containing protein 1A (ARID1A) isoform a [Homo sapiens] (SEQ ID NO: 9) |
| 1 maaqvapaaa sslgnppppp pselkkaeqq qreeaggeaa aaaaaergem kaaagqeseg 61 pavgppqplg kelqdgaesn gggggggags gggpgaepdl knsngnagpr palnnnltep 121 pgggggssd gvgapphsaa aalpppaygf ggpygrspsa vaaaaaavfh qqhggqqspg 181 laalqsgggg glepyagpqq nshdhgfpnh qynsyypnrs aypppapaya lssprggtpg 241 sgaaaaagsk pppsssasas sssssfaqqr fgamggggps aagggtpqpt atptlnqllt 301 spssargyqg ypggdysggp qdggagkgpa dmasqcwgaa aaaaaaaaas ggaqqrshha 361 pmspgssggg gqplartpqp sspmdqmgkm rpqpyggtnp ysqqqgppsg pqqghgypgq 421 pygsqtpqry pmtmqgraqs amgglsytqq ippygqqgps gygqqgqtpy ynqqsphpqq 481 qqppysqqpp sqtphaqpsy qqqpqsqppq lqssqppysq qpsqpphqqs papypsqqst 541 tqqhpqsqpp ysqpqaqspy qqqqpqqpap stlsqqaayp qpqsqqsqqt aysqqrfppp 601 qelsqdsfgs qassapsmts skggqedmnl slqsrpsslp dlsgsiddlp mgtegalspg 661 vstsgisssq geqsnpaqsp fsphtsphlp girgpspspv gspasvaqsr sgplspaavp 721 gnqmpprpps gqsdsimhps mnqssiaqdr gymqrnpqmp qysspqpgsa lsprqpsggq 781 ihtgmgsyqq nsmgsygpqg gqygpqggyp rqpnynalpn anypsagmag ginpmgaggq 841 mhgqpgippy gtlppgrmsh asmgnrpygp nmanmppqvg sgmcpppggm nrktqetava 901 mhvaansiqn rppgypnmnq ggmmgtgppy gqginsmagm inpqgppysm ggtmannsag 961 maaspemmgl gdvkltpatk mnnkadgtpk teskskksss stttnekitk lyelggeper 1021 kmwvdrylaf teekamgmtn lpavgrkpld lyrlyvsvke iggltqvnkn kkwrelatnl 1081 nvgtsssaas slkkqyiqcl yafeckierg edpppdifaa adskksqpki qppspagsgs 1141 mqgpqtpqst sssmaeggdl kpptpastph sqipplpgms rsnsvgiqda fndgsdstfq 1201 krnsmtpnpg yqpsmntsdm mgrmsyepnk dpygsmrkap gsdpfmssgq gpnggmgdpy 1261 sraagpglgn vamgprqhyp yggpydrvrt epgigpegnm stgapqpnlm psnpdsgmys 1321 psryppqqqq qqqqrhdsyg nqfstqgtps gspfpsqqtt myqqqqqnyk rpmdgtygpp 1381 akrhegemys vpystgqgqp qqqqlppaqp qpasqqqaaq pspqqdvynq ygnaypatat 1441 aaterrpagg pqnqfpfqfg rdrvsappgt naqqnmppqm mggpiqasae vaqqgtmwqg 1501 rndmtynyan rqstgsapqg payhgvnrtd emlhtdqran hegswpshgt rqppygpsap 1561 vppmtrppps nyqpppsmqn hipqvsspap lprpmenrts pskspflhsg mkmqkagppv 1621 pashiapapv qppmirrdit fppgsveatq pvlkqrrrlt mkdigtpeaw rvmmslksgl 1681 laestwaldt inillyddns imtfnlsqlp gllellveyf rrclieifgi lkeyevgdpg 1741 qrtlldpgrf skvsspapme ggeeeeellg pkleeeeeee vvendeeiaf sgkdkpasen 1801 seekliskfd klpvkivqkn dpfvvdcsdk lgrvqefdsg llhwrigggd ttehiqthfe 1861 sktellpsrp hapcppaprk hvttaegtpg ttdqegpppd gppekritat mddmlstrss 1921 tltedgakss eaikesskfp fgispaqshr nikiledeph skdetplctl ldwqdslakr 1981 cvcvsntirs lsfvpgndfe mskhpgllli lgklillhhk hperkqaplt yekeeeqdqg 2041 vscnkvewww dclemlrent lvtlanisgq ldlspypesi clpvldgllh wavcpsaeaq 2101 dpfstlgpna vlspqrlvle tlsklsiqdn nvdlilatpp fsrleklyst mvrflsdrkn 2161 pvcremavvl lanlaqgdsl aaraiavqkg signllgfle dslaatqfqq sqasllhmqn 2221 ppfeptsvdm mrraaralla lakvdenhse ftlyesrlld isvsplmnsl vsqvicdvlf 2281 ligqs |

| |
|---|
| Homo sapiens AT rich interactive domain 1A (SWI-like) (ARID1A), transcript variant 1, mRNA (SEQ ID NO: 10) |
| 1 cagaaagcgg agagtcacag cggggccagg ccctggggag cggagcctcc accgcccccc 61 tcattcccag gcaagggctt ggggggaatg agccgggaga gccgggtccc gagcctacag 121 agccgggagc agctgagccg ccggcgcctc ggccgccgcc gccgcctcct cctcctccgc 181 cgccgccagc ccggagcctg agccggcggg gcggggggga gaggagcgag cgcagcgcag 241 cagcggagcc ccgcgaggcc cgcccgggcg ggtgggaggg gcagcccggg ggactgggcc 301 ccggggcggg gtgggagggg gggagaagac gaagacaggg ccgggtctct ccgcggacga 361 gacagcgggg atcatggccg cgcaggtcgc ccccgccgcc gccagcagcc tgggcaaccc 421 gccgccgccg ccgccctcgg agctgaagaa agccgagcag cagcagcggg aggaggcggg 481 gggcgaggcg gcggcggcgg cagcggccga gcgcggggaa atgaaggcag ccgccgggca 541 ggaaagcgag ggccccgccg tggggccgcc gcagccgctg ggaaaggagc tgcaggacgg 601 ggccgagagc aatgggggtg gcggcggcgg cggagccggc agcggcggcg ggcccggcgc 661 ggagccggac ctgaagaact cgaacgggaa cgcgggccct aggcccgccc tgaacaataa 721 cctcacggag ccgcccggcg gcggcggtgg cggcagcagc gatggggtgg gggcgcctcc 781 tcactcagcc gcggccgcct tgccgccccc agcctacggc ttcgggcaac cctacggccg 841 gagcccgtct gccgtcgccg ccgccgcggc cgccgtcttc caccaacaac atggcggaca 901 acaaagccct ggcctggcag cgctgcagag cggcggcggc gggggcctgg agccctacgc 961 ggggccccag cagaactctc acgaccacgg cttccccaac caccagtaca actcctacta 1021 ccccaaccgc agcgcctacc ccccgcccgc cccggcctac gcgctgagct ccccgagagg 1081 tggcactccg ggctccggcg cggcggcggc tgccggctcc aagccgcctc cctcctccag 1141 cgcctccgcc tcctcgtcgt cttcgtcctt cgctcagcag cgcttcgggg ccatgggggg 1201 aggcggcccc tccgcggccg gcgggggaac tccccagccc accgccaccc ccaccctcaa 1261 ccaactgctc acgtcgccca gctcggcccg gggctaccag ggctaccccg ggggcgacta 1321 cagtggcggg ccccaggacg ggggcgccgg caagggcccg gcggacatgg cctcgcagtg 1381 ttggggggct gcggcggcgg cagctgcggc ggcggccgcc tcgggagggg cccaacaaag 1441 gagccaccac gcgcccatga gccccgggag cagcggcggc ggggggcagc cgctcgcccg 1501 gacccctcag ccatccagtc caatggatca gatgggcaag atgagacctc agccatatgg 1561 cgggactaac ccatactcgc agcaacaggg acctccgtca ggaccgcagc aaggacatgg 1621 gtacccaggg cagccatacg ggtcccagac cccgcagcgg tacccgatga ccatgcaggg 1681 ccgggcgcag agtgccatgg gcggcctctc ttatacacag cagattcctc cttatggaca 1741 acaaggcccc agcgggtatg gtcaacaggg ccagactcca tattacaacc agcaaagtcc 1801 tcaccctcag cagcagcagc caccctactc ccagcaacca ccgtcccaga cccctcatgc 1861 ccaaccttcg tatcagcagc agccacagtc tcaaccacca cagctccagt cctctcagcc 1921 tccatactcc cagcagccat cccagcctcc acatcagcag tccccggctc catacccctc 1981 ccagcagtcg acgacacagc agcaccccca gagccagccc ccctactcac agccacaggc 2041 tcagtctcct taccagcagc agcaacctca gcagccagca ccctcgacgc tctcccagca 2101 ggctgcgtat cctcagcccc agtctcagca gtcccagcaa actgcctatt cccagcagcg 2161 cttccctcca ccgcaggagc tatctcaaga ttcatttggg tctcaggcat cctcagcccc 2221 ctcaatgacc tccagtaagg gagggcaaga agatatgaac ctgagccttc agtcaagacc 2281 ctccagcttg cctgatctat ctggttcaat agatgacctc cccatgggga cagaaggagc 2341 tctgagtcct ggagtgagca catcagggat ttccagcagc caaggagagc agagtaatcc 2401 agctcagtct cctttctctc ctcatacctc ccctcacctg cctggcatcc gaggcccttc 2461 cccgtcccct gttggctctc ccgccagtgt tgctcagtct cgctcaggac cactctcgcc 2521 tgctgcagtg ccaggcaacc agatgccacc tcggccaccc agtggccagt cggacagcat 2581 catgcatcct tccatgaacc aatcaagcat tgcccaagat cgaggttata tgcagaggaa 2641 cccccagatg ccccagtaca gttcccccca gcccggctca gccttatctc cgcgtcagcc 2701 ttccggagga cagatacaca caggcatggg ctcctaccag cagaactcca tggggagcta 2761 tggtccccag gggggtcagt atggcccaca aggtggctac cccaggcagc caaactataa 2821 tgccttgccc aatgccaact accccagtgc aggcatggct ggaggcataa accccatggg 2881 tgccggaggt caaatgcatg gacagcctgg catcccacct tatggcacac tccctccagg 2941 gaggatgagt cacgcctcca tgggcaaccg gccttatggc cctaacatgg ccaatatgcc 3001 acctcaggtt gggtcaggga tgtgtccccc accagggggc atgaaccgga aaacccaaga 3061 aactgctgtc gccatgcatg ttgctgccaa ctctatccaa aacaggccgc caggctaccc 3121 caatatgaat caagggggca tgatgggaac tggacctcct tatggacaag ggattaatag 3181 tatggctggc atgatcaacc ctcagggacc cccatattcc atgggtggaa ccatggccaa 3241 caattctgca gggatggcag ccagcccaga gatgatgggc cttggggatg taaagttaac 3301 tccagccacc aaaatgaaca acaaggcaga tgggacaccc aagacagaat ccaaatccaa 3361 gaaatccagt tcttctacta caaccaatga gaagatcacc aagttgtatg agctgggtgg 3421 tgagcctgag aggaagatgt gggtggaccg ttatctggcc ttcactgagg agaaggccat |
| 3481 gggcatgaca aatctgcctg ctgtgggtag gaaacctctg gacctctatc gcctctatgt 3541 gtctgtgaag gagattggtg gattgactca ggtcaacaag aacaaaaaat ggcgggaact 3601 tgcaaccaac ctcaatgtgg gcacatcaag cagtgctgcc agctccttga aaaagcagta 3661 tatccagtgt ctctatgcct ttgaatgcaa gattgaacgg ggagaagacc ctcccccaga 3721 catctttgca gctgctgatt ccaagaagtc ccagcccaag atccagcctc cctctcctgc 3781 gggatcagga tctatgcagg ggccccagac tccccagtca accagcagtt ccatggcaga 3841 aggaggagac ttaaagccac caactccagc atccacacca cacagtcaga tccccccatt 3901 gccaggcatg agcaggagca attcagttgg gatccaggat gcctttaatg atggaagtga 3961 ctccacattc cagaagcgga attccatgac tccaaaccct gggtatcagc ccagtatgaa 4021 tacctctgac atgatggggc gcatgtccta tgagccaaat aaggatcctt atggcagcat 4081 gaggaaagct ccagggagtg atcccttcat gtcctcaggg caggccccca acggcgggat 4141 gggtgacccc tacagtcgtg ctgccggccc tgggctagga aatgtggcga tgggaccacg 4201 acagcactat ccctatggag gtccttatga cagagtgagg acggagcctg gaatagggcc 4261 tgagggaaac atgagcactg gggccccaca gccgaatctc atgccttcca acccagactc 4321 ggggatgtat tctcctagcc gctacccccc gcagcagcag cagcagcagc agcaacgaca 4381 tgattcctat ggcaatcagt tctccaccca aggcacccct tctggcagcc ccttccccag 4441 ccagcagact acaatgtatc aacagcaaca gcagaattac aagcggccaa tggatggcac 4501 atatggccct cctgccaagc ggcacgaagg ggagatgtac agcgtgccat acagcactgg 4561 gcaggggcag cctcagcagc agcagttgcc cccagcccag ccccagcctg ccagccagca 4621 acaagctgcc cagccttccc ctcagcaaga tgtatacaac cagtatggca atgcctatcc 4681 tgccactgcc acagctgcta ctgagcgccg accagcaggc ggcccccaga accaatttcc 4741 attccagttt ggccgagacc gtgtctctgc accccctggc accaatgccc agcaaaacat 4801 gccaccacaa atgatgggcg gccccataca ggcatcagct gaggttgctc agcaaggcac 4861 catgtggcag gggcgtaatg acatgaccta taattatgcc aacaggcaga gcacgggctc 4921 tgccccccag ggccccgcct atcatggcgt gaaccgaaca gatgaaatgc tgcacacaga 4981 tcagagggcc aaccacgaag gctcgtggcc ttcccatggc acacgccagc ccccatatgg 5041 tccctctgcc cctgtgcccc ccatgacaag gccccctcca tctaactacc agcccccacc 5101 aagcatgcag aatcacattc ctcaggtatc cagccctgct cccctgcccc ggccaatgga 5161 gaaccgcacc tctcctagca agtctccatt cctgcactct gggatgaaaa tgcagaaggc 5221 aggtccccca gtacctgcct cgcacatagc acctgcccct gtgcagcccc ccatgattcg 5281 gcgggatatc accttcccac ctggctctgt tgaagccaca cagcctgtgt tgaagcagag 5341 gaggcggctc acaatgaaag acattggaac cccggaggca tggcgggtaa tgatgtccct 5401 caagtctggt ctcctggcag agagcacatg ggcattagat accatcaaca tcctgctgta 5461 tgatgacaac agcatcatga ccttcaacct cagtcagctc ccagggttgc tagagctcct 5521 tgtagaatat ttccgacgat gcctgattga gatctttggc attttaaagg agtatgaggt 5581 gggtgaccca ggacagagaa cgctactgga tcctgggagg ttcagcaagg tgtctagtcc 5641 agctcccatg gagggtgggg aagaagaaga agaacttcta ggtcctaaac tagaagagga 5701 agaagaagag gaagtagttg aaaatgatga ggagatagcc ttttcaggca aggacaagcc 5761 agcttcagag aatagtgagg agaagctgat cagtaagttt gacaagcttc cagtaaagat 5821 cgtacagaag aatgatccat ttgtggtgga ctgctcagat aagcttgggc gtgtgcagga 5881 gtttgacagt ggcctgctgc actggcggat tggtgggggg gacaccactg agcatatcca 5941 gacccacttc gagagcaaga cagagctgct gccttcccgg cctcacgcac cctgcccacc 6001 agcccctcgg aagcatgtga caacagcaga gggtacacca gggacaacag accaggaggg 6061 gcccccacct gatggacctc cagaaaaacg gatcacagcc actatggatg acatgttgtc 6121 tactcggtct agcaccttga ccgaggatgg agctaagagt tcagaggcca tcaaggagag 6181 cagcaagttt ccatttggca ttagcccagc acagagccac cggaacatca agatcctaga 6241 ggacgaaccc cacagtaagg atgagacccc actgtgtacc cttctggact ggcaggattc 6301 tcttgccaag cgctgcgtct gtgtgtccaa taccattcga agcctgtcat ttgtgccagg 6361 caatgacttt gagatgtcca aacacccagg gctgctgctc atcctgggca agctgatcct 6421 gctgcaccac aagcacccag aacggaagca ggcaccacta acttatgaaa aggaggagga 6481 acaggaccaa ggggtgagct gcaacaaagt ggagtggtgg tgggactgct tggagatgct 6541 ccgggaaaac accttggtta cactcgccaa catctcgggg cagttggacc tatctccata 6601 ccccgagagc atttgcctgc ctgtcctgga cggactccta cactgggcag tttgcccttc 6661 agctgaagcc caggacccct tttccaccct gggccccaat gccgtccttt ccccgcagag 6721 actggtcttg gaaaccctca gcaaactcag catccaggac aacaatgtgg acctgattct 6781 ggccacaccc cccttcagcc gcctggagaa gttgtatagc actatggtgc gcttcctcag 6841 tgaccgaaag aacccggtgt gccgggagat ggctgtggta ctgctggcca acctggctca 6901 gggggacagc ctggcagctc gtgccattgc agtgcagaag ggcagtatcg gcaacctcct 6961 gggcttccta gaggacagcc ttgccgccac acagttccag cagagccagg ccagcctcct 7021 ccacatgcag aacccaccct ttgagccaac tagtgtggac atgatgcggc gggctgcccg |
| 7081 cgcgctgctt gccttggcca aggtggacga gaaccactca gagtttactc tgtacgaatc 7141 acggctgttg gacatctcgg tatcaccgtt gatgaactca ttggtttcac aagtcatttg 7201 tgatgtactg tttttgattg gccagtcatg acagccgtgg gacacctccc cccccgtgt 7261 gtgtgtgcgt gtgtggagaa cttagaaact gactgttgcc ctttatttat gcaaaaccac 7321 ctcagaatcc agtttaccct gtgctgtcca gcttctccct tgggaaaaag tctctcctgt 7381 ttctctctcc tccttccacc tcccctccct ccatcacctc acgcctttct gttccttgtc 7441 ctcaccttac tcccctcagg accctacccc accctctttg aaaagacaaa gctctgccta 7501 catagaagac tttttttatt ttaaccaaag ttactgttgt ttacagtgag tttggggaaa 7561 aaaaataaaa taaaaatggc tttcccagtc cttgcatcaa cggatgccac catttcataa 7621 ctgtttttaa tggtaaaaaa aaaaaaaaaa aatacaaaaa aaaattctga aggacaaaaa 7681 aggtgactgc tgaactgtgt gtggtttatt gttgtacatt cacaatcttg caggagccaa 7741 gaagttcgca gttgtgaaca gaccctgttc actggagagg cctgtgcagt agagtgtaga 7801 ccctttcatg tactgtactg tacacctgat actgtaaaca tactgtaata ataatgtctc 7861 acatggaaac agaaaacgct gggtcagcag caagctgtag tttttaaaaa tgtttttagt 7921 taaacgttga ggagaaaaaa aaaaaaggct tttcccccaa agtatcatgt gtgaacctac 7981 aacaccctga cctctttctc tcctccttga ttgtatgaat aaccctgaga tcacctctta 8041 gaactggttt taacctttag ctgcagcggc tacgctgcca cgtgtgtata tatatgacgt 8101 tgtacattgc acataccctt ggatccccac agtttggtcc tcctcccagc taccccttta 8161 tagtatgacg agttaacaag ttggtgacct gcacaaagcg agacacagct atttaatctc 8221 ttgccagata tcgcccctct tggtgcgatg ctgtacaggt ctctgtaaaa agtccttgct 8281 gtctcagcag ccaatcaact tatagtttat ttttttctgg gtttttgttt tgttttgttt 8341 tctttctaat cgaggtgtga aaaagttcta ggttcagttg aagttctgat gaagaaacac 8401 aattgagatt ttttcagtga taaaatctgc atatttgtat ttcaacaatg tagctaaaac 8461 ttgatgtaaa ttcctccttt ttttcctttt ttggcttaat gaatatcatt tattcagtat 8521 gaaatcttta tactatatgt tccacgtgtt aagaataaat gtacattaaa tcttggtaag 8581 acttt |

| |
|---|
| AT-rich interactive domain-containing protein 1A (ARID1A) isoform b (SEQ ID NO: 11) |
| 1 maaqvapaaa sslgnppppp pselkkaeqq qreeaggeaa aaaaaergem kaaagqeseg 61 pavgppqplg kelqdgaesn gggggggags gggpgaepdl knsngnagpr palnnnltep 121 pggggggssd gvgapphsaa aalpppaygf gqpygrspsa vaaaaaavfh qqhggqqspg 181 laalqsgggg glepyagpqq nshdhgfpnh qynsyypnrs aypppapaya lssprggtpg 241 sgaaaaagsk pppsssasas sssssfaqqr fgamggggps aagggtpqpt atptlnqllt 301 spssargyqg ypggdysggp qdggagkgpa dmasqcwgaa aaaaaaaaas ggaqqrshha 361 pmspgssggg gqplartpqp sspmdqmgkm rpqpyggtnp ysqqqgppsg pqqghgypgq 421 pygsqtpqry pmtmqgraqs amgglsytqq ippygqqgps gygqqgqtpy ynqqsphpqq 481 qqppysqqpp sqtphaqpsy qqqpqsqppq lqssqppysq qpsqpphqqs papypsqqst 541 tqqhpqsqpp ysqpqaqspy qqqqpqqpap stlsqqaayp qpqsqqsqqt aysqqrfppp 601 qelsqdsfgs qassapsmts skggqedmnl slqsrpsslp dlsgsiddlp mgtegalspg 661 vstsgisssq geqsnpaqsp fsphtsphlp girgpspspv gspasvaqsr sgplspaavp 721 gnqmpprpps gqsdsimhps mnqssiaqdr gymqrnpqmp qysspqpgsa lsprqpsggq 781 ihtgmgsyqq nsmgsygpqg gqygpqggyp rqpnynalpn anypsagmag ginpmgaggq 841 mhgqpgippy gtlppgrmsh asmgnrpygp nmanmppqvg sgmcpppggm nrktqetava 901 mhvaansiqn rppgypnmnq ggmmgtgppy gqginsmagm inpqgppysm ggtmannsag 961 maaspemmgl gdvkltpatk mnnkadgtpk teskskksss stttnekitk lyelggeper 1021 kmwvdrylaf teekamgmtn lpavgrkpld lyrlyvsvke iggltqvnkn kkwrelatnl 1081 nvgtsssaas slkkqyiqcl yafeckierg edpppdifaa adskksqpki qppspagsgs 1141 mqgpqtpqst sssmaeggdl kpptpastph sqipplpgms rsnsvgiqda fndgsdstfq 1201 krnsmtpnpg yqpsmntsdm mgrmsyepnk dpygsmrkap gsdpfmssgq gpnggmgdpy 1261 sraagpglgn vamgprqhyp yggpydrvrt epgigpegnm stgapqpnlm psnpdsgmys 1321 psryppqqqq qqqqrhdsyg nqfstqgtps gspfpsqqtt myqqqqqvss paplprpmen 1381 rtspskspfl hsgmkmqkag ppvpashiap apvqppmirr ditfppgsve atqpvlkqrr 1441 rltmkdigtp eawrvmmslk sgllaestwa ldtinillyd dnsimtfnls qlpgllellv 1501 eyfrrcliei fgilkeyevg dpgqrtlldp grfskvsspa pmeggeeeee llgpkleeee 1561 eeevvendee iafsgkdkpa senseeklis kfdklpvkiv qkndpfvvdc sdklgrvqef 1621 dsgllhwrig ggdttehiqt hfesktellp srphapcppa prkhvttaeg tpgttdqegp 1681 ppdgppekri tatmddmlst rsstltedga ksseaikess kfpfgispaq shrnikiled 1741 ephskdetpl ctlldwqdsl akrcvcvsnt irslsfvpgn dfemskhpgl llilgklill 1801 hhkhperkqa pltyekeeeq dqgvscnkve wwwdclemlr entlvtlani sgqldlspyp |
| 1861 esiclpvldg llhwavcpsa eaqdpfstlg pnavlspqrl vletlsklsi qdnnvdlila 1921 tppfsrlekl ystmvrflsd rknpvcrema vvllanlaqg dslaaraiav qkgsignllg 1981 fledslaatq fqqsqasllh mqnppfepts vdmmrraara llalakvden hseftlyesr 2041 lldisvsplm nslvsqvicd vlfligqs |

| |
|---|
| Homo sapiens AT rich interactive domain 1A (SWI-like) (ARID1A), transcript variant 2, mRNA (SEQ ID NO: 12) |
| 1 cagaaagcgg agagtcacag cggggccagg ccctggggag cggagcctcc accgcccccc 61 tcattcccag gcaagggctt ggggggaatg agccgggaga gccgggtccc gagcctacag 121 agccgggagc agctgagccg ccggcgcctc ggccgccgcc gccgcctcct cctcctccgc 181 cgccgccagc ccggagcctg agccggcggg gcggggggga gaggagcgag cgcagcgcag 241 cagcggagcc ccgcgaggcc cgcccgggcg ggtggggagg gcagcccggg ggactgggcc 301 ccgggcgggg gtgggagggg gggagaagac gaagacaggg ccgggtctct ccgcggacga 361 gacagcgggg atcatggccg cgcaggtcgc ccccgccgcc gccagcagcc tgggcaaccc 421 gccgccgccg ccgccctcgg agctgaagaa agccgagcag cagcagcggg aggaggcggg 481 gggcgaggcg gcggcggcgg cagcggccga gcgcggggaa atgaaggcag ccgccgggca 541 ggaaagcgag ggccccgccg tggggccgcc gcagccgctg ggaaaggagc tgcaggacgg 601 ggccgagagc aatgggggtg gcggcggcgg cggagccggc agcggcggcg ggcccggcgc 661 ggagccggac ctgaagaact cgaacgggaa cgcgggccct aggcccgccc tgaacaataa 721 cctcacggag ccgcccggcg gcggcggtgg cggcagcagc gatggggtgg gggcgcctcc 781 tcactcagcc gcggccgcct tgccgccccc agcctacggc ttcgggcaac cctacggccg 841 gagcccgtct gccgtcgccg ccgccgcggc cgccgtcttc caccaacaac atggcggaca 901 acaaagccct ggcctggcag cgctgcagag cggcggcggc gggggcctgg agccctacgc 961 ggggccccag cagaactctc acgaccacgg cttccccaac caccagtaca actcctacta 1021 ccccaaccgc agcgcctacc ccccgcccgc cccggcctac gcgctgagct ccccgagagg 1081 tggcactccg ggctccggcg cggcggcggc tgccggctcc aagccgcctc cctcctccag 1141 cgcctccgcc tcctcgtcgt cttcgtcctt cgctcagcag cgcttcgggg ccatgggggg 1201 aggcggcccc tccgcggccg gcgggggaac tccccagccc accgccaccc ccaccctcaa 1261 ccaactgctc acgtcgccca gctcggcccg gggctaccag ggctaccccg gggcgacta 1321 cagtggcggg ccccaggacg ggggcgccgg caagggcccg gcggacatgg cctcgcagtg 1381 ttggggggct gcggcggcgg cagctgcggc ggcggccgcc tcgggagggg cccaacaaag 1441 gagccaccac gcgcccatga gccccgggag cagcggcggc ggggggcagc cgctcgcccg 1501 gacccctcag ccatccagtc caatggatca gatgggcaag atgagacctc agccatatgg 1561 cgggactaac ccatactcgc agcaacaggg acctccgtca ggaccgcagc aaggacatgg 1621 gtacccaggg cagccatacg ggtcccagac cccgcagcgg tacccgatga ccatgcaggg 1681 ccgggcgcag agtgccatgg gcggcctctc ttatacacag cagattcctc cttatggaca 1741 acaaggcccc agcgggtatg gtcaacaggg ccagactcca tattacaacc agcaaagtcc 1801 tcaccctcag cagcagcagc caccctactc ccagcaacca ccgtcccaga cccctcatgc 1861 ccaaccttcg tatcagcagc agccacagtc tcaaccacca cagctccagt cctctcagcc 1921 tccatactcc cagcagccat cccagcctcc acatcagcag tccccggctc catacccctc 1981 ccagcagtcg acgacacagc agcaccccca gagccagccc ccctactcac agccacaggc 2041 tcagtctcct taccagcagc agcaacctca gcagccagca ccctcgacgc tctcccagca 2101 ggctgcgtat cctcagcccc agtctcagca gtcccagcaa actgcctatt cccagcagcg 2161 cttccctcca ccgcaggagc tatctcaaga ttcatttggg tctcaggcat cctcagcccc 2221 ctcaatgacc tccagtaagg gagggcaaga agatatgaac ctgagccttc agtcaagacc 2281 ctccagcttg cctgatctat ctggttcaat agatgacctc cccatgggga cagaaggagc 2341 tctgagtcct ggagtgagca catcagggat ttccagcagc caaggagagc agagtaatcc 2401 agctcagtct cctttctctc ctcatacctc ccctcacctg cctggcatcc gaggcccttc 2461 cccgtcccct gttggctctc ccgccagtgt tgctcagtct cgctcaggac cactctcgcc 2521 tgctgcagtg ccaggcaacc agatgccacc tcggccaccc agtggccagt cggacagcat 2581 catgcatcct tccatgaacc aatcaagcat tgcccaagat cgaggttata tgcagaggaa 2641 cccccagatg ccccagtaca gttcccccca gcccggctca gccttatctc cgcgtcagcc 2701 ttccggagga cagatacaca caggcatggg ctcctaccag cagaactcca tggggagcta 2761 tggtccccag gggggtcagt atggcccaca aggtggctac cccaggcagc caaactataa 2821 tgccttgccc aatgccaact accccagtgc aggcatggct ggaggcataa accccatggg 2881 tgccggaggt caaatgcatg gacagcctgg catcccacct tatggcacac tccctccagg 2941 gaggatgagt cacgcctcca tgggcaaccg gccttatggc cctaacatgg ccaatatgcc 3001 acctcaggtt gggtcaggga tgtgtccccc accagggggc atgaaccgga aaacccaaga |
| 3061 aactgctgtc gccatgcatg ttgctgccaa ctctatccaa aacaggccgc caggctaccc 3121 caatatgaat caagggggca tgatgggaac tggacctcct tatggacaag ggattaatag 3181 tatggctggc atgatcaacc ctcagggacc cccatattcc atgggtggaa ccatggccaa 3241 caattctgca gggatggcag ccagcccaga gatgatgggc cttggggatg taaagttaac 3301 tccagccacc aaaatgaaca acaaggcaga tgggacaccc aagacagaat ccaaatccaa 3361 gaaatccagt tcttctacta caaccaatga gaagatcacc aagttgtatg agctgggtgg 3421 tgagcctgag aggaagatgt gggtggaccg ttatctggcc ttcactgagg agaaggccat 3481 gggcatgaca aatctgcctg ctgtgggtag gaaacctctg gacctctatc gcctctatgt 3541 gtctgtgaag gagattggtg gattgactca ggtcaacaag aacaaaaaat ggcgggaact 3601 tgcaaccaac ctcaatgtgg gcacatcaag cagtgctgcc agctccttga aaaagcagta 3661 tatccagtgt ctctatgcct ttgaatgcaa gattgaacgg ggagaagacc ctcccccaga 3721 catctttgca gctgctgatt ccaagaagtc ccagcccaag atccagcctc cctctcctgc 3781 gggatcagga tctatgcagg ggccccagac tccccagtca accagcagtt ccatggcaga 3841 aggaggagac ttaaagccac caactccagc atccacacca cacagtcaga tccccccatt 3901 gccaggcatg agcaggagca attcagttgg gatccaggat gcctttaatg atggaagtga 3961 ctccacattc cagaagcgga attccatgac tccaaaccct gggtatcagc ccagtatgaa 4021 tacctctgac atgatggggc gcatgtccta tgagccaaat aaggatcctt atggcagcat 4081 gaggaaagct ccagggagtg atcccttcat gtcctcaggg cagggcccca acggcgggat 4141 gggtgacccc tacagtcgtg ctgccggccc tgggctagga aatgtggcga tgggaccacg 4201 acagcactat ccctatggag gtccttatga cagagtgagg acggagcctg gaatagggcc 4261 tgagggaaac atgagcactg gggccccaca gccgaatctc atgccttcca acccagactc 4321 ggggatgtat tctcctagcc gctacccccc gcagcagcag cagcagcagc agcaacgaca 4381 tgattcctat ggcaatcagt tctccaccca aggcacccct tctggcagcc ccttccccag 4441 ccagcagact acaatgtatc aacagcaaca gcaggtatcc agccctgctc ccctgccccg 4501 gccaatggag aaccgcacct ctcctagcaa gtctccattc ctgcactctg ggatgaaaat 4561 gcagaaggca ggtcccccag tacctgcctc gcacatagca cctgcccctg tgcagccccc 4621 catgattcgg cgggatatca ccttcccacc tggctctgtt gaagccacac agcctgtgtt 4681 gaagcagagg aggcggctca caatgaaaga cattggaacc ccggaggcat ggcgggtaat 4741 gatgtccctc aagtctggtc tcctggcaga gagcacatgg gcattagata ccatcaacat 4801 cctgctgtat gatgacaaca gcatcatgac cttcaacctc agtcagctcc cagggttgct 4861 agagctcctt gtagaatatt tccgacgatg cctgattgag atctttggca ttttaaagga 4921 gtatgaggtg ggtgacccag gacagagaac gctactggat cctgggaggt tcagcaaggt 4981 gtctagtcca gctcccatgg agggtgggga agaagaagaa gaacttctag gtcctaaact 5041 agaagaggaa gaagaagagg aagtagttga aaatgatgag gagatagcct tttcaggcaa 5101 ggacaagcca gcttcagaga atagtgagga gaagctgatc agtaagtttg acaagcttcc 5161 agtaaagatc gtacagaaga atgatccatt tgtggtggac tgctcagata agcttgggcg 5221 tgtgcaggag tttgacagtg gcctgctgca ctggcggatt ggtggggggg acaccactga 5281 gcatatccag acccacttcg agagcaagac agagctgctg ccttcccggc ctcacgcacc 5341 ctgcccacca gcccctcgga agcatgtgac aacagcagag ggtacaccag ggacaacaga 5401 ccaggagggg cccccacctg atggacctcc agaaaaacgg atcacagcca ctatggatga 5461 catgttgtct actcggtcta gcaccttgac cgaggatgga gctaagagtt cagaggccat 5521 caaggagagc agcaagtttc catttggcat tagcccagca cagagccacc ggaacatcaa 5581 gatcctagag gacgaacccc acagtaagga tgagacccca ctgtgtaccc ttctggactg 5641 gcaggattct cttgccaagc gctgcgtctg tgtgtccaat accattcgaa gcctgtcatt 5701 tgtgccaggc aatgactttg agatgtccaa acacccaggg ctgctgctca tcctgggcaa 5761 gctgatcctg ctgcaccaca agcacccaga acggaagcag gcaccactaa cttatgaaaa 5821 ggaggaggaa caggaccaag gggtgagctg caacaaagtg gagtggtggt gggactgctt 5881 ggagatgctc cgggaaaaca ccttggttac actcgccaac atctcggggc agttggacct 5941 atctccatac cccgagagca tttgcctgcc tgtcctggac ggactcctac actgggcagt 6001 ttgcccttca gctgaagccc aggacccctt ttccaccctg ggccccaatg ccgtcctttc 6061 cccgcagaga ctggtcttgg aaaccctcag caaactcagc atccaggaca acaatgtgga 6121 cctgattctg gccacacccc ccttcagccg cctggagaag ttgtatagca ctatggtgcg 6181 cttcctcagt gaccgaaaga acccggtgtg ccgggagatg gctgtggtac tgctggccaa 6241 cctggctcag ggggacagcc tggcagctcg tgccattgca gtgcagaagg gcagtatcgg 6301 caacctcctg ggcttcctag aggacagcct tgccgccaca cagttccagc agagccaggc 6361 cagcctcctc cacatgcaga acccaccctt tgagccaact agtgtggaca tgatgcggcg 6421 ggctgcccgc gcgctgcttg ccttggccaa ggtggacgag aaccactcag agtttactct 6481 gtacgaatca cggctgttgg acatctcggt atcaccgttg atgaactcat tggtttcaca 6541 agtcatttgt gatgtactgt ttttgattgg ccagtcatga cagccgtggg acacctcccc 6601 cccccgtgtg tgtgtgcgtg tgtggagaac ttagaaactg actgttgccc tttatttatg |
| 6661 caaaaccacc tcagaatcca gtttaccctg tgctgtccag cttctccctt gggaaaaagt 6721 ctctcctgtt tctctctcct ccttccacct cccctccctc catcacctca cgcctttctg 6781 ttccttgtcc tcaccttact cccctcagga ccctacccca ccctctttga aaagacaaag 6841 ctctgcctac atagaagact ttttttattt taaccaaagt tactgttgtt tacagtgagt 6901 ttggggaaaa aaaataaaat aaaaatggct ttcccagtcc ttgcatcaac gggatgccac 6961 atttcataac tgtttttaat ggtaaaaaaa aaaaaaaaaa atacaaaaaa aaattctgaa 7021 ggacaaaaaa ggtgactgct gaactgtgtg tggtttattg ttgtacattc acaatcttgc 7081 aggagccaag aagttcgcag ttgtgaacag accctgttca ctggagaggc ctgtgcagta 7141 gagtgtagac cctttcatgt actgtactgt acacctgata ctgtaaacat actgtaataa 7201 taatgtctca catggaaaca gaaaacgctg ggtcagcagc aagctgtagt ttttaaaaat 7261 gtttttagtt aaacgttgag gagaaaaaaa aaaaaggctt ttcccccaaa gtatcatgtg 7321 tgaacctaca acaccctgac ctctttctct cctccttgat tgtatgaata accctgagat 7381 cacctcttag aactggtttt aacctttagc tgcagcggct acgctgccac gtgtgtatat 7441 atatgacgtt gtacattgca catacccttg gatccccaca gtttggtcct cctcccagct 7501 acccctttat agtatgacga gttaacaagt tggtgacctg cacaaagcga gacacagcta 7561 tttaatctct tgccagatat cgcccctctt ggtgcgatgc tgtacaggtc tctgtaaaaa 7621 gtccttgctg tctcagcagc caatcaactt atagtttatt tttttctggg tttttgtttt 7681 gttttgtttt ctttctaatc gaggtgtgaa aaagttctag gttcagttga agttctgatg 7741 aagaaacaca attgagattt tttcagtgat aaaatctgca tatttgtatt tcaacaatgt 7801 agctaaaact tgatgtaaat tcctcctttt tttccttttt tggcttaatg aatatcattt 7861 attcagtatg aaatctttat actatatgtt ccacgtgtta agaataaatg tacattaaat 7921 cttggtaaga cttt |

The term "inducing neuronal differentiation" used herein refers to causing a cell to develop into a cell

According to the methods of the disclosure, a "normal" cell may be used as a basis of comparison for one or more characteristics of a cancer cell, including expression and/or function of SNF5, ATRX, and/or ARID1A. As used herein, a "normal cell" is a cell that cannot be classified as part of a "cell proliferative disorder". A normal cell lacks unregulated or abnormal growth, or both, that can lead to the development of an unwanted condition or disease. Preferably, a normal cell expresses a comparable amount of EZH2 as a cancer cell. Preferably a normal cell contains a wild type sequence for a SNF5, ATRX, and/or ARID1A gene, expresses a SNF5, ATRX, and/or ARID1A transcript without mutations, and expresses a SNF5, ATRX, and/or ARID1A protein without mutations that retains all functions a normal activity levels.

As used herein, "contacting a cell" refers to a condition in which a compound or other composition of matter is in direct contact with a cell, or is close enough to induce a desired biological effect in a cell.

As used herein, "treating" or "treat" describes the management and care of a subject for the purpose of combating a disease, condition, or disorder and includes the administration of an EZH2 inhibitor of the disclosure, or a pharmaceutically acceptable salt, prodrug, metabolite, polymorph or solvate thereof, to alleviate the symptoms or complications of cancer or to eliminate the cancer.

As used herein, the term "alleviate" is meant to describe a process by which the severity of a sign or symptom of cancer is decreased. Importantly, a sign or symptom can be alleviated without being eliminated. In a preferred embodiment, the administration of pharmaceutical compositions of the disclosure leads to the elimination of a sign or symptom, however, elimination is not required. Effective dosages are expected to decrease the severity of a sign or symptom. For instance, a sign or symptom of a disorder such as cancer, which can occur in multiple locations, is alleviated if the severity of the cancer is decreased within at least one of multiple locations.

As used herein, the term "severity" is meant to describe the potential of cancer to transform from a precancerous, or benign, state into a malignant state. Alternatively, or in addition, severity is meant to describe a cancer stage, for example, according to the TNM system (accepted by the International Union Against Cancer (UICC) and the American Joint Committee on Cancer (AJCC)) or by other art-recognized methods. Cancer stage refers to the extent or severity of the cancer, based on factors such as the location of the primary tumor, tumor size, number of tumors, and lymph node involvement (spread of cancer into lymph nodes). Alternatively, or in addition, severity is meant to describe the tumor grade by art-recognized methods (see, National Cancer Institute, www.cancer.gov). Tumor grade is a system used to classify cancer cells in terms of how abnormal they look under a microscope and how quickly the tumor is likely to grow and spread. Many factors are considered when determining tumor grade, including the structure and growth pattern of the cells. The specific factors used to determine tumor grade vary with each type of cancer. Severity also describes a histologic grade, also called differentiation, which refers to how much the tumor cells resemble normal cells of the same tissue type (see, National Cancer Institute, www.cancer.gov). Furthermore, severity describes a nuclear grade, which refers to the size and shape of the nucleus in tumor cells and the percentage of tumor cells that are dividing (see, National Cancer Institute, www.cancer.gov).

In another aspect of the disclosure, severity describes the degree to which a tumor has secreted growth factors, degraded the extracellular matrix, become vascularized, lost adhesion to juxtaposed tissues, or metastasized. Moreover, severity describes the number of locations to which a primary tumor has metastasized. Finally, severity includes the difficulty of treating tumors of varying types and locations. For example, inoperable tumors, those cancers which have greater access to multiple body systems (hematological and immunological tumors), and those which are the most resistant to traditional treatments are considered most severe. In these situations, prolonging the life expectancy of the subject and/or reducing pain, decreasing the proportion of cancerous cells or restricting cells to one system, and improving cancer stage/tumor grade; histological grade/nuclear grade are considered alleviating a sign or symptom of the cancer.

As used herein the term "symptom" is defined as an indication of disease, illness, injury, or that something is not right in the body. Symptoms are felt or noticed by the individual experiencing the symptom, but may not easily be noticed by others. Others are defined as non-health-care professionals.

As used herein the term "sign" is also defined as an indication that something is not right in the body. But signs are defined as things that can be seen by a doctor, nurse, or other health care professional.

Cancer is a group of diseases that may cause almost any sign or symptom. The signs and symptoms will depend on where the cancer is, the size of the cancer, and how much it affects the nearby organs or structures. If a cancer spreads (metastasizes), then symptoms may appear in different parts of the body.

As a cancer grows, it begins to push on nearby organs, blood vessels, and nerves. This pressure creates some of the signs and symptoms of cancer. Cancers may form in places where it does not cause any symptoms until the cancer has grown quite large.

Cancer may also cause symptoms such as fever, fatigue, or weight loss. This may be because cancer cells use up much of the body's energy supply or release substances that change the body's metabolism. Or the cancer may cause the immune system to react in ways that produce these symptoms. While the signs and symptoms listed above are the more common ones seen with cancer, there are many others that are less common and are not listed here. However, all art-recognized signs and symptoms of cancer are contemplated and encompassed by the disclosure.

Treating cancer may result in a reduction in size of a tumor. A reduction in size of a tumor may also be referred to as "tumor regression". Preferably, after treatment according to the methods of the disclosure, tumor size is reduced by 5% or greater relative to its size prior to treatment; more preferably, tumor size is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Size of a tumor may be measured by any reproducible means of measurement. The size of a tumor may be measured as a diameter of the tumor.

Treating cancer may result in a reduction in tumor volume. Preferably, after treatment according to the methods of the disclosure, tumor volume is reduced by 5% or greater relative to its size prior to treatment; more preferably, tumor volume is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Tumor volume may be measured by any reproducible means of measurement.

Treating cancer may result in a decrease in number of tumors. Preferably, after treatment, tumor number is reduced by 5% or greater relative to number prior to treatment; more preferably, tumor number is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. Number of tumors may be measured by any reproducible means of measurement. The number of tumors may be measured by counting tumors visible to the naked eye or at a specified magnification. Preferably, the specified magnification is 2x, 3x, 4x, 5x, 10x, or 50x.

Treating cancer may result in a decrease in number of metastatic lesions in other tissues or organs distant from the primary tumor site. Preferably, after treatment according to the methods of the disclosure, the number of metastatic lesions is reduced by 5% or greater relative to number prior to treatment; more preferably, the number of metastatic lesions is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. The number of metastatic lesions may be measured by any reproducible means of measurement. The number of metastatic lesions may be measured by counting metastatic lesions visible to the naked eye or at a specified magnification. Preferably, the specified magnification is 2x, 3x, 4x, 5x, 10x, or 50x.

An effective amount of an EZH2 inhibitor of the disclosure, or a pharmaceutically acceptable salt, prodrug, metabolite, polymorph or solvate thereof, is not significantly cytotoxic to normal cells. For example, a therapeutically effective amount of an EZH2 inhibitor of the disclosure is not significantly cytotoxic to normal cells if administration of the EZH2 inhibitor of the disclosure in a therapeutically effective amount does not induce cell death in greater than 10% of normal cells. A therapeutically effective amount of an EZH2 inhibitor of the disclosure does not significantly affect the viability of normal cells if administration of the compound in a therapeutically effective amount does not induce cell death in greater than 10% of normal cells.

Contacting a cell with an EZH2 inhibitor of the disclosure, or a pharmaceutically acceptable salt, prodrug, metabolite, polymorph or solvate thereof, can inhibit EZH2 activity selectively in cancer cells. Administering to a subject in need thereof an EZH2 inhibitor of the disclosure, or a pharmaceutically acceptable salt, prodrug, metabolite, polymorph or solvate thereof, can inhibit EZH2 activity selectively in cancer cells.

### Medulloblastoma

Medulloblastoma is a fast-growing, aggressive, high-grade brain tumor. Regardless of the subtype, medulloblastoma always occurs in the cerebellum of the brain, and more specifically, within the posterior fossa of the cerebellum. The cerebellum controls balance and other complex motor functions.

Medulloblastoma rarely spreads beyond the central nervous system (CNS) (i.e., the brain and spinal cord); however, metastatic medulloblastoma may spread to the bones and bone marrow. Medulloblastoma cells arise from immature cells in the cerebellum that frequently divide under normal conditions to produce and replace cells of the cerebellum.

Medulloblastoma is relatively rare, accounting for less than 2% of all primary brain tumors and 18% of all pediatric brain tumors. More than 70% of all pediatric medulloblastomas are diagnosed in children under age 10. Medulloblastoma can occur in adults, and when found, occur most often in adults aged 20-44. Medulloblastoma occurs more frequently in males than females.

Subtypes of medulloblastoma include, but are not limited to, classic medulloblastoma, desmoplastic nodular medulloblastoma, large-cell or anaplastic medulloblastoma, medulloblastoma with neuroblastic or neuronal differentiation, medulloblastoma with glial differentiation, medullomyoblastoma and melanotic medulloblastoma. As used in this disclosure, the term "medulloblastoma" may include all subtypes of this cancer. Medulloblastoma may also be referred to as cerebellar primitive neuroectodermal tumor (PNET).

Symptoms of medulloblastoma include, but are not limited to, behavioral changes, changes in appetite, and symptoms of increased pressure on the brain (e.g., headache, nausea, vomiting, and drowsiness, as well as problems with coordination (e.g. clumsiness, problems with handwriting, and visual problems)). Unusual eye movements may also occur. If the cancer has spread to the spinal cord, symptoms may include back pain, trouble walking, and/or problems controlling bladder and bowel functions.

Medulloblastoma is often treated with surgery as a first line therapy in combination with or followed by radiation therapy and/or chemotherapy. Subjects of the disclosure in need of treatment with an EZH2 inhibitor, and, preferably, treatment with Tazemetostat, may be treated with an EZH2 inhibitor in combination with surgery, radiation, and/or chemotherapy. Subjects of the disclosure in need of treatment with an EZH2 inhibitor, and, preferably, treatment with Tazemetostat, may have undergone surgery, radiation, or a course of chemotherapy prior to treatment with an EZH2 inhibitor of the disclosure. Subjects of the disclosure in need of treatment with an EZH2 inhibitor, and, preferably, treatment with Tazemetostat, may have undergone surgery, radiation, or a course of chemotherapy prior to treatment with an EZH2 inhibitor of the disclosure and may have experienced no benefit from the surgery, radiation, and/or chemotherapy. EZH2 inhibitors of the disclosure, including, but not limited to, tazemetostat, may be used as a first line therapy prior to recommending or performing surgery, radiation, and/or chemotherapy to the subject.

### EZH2 Inhibitors

EZH2 inhibitors of the disclosure comprise tazemetostat (EPZ-6438 or Compound A): 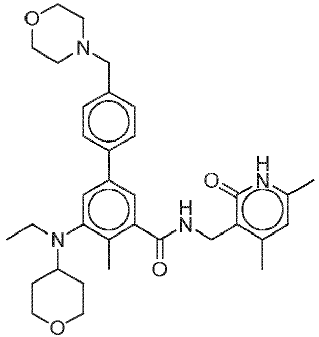 or a pharmaceutically acceptable salt thereof.

Tazemetostat is also described in US Patent Nos. 8,410,088, 8,765,732, and 9,090,562 (the contents of which are each incorporated herein in their entireties).

Tazemetostat or a pharmaceutically acceptable salt thereof, as described herein, is potent in targeting both WT and mutant EZH2. Tazemetostat is orally bioavailable and has high selectivity to EZH2 compared with other histone methyltransferases (i.e. >20,000 fold selectivity by Ki). Importantly, tazemetostat has targeted methyl mark inhibition that results in the killing of genetically defined cancer cells *in vitro.* Animal models have also shown sustained *in vivo* efficacy following inhibition of the target methyl mark. Clinical trial results described herein also demonstrate the safety and efficacy of tazemetostat.

In one embodiment, tazemetostat or a pharmaceutically acceptable salt thereof is administered to the subject at a dose of approximately 100 mg to approximately 3200 mg daily, such as about 100 mg BID to about 1600 mg BID (e.g., 100 mg BID, 200 mg BID, 400 mg BID, 800 mg BID, or 1600 mg BID), for treating a NHL. On one embodiment the dose is 800 mg BID.

EZH2 inhibitors of the disclosure may comprise, consist essentially of or consist of: 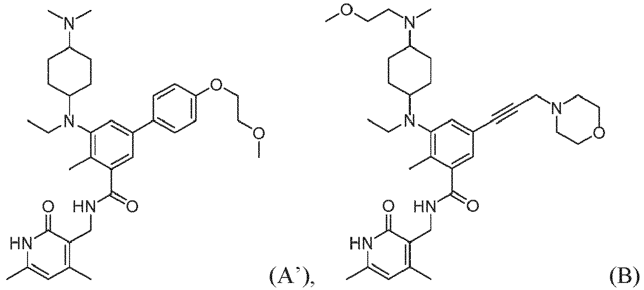 or 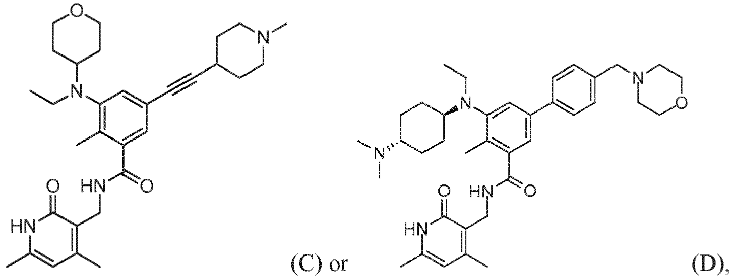 or stereoisomers thereof or pharmaceutically acceptable salts and solvates thereof.

EZH2 inhibitors of the disclosure may comprise, consist essentially of or consist of Compound E: 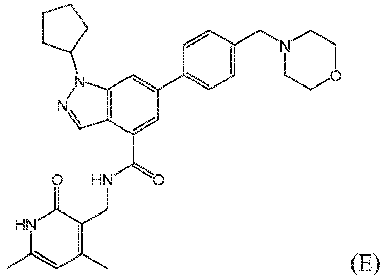 or pharmaceutically acceptable salts thereof.

EZH2 inhibitors of the disclosure may comprise, consist essentially of or consist of GSK-126, having the following formula: 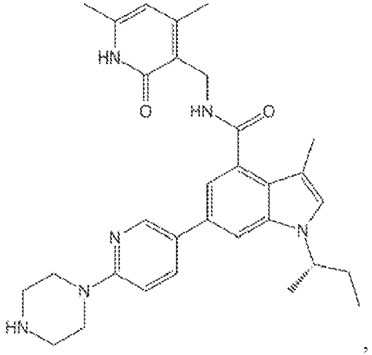 stereoisomers thereof, or pharmaceutically acceptable salts or solvates thereof.

EZH2 inhibitors of the disclosure may comprise, consist essentially of or consist of Compound F: 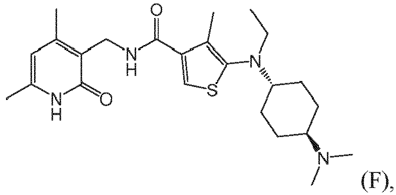 or stereoisomers thereof or pharmaceutically acceptable salts and solvates thereof.

EZH2 inhibitors of the disclosure may comprise, consist essentially of or consist of any one of Compounds Ga-Gc: 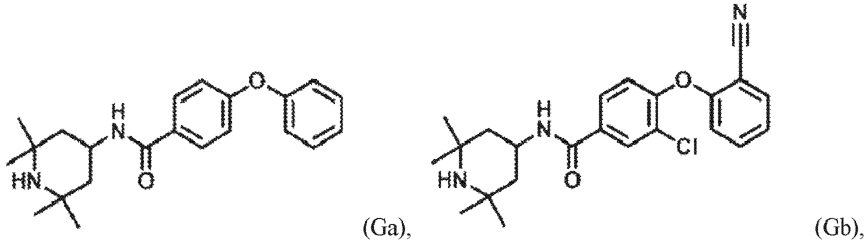 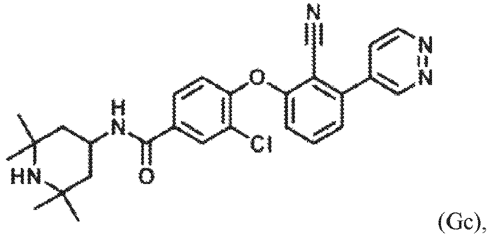 or a stereoisomer, pharmaceutically acceptable salt or solvate thereof.

EZH2 inhibitors of the disclosure may comprise, consist essentially of or consist of CPI-1205 or GSK343.

Additional suitable EZH2 inhibitors will be apparent to those skilled in the art. In some embodiments of the strategies, treatment modalities, methods, combinations, and compositions provided herein, the EZH2 inhibitor is an EZH2 inhibitor described in US 8,536,179 (describing GSK-126 among other compounds and corresponding to WO 2011/140324), the entire contents of each of which are incorporated herein by reference.

In some embodiments of the strategies, treatment modalities, methods, combinations, and compositions provided herein, the EZH2 inhibitor is an EZH2 inhibitor described in PCT/US2014/015706, published as WO 2014/124418, in PCT/US2013/025639, published as WO 2013/120104, and in US 14/839,273, published as US 2015/0368229, the entire contents of each of which are incorporated herein by reference.

In one embodiment, the compound disclosed herein is the compound itself, i.e., the free base or "naked" molecule. In another embodiment, the compound is a salt thereof, e.g., a mono-HCl or tri-HCl salt, mono-HBr or tri-HBr salt of the naked molecule.

Compounds disclosed herein that contain nitrogens can be converted to N-oxides by treatment with an oxidizing agent (e.g., 3-chloroperoxybenzoic acid (mCPBA) and/or hydrogen peroxides) to afford other compounds suitable for any methods disclosed herein. Thus, all shown and claimed nitrogen-containing compounds are considered, when allowed by valency and structure, to include both the compound as shown and its N-oxide derivative (which can be designated as N→O or N⁺-O⁻). Furthermore, in other instances, the nitrogens in the compounds disclosed herein can be converted to N-hydroxy or N-alkoxy compounds. For example, N-hydroxy compounds can be prepared by oxidation of the parent amine by an oxidizing agent such as m-CPBA. All shown and claimed nitrogen-containing compounds are also considered, when allowed by valency and structure, to cover both the compound as shown and its N-hydroxy (*i.e.,* N-OH) and N-alkoxy (*i.e.,* N-OR, wherein R is substituted or unsubstituted C₁-C ₆ alkyl, C₁-C₆ alkenyl, C₁-C₆ alkynyl, 3-14-membered carbocycle or 3-14-membered heterocycle) derivatives.

"Isomerism" means compounds that have identical molecular formulae but differ in the sequence of bonding of their atoms or in the arrangement of their atoms in space. Isomers that differ in the arrangement of their atoms in space are termed "stereoisomers." Stereoisomers that are not mirror images of one another are termed "diastereoisomers," and stereoisomers that are non-superimposable mirror images of each other are termed "enantiomers" or sometimes optical isomers. A mixture containing equal amounts of individual enantiomeric forms of opposite chirality is termed a "racemic mixture."

A carbon atom bonded to four nonidentical substituents is termed a "chiral center."

"Chiral isomer" means a compound with at least one chiral center. Compounds with more than one chiral center may exist either as an individual diastereomer or as a mixture of diastereomers, termed "diastereomeric mixture." When one chiral center is present, a stereoisomer may be characterized by the absolute configuration (R or S) of that chiral center. Absolute configuration refers to the arrangement in space of the substituents attached to the chiral center. The substituents attached to the chiral center under consideration are ranked in accordance with the *Sequence Rule* of Cahn, Ingold and Prelog. (Cahn et al., Angew. Chem. Inter. Edit. 1966, 5, 385; errata 511; Cahn et al., Angew. Chem. 1966, 78, 413; Cahn and Ingold, J. Chem. Soc. 1951 (London), 612; Cahn et al., Experientia 1956, 12, 81; Cahn, J. Chem. Educ. 1964, 41, 116).

"Geometric isomer" means the diastereomers that owe their existence to hindered rotation about double bonds or a cycloalkyl linker (e.g., 1,3-cyclobutyl). These configurations are differentiated in their names by the prefixes cis and trans, or Z and E, which indicate that the groups are on the same or opposite side of the double bond in the molecule according to the Cahn-Ingold-Prelog rules.

It is to be understood that the compounds disclosed herein may be depicted as different chiral isomers or geometric isomers. It should also be understood that when compounds have chiral isomeric or geometric isomeric forms, all isomeric forms are intended to be included in the scope of the disclosure, and the naming of the compounds does not exclude any isomeric forms.

Furthermore, the structures and other compounds discussed in this disclosure include all atropic isomers thereof. "Atropic isomers" are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

"Tautomer" is one of two or more structural isomers that exist in equilibrium and is readily converted from one isomeric form to another. This conversion results in the formal migration of a hydrogen atom accompanied by a switch of adjacent conjugated double bonds. Tautomers exist as a mixture of a tautomeric set in solution. In solutions where tautomerization is possible, a chemical equilibrium of the tautomers will be reached. The exact ratio of the tautomers depends on several factors, including temperature, solvent and pH. The concept of tautomers that are interconvertible by tautomerizations is called tautomerism.

Of the various types of tautomerism that are possible, two are commonly observed. In keto-enol tautomerism a simultaneous shift of electrons and a hydrogen atom occurs. Ring-chain tautomerism arises as a result of the aldehyde group (-CHO) in a sugar chain molecule reacting with one of the hydroxy groups (-OH) in the same molecule to give it a cyclic (ring-shaped) form as exhibited by glucose.

Common tautomeric pairs are: ketone-enol, amide-nitrile, lactam-lactim, amide-imidic acid tautomerism in heterocyclic rings (*e.g*., in nucleobases such as guanine, thymine and cytosine), imine-enamine and enamine-enamine. An example of keto-enol equilibria is between pyridin-2(1H)-ones and the corresponding pyridin-2-ols, as shown below.

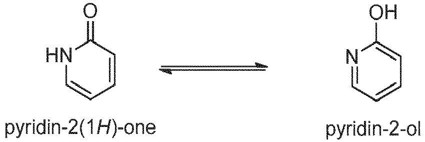

It is to be understood that the compounds disclosed herein may be depicted as different tautomers. It should also be understood that when compounds have tautomeric forms, all tautomeric forms are intended to be included in the scope of the disclosure, and the naming of the compounds does not exclude any tautomer form.

The compounds disclosed herein include the compounds themselves, as well as their salts and their solvates, if applicable. A salt, for example, can be formed between an anion and a positively charged group (e.g., amino) on an aryl- or heteroaryl-substituted benzene compound. Suitable anions include chloride, bromide, iodide, sulfate, bisulfate, sulfamate, nitrate, phosphate, citrate, methanesulfonate, trifluoroacetate, glutamate, glucuronate, glutarate, malate, maleate, succinate, fumarate, tartrate, tosylate, salicylate, lactate, naphthalenesulfonate, and acetate (e.g., trifluoroacetate). The term "pharmaceutically acceptable anion" refers to an anion suitable for forming a pharmaceutically acceptable salt. Likewise, a salt can also be formed between a cation and a negatively charged group (e.g., carboxylate) on an aryl- or heteroaryl-substituted benzene compound. Suitable cations include sodium ion, potassium ion, magnesium ion, calcium ion, and an ammonium cation such as tetramethylammonium ion. The aryl- or heteroaryl-substituted benzene compounds also include those salts containing quaternary nitrogen atoms. In the salt form, it is understood that the ratio of the compound to the cation or anion of the salt can be 1: 1, or any ration other than 1:1, e.g., 3:1, 2:1, 1:2, or 1:3.

Additionally, the compounds disclosed herein, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvate" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate; and if the solvent is alcohol, the solvate formed is an alcoholate. Hydrates are formed by the combination of one or more molecules of water with one molecule of the substance in which the water retains its molecular state as H₂O.

As used herein, the term "analog" refers to a chemical compound that is structurally similar to another but differs slightly in composition (as in the replacement of one atom by an atom of a different element or in the presence of a particular functional group, or the replacement of one functional group by another functional group). Thus, an analog is a compound that is similar or comparable in function and appearance, but not in structure or origin to the reference compound.

As defined herein, the term "derivative" refers to compounds that have a common core structure, and are substituted with various groups as described herein. For example, all of the compounds represented by Formula (I) are aryl- or heteroaryl-substituted benzene compounds, and have Formula (I) as a common core.

The term "bioisostere" refers to a compound resulting from the exchange of an atom or of a group of atoms with another, broadly similar, atom or group of atoms. The objective of a bioisosteric replacement is to create a new compound with similar biological properties to the parent compound. The bioisosteric replacement may be physicochemically or topologically based. Examples of carboxylic acid bioisosteres include, but are not limited to, acyl sulfonimides, tetrazoles, sulfonates and phosphonates. See, *e.g*., Patani and LaVoie, Chem. Rev. 96, 3147-3176, 1996.

The present disclosure is intended to include all isotopes of atoms occurring in the present compounds. Isotopes include those atoms having the same atomic number but different mass numbers. By way of general example and without limitation, isotopes of hydrogen include tritium and deuterium, and isotopes of carbon include C-13 and C-14.

### Pharmaceutical Formulations

The present disclosure also provides pharmaceutical compositions comprising at least one EZH2 inhibitor described herein in combination with at least one pharmaceutically acceptable excipient or carrier.

A "pharmaceutical composition" is a formulation containing the EZH2 inhibitors of the present disclosure in a form suitable for administration to a subject. In one embodiment, the pharmaceutical composition is in bulk or in unit dosage form. The unit dosage form is any of a variety of forms, including, for example, a capsule, an IV bag, a tablet, a single pump on an aerosol inhaler or a vial. The quantity of active ingredient (*e.g*., a formulation of the disclosed compound or salt, hydrate, solvate or isomer thereof) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, inhalational, buccal, sublingual, intrapleural, intrathecal, intranasal, and the like. Dosage forms for the topical or transdermal administration of a compound of this disclosure include powders, sprays, ointments, pastes, creams, lotions, gels, solutions, patches and inhalants. In one embodiment, the active compound is mixed under sterile conditions with a pharmaceutically acceptable carrier, and with any preservatives, buffers or propellants that are required.

As used herein, the phrase "pharmaceutically acceptable" refers to those compounds, materials, compositions, carriers, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable excipient" means an excipient that is useful in preparing a pharmaceutical composition that is generally safe, non-toxic and neither biologically nor otherwise undesirable, and includes excipient that is acceptable for veterinary use as well as human pharmaceutical use. A "pharmaceutically acceptable excipient" as used in the disclosure includes both one and more than one such excipient.

A pharmaceutical composition of the disclosure is formulated to be compatible with its intended route of administration. Examples of routes of administration include parenteral, *e.g.,* intravenous, intradermal, subcutaneous, oral (e.g., inhalation), transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

A compound or pharmaceutical composition of the disclosure can be administered to a subject in many of the well-known methods currently used for chemotherapeutic treatment. For example, for treatment of cancers, a compound of the disclosure may be injected directly into tumors, injected into the blood stream or body cavities or taken orally or applied through the skin with patches. The dose chosen should be sufficient to constitute effective treatment but not as high as to cause unacceptable side effects. The state of the disease condition (e.g., cancer, precancer, and the like) and the health of the patient should preferably be closely monitored during and for a reasonable period after treatment.

The term "therapeutically effective amount", as used herein, refers to an amount of an EZH2 inhibitor, composition, or pharmaceutical composition thereof effective to treat, ameliorate, or prevent an identified disease or condition, or to exhibit a detectable therapeutic or inhibitory effect. The effect can be detected by any assay method known in the art. The precise effective amount for a subject will depend upon the subject's body weight, size, and health; the nature and extent of the condition; and the therapeutic or combination of therapeutics selected for administration. Therapeutically effective amounts for a given situation can be determined by routine experimentation that is within the skill and judgment of the clinician. In a preferred aspect, the disease or condition to be treated is cancer, including but not limited to, medulloblastoma.

For any EZH2 inhibitor of the disclosure, the therapeutically effective amount can be estimated initially either in cell culture assays, *e.g.,* of neoplastic cells, or in animal models, usually rats, mice, rabbits, dogs, or pigs. The animal model may also be used to determine the appropriate concentration range and route of administration. Such information can then be used to determine useful doses and routes for administration in humans. Therapeutic/prophylactic efficacy and toxicity may be determined by standard pharmaceutical procedures in cell cultures or experimental animals, *e.g.,* ED₅₀ (the dose therapeutically effective in 50% of the population) and LD₅₀ (the dose lethal to 50% of the population). The dose ratio between toxic and therapeutic effects is the therapeutic index, and it can be expressed as the ratio, LD₅₀/ED₅₀. Pharmaceutical compositions that exhibit large therapeutic indices are preferred. The dosage may vary within this range depending upon the dosage form employed, sensitivity of the patient, and the route of administration.

Dosage and administration are adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

The pharmaceutical compositions containing an EZH2 inhibitor of the present disclosure may be manufactured in a manner that is generally known, *e.g*., by means of conventional mixing, dissolving, granulating, dragee-making, levigating, emulsifying, encapsulating, entrapping, or lyophilizing processes. Pharmaceutical compositions may be formulated in a conventional manner using one or more pharmaceutically acceptable carriers comprising excipients and/or auxiliaries that facilitate processing of the active compounds into preparations that can be used pharmaceutically. Of course, the appropriate formulation is dependent upon the route of administration chosen.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible pharmaceutically acceptable carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser, which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The active compounds (i.e. EZH2 inhibitors of the disclosure) can be prepared with pharmaceutically acceptable carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the disclosure are dictated by and directly dependent on the unique characteristics of the active compound and the particular therapeutic effect to be achieved.

In therapeutic applications, the dosages of the pharmaceutical compositions used in accordance with the disclosure vary depending on the agent, the age, weight, and clinical condition of the recipient patient, and the experience and judgment of the clinician or practitioner administering the therapy, among other factors affecting the selected dosage. Generally, the dose should be sufficient to result in slowing, and preferably regressing, the growth of the tumors and also preferably causing complete regression of the cancer. An effective amount of a pharmaceutical agent is that which provides an objectively identifiable improvement as noted by the clinician or other qualified observer. For example, regression of a tumor in a patient may be measured with reference to the diameter of a tumor. Decrease in the diameter of a tumor indicates regression. Regression is also indicated by failure of tumors to reoccur after treatment has stopped. As used herein, the term "dosage effective manner" refers to amount of an active compound to produce the desired biological effect in a subject or cell.

The pharmaceutical compositions can be included in a container, pack, or dispenser together with instructions for administration.

The compounds of the present disclosure are capable of further forming salts. All of these forms are also contemplated within the scope of the claimed disclosure.

As used herein, "pharmaceutically acceptable salts" refer to derivatives of the compounds of the present disclosure wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicyclic, stearic, subacetic, succinic, sulfamic, sulfanilic, sulfuric, tannic, tartaric, toluene sulfonic, and the commonly occurring amine acids, *e.g*., glycine, alanine, phenylalanine, arginine, etc.

Other examples of pharmaceutically acceptable salts include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The present disclosure also encompasses salts formed when an acidic proton present in the parent compound either is replaced by a metal ion, *e.g*., an alkali metal ion, an alkaline earth ion, or an aluminum ion; or coordinates with an organic base such as ethanolamine, diethanolamine, triethanolamine, tromethamine, N-methylglucamine, and the like.

It should be understood that all references to pharmaceutically acceptable salts include solvent addition forms (solvates) or crystal forms (polymorphs) as defined herein, of the same salt.

The EZH2 inhibitors of the present disclosure can also be prepared as esters, for example, pharmaceutically acceptable esters. For example, a carboxylic acid function group in a compound can be converted to its corresponding ester, *e.g*., a methyl, ethyl or other ester. Also, an alcohol group in a compound can be converted to its corresponding ester, *e.g*., an acetate, propionate or other ester.

The EZH2 inhibitors of the present disclosure can also be prepared as prodrugs, for example, pharmaceutically acceptable prodrugs. The terms "pro-drug" and "prodrug" are used interchangeably herein and refer to any compound which releases an active parent drug *in vivo.* Since prodrugs are known to enhance numerous desirable qualities of pharmaceuticals (*e.g.*, solubility, bioavailability, manufacturing, etc.), the compounds of the present disclosure can be delivered in prodrug form. Thus, the present disclosure is intended to cover prodrugs of the presently claimed compounds, methods of delivering the same and compositions containing the same. "Prodrugs" are intended to include any covalently bonded carriers that release an active parent drug of the present disclosure *in vivo* when such prodrug is administered to a subject. Prodrugs in the present disclosure are prepared by modifying functional groups present in the compound in such a way that the modifications are cleaved, either in routine manipulation or *in vivo,* to the parent compound. Prodrugs include compounds of the present disclosure wherein a hydroxy, amino, sulfhydryl, carboxy or carbonyl group is bonded to any group that may be cleaved *in vivo* to form a free hydroxyl, free amino, free sulfhydryl, free carboxy or free carbonyl group, respectively.

Examples of prodrugs include, but are not limited to, esters (*e.g.*, acetate, dialkylaminoacetates, formates, phosphates, sulfates and benzoate derivatives) and carbamates (*e.g.*, N,N-dimethylaminocarbonyl) of hydroxy functional groups, esters (*e.g.*, ethyl esters, morpholinoethanol esters) of carboxyl functional groups, N-acyl derivatives (*e.g.*, N-acetyl) N-Mannich bases, Schiff bases and enaminones of amino functional groups, oximes, acetals, ketals and enol esters of ketone and aldehyde functional groups in compounds of the disclosure, and the like, See Bundegaard, H., Design of Prodrugs, p1-92, Elesevier, New York-Oxford (1985).

The EZH2 inhibitors, or pharmaceutically acceptable salts, esters or prodrugs thereof, are administered orally, nasally, transdermally, pulmonary, inhalationally, buccally, sublingually, intraperintoneally, subcutaneously, intramuscularly, intravenously, rectally, intrapleurally, intrathecally and parenterally. In one embodiment, the compound is administered orally. One skilled in the art will recognize the advantages of certain routes of administration.

The dosage regimen utilizing the compounds is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter or arrest the progress of the condition.

The dosage regimen can be daily administration (*e.g.* every 24 hours) of a compound of the present disclosure. The dosage regimen can be daily administration for consecutive days, for example, at least two, at least three, at least four, at least five, at least six or at least seven consecutive days. Dosing can be more than one time daily, for example, twice, three times or four times daily (per a 24 hour period). The dosing regimen can be a daily administration followed by at least one day, at least two days, at least three days, at least four days, at least five days, or at least six days, without administration.

Techniques for formulation and administration of the disclosed compounds of the disclosure can be found in Remington: the Science and Practice of Pharmacy, 19th edition, Mack Publishing Co., Easton, PA (1995). In an embodiment, the compounds described herein, and the pharmaceutically acceptable salts thereof, are used in pharmaceutical preparations in combination with a pharmaceutically acceptable carrier or diluent. Suitable pharmaceutically acceptable carriers include inert solid fillers or diluents and sterile aqueous or organic solutions. The compounds will be present in such pharmaceutical compositions in amounts sufficient to provide the desired dosage amount in the range described herein.

All percentages and ratios used herein, unless otherwise indicated, are by weight.

Other features and advantages of the present disclosure are apparent from the different examples. The provided examples illustrate different components and methodology useful in practicing the present disclosure. The examples do not limit the claimed disclosure. Based on the present disclosure the skilled artisan can identify and employ other components and methodology useful for practicing the present disclosure.

### EXAMPLES

In order that the invention disclosed herein may be more efficiently understood, examples are provided below. It should be understood that these examples are for illustrative purposes only and are not to be construed as limiting the disclosure in any manner.

### Example 1: Tazemetostat decreases medulloblastoma cell growth.

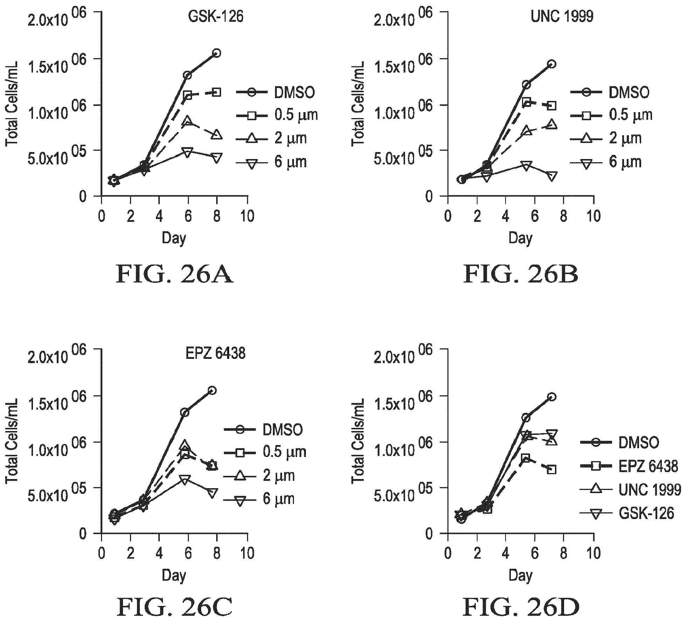

Medulloblastoma cells are treated with either a negative control (DMSO) or varying concentrations of tazemetostat (EPZ 6438): 0.5 µM, 2 µM and 6 µM. The total cells per milliliter of culture were counted each day for 10 days. While each tazemetostat treatment demonstrated a significant decrease on medulloblastoma cell growth compared to wild type (Figure 26C), the effect was concentration dependent.

When compared to the efficacy of other small molecule EZH2 inhibitors, including GSK-126 and UNC 1999, Tazemetostat demonstrated a superior ability to decrease medulloblastoma cell growth (Figure 26D).

### Example 2: Tazemetostat decreases medulloblastoma cell growth in an ex vivo slice culture.

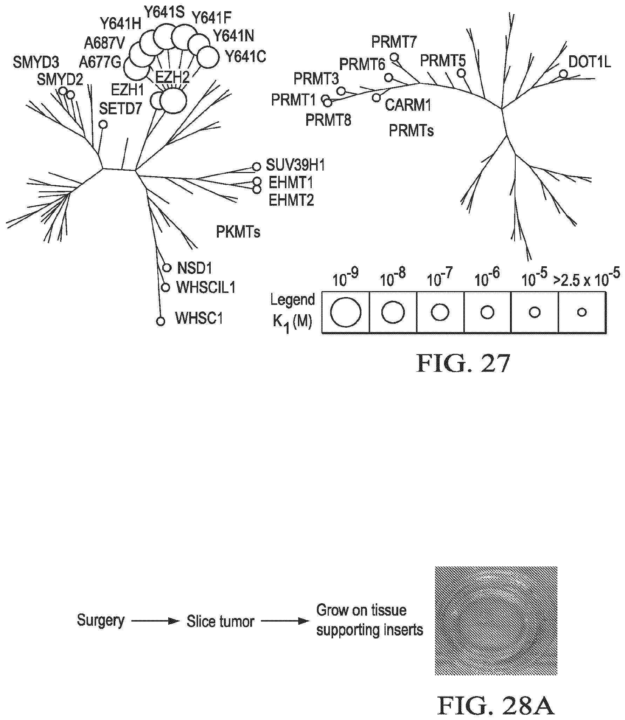

A 5 year old patient having medulloblastoma underwent surgery to remove a slice of tumor tissue for testing. The medulloblastoma slice was cultured ex vivo on tissue supporting inserts (Figure 28A). Portions of the slice culture were untreated, treated with a lower concentration of tazemetostat (500 nM) or a higher concentration of tazemetostat (2 µM) for 4 days. Following the treatment period, the cells of the slice culture were treated with BrdU for 4 hours prior to disaggregation and sorting by flow cytometry.

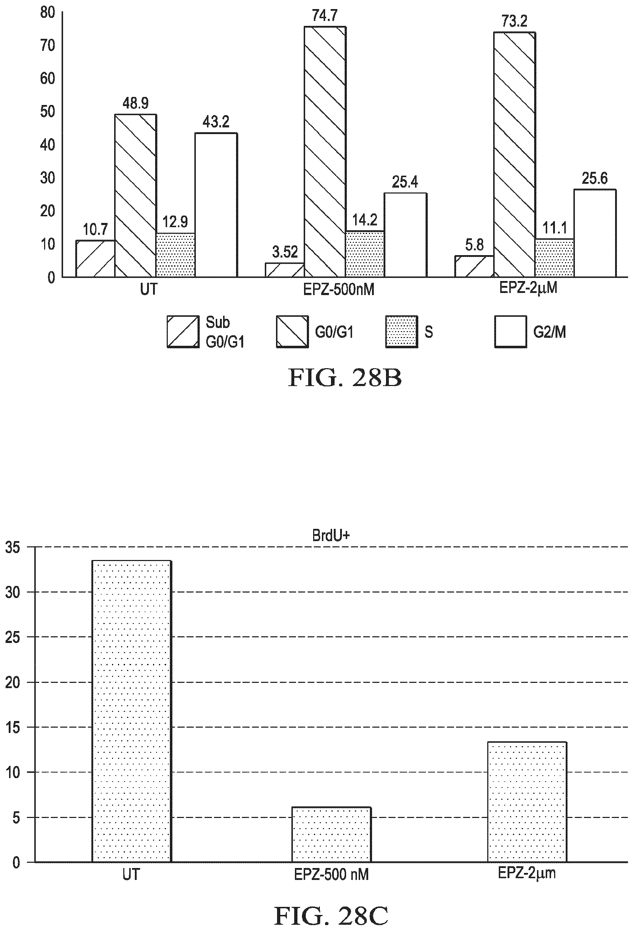

Figure 28B provides the results of the treatment by depicting the percent of cells in each of four cell cycle stages (sub G0/G1, Go/G1, S or G2/M) following each one of the treatment conditions. The data demonstrate that, compared to the untreated control, an increased proportion of medulloblastoma cells treated with tazemetostat are in the G0/G1 stage and a decreased proportion of medulloblastoma cells treated with tazemetostat are in the G2/M stage. The data indicate that treatment with tazemetostat inhibits proliferation/growth of medulloblastoma cells by interfering with cell division.

Figure 28C confirms the results of Figure 28B showing that the number of cells synthesizing DNA is significantly decreased in the tazemetostat-treated cells as evidenced by decreased incorporation of BrdU.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow. Where names of cell lines or genes are used, abbreviations and names conform to the nomenclature of the American Type Culture Collection (ATCC) or the National Center for Biotechnology Information (NCBI), unless otherwise noted or evident from the context.

The invention can be embodied in other specific forms without departing from the spirit or essential characteristics thereof. The foregoing embodiments are therefore to be considered in all respects illustrative rather than limiting on the invention described herein. Scope of the invention is thus indicated by the appended claims rather than by the foregoing description, and all changes that come within the meaning and range of equivalency of the claims are intended to be embraced therein.

**The following are clauses describing embodiments of the invention. They are not claims.**
1. A method of treating a medulloblastoma in a subject in need thereof comprising administering to the subject a therapeutically-effective amount of an enhancer of a zeste homolog 2 (EZH2) inhibitor.
2. The method of clause 1, wherein the EZH2 inhibitor comprises 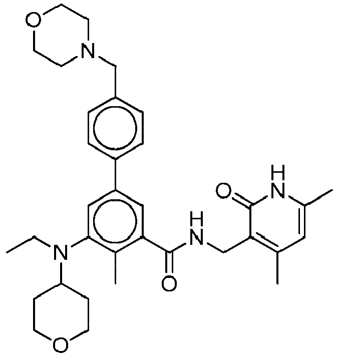 (tazemetostat), or a pharmaceutically-acceptable salt thereof.
3. The method of clause 1, wherein the EZH2 inhibitor comprises 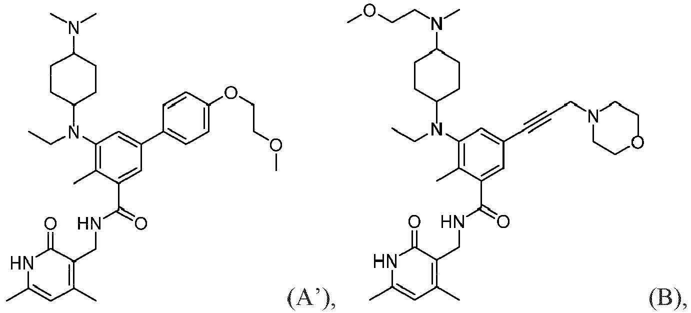 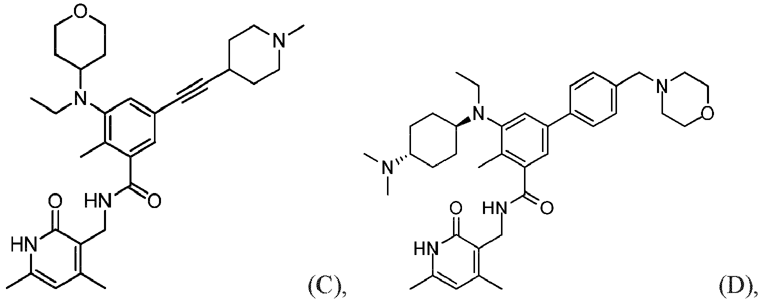 a stereoisomer, a pharmaceutically acceptable salt and/or a solvate thereof.
4. The method of clause 1, wherein the EZH2 inhibitor comprises 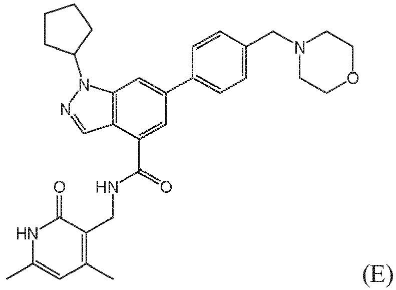 or a pharmaceutically acceptable salt thereof.
5. The method of clause 1, wherein the EZH2 inhibitor comprises 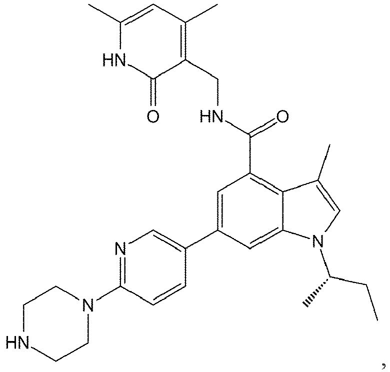 a stereoisomer, a pharmaceutically acceptable salt and/or a solvate thereof.
6. The method of clause 1, wherein the EZH2 inhibitor comprises 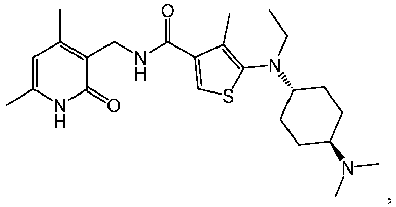 a stereoisomer, a pharmaceutically acceptable salt and/or a solvate thereof.
7. The method of clause 1, wherein the EZH2 inhibitor comprises 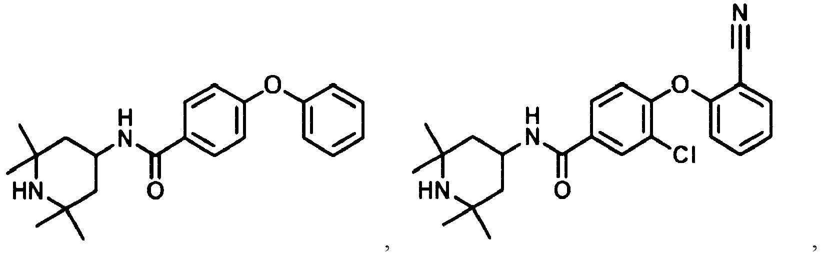 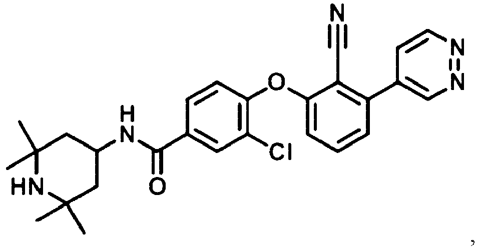 a stereoisomer, a pharmaceutically acceptable salt and/or a solvate thereof.
8. The method of any one of the foregoing clauses, wherein the EZH2 inhibitor is administered orally.
9. The method of any one of the foregoing clauses, wherein the EZH2 inhibitor is formulated as an oral tablet.
10. The method of any one of the foregoing clauses, wherein the EZH2 inhibitor is administered at a dose of between 10 mg/kg/day and 1600 mg/kg/day.
11. The method of clause 10, wherein the EZH2 inhibitor is administered at a dose of about 100, 200, 400, 800, or 1600 mg.
12. The method of clause 11, wherein the EZH2 inhibitor is administered at a dose of about 800 mg.
13. The method of any one of clauses 1-8, wherein the EZH2 inhibitor is formulated as an oral suspension.
14. The method of any one of clauses 1-8, wherein the EZH2 inhibitor is formulated for administration to cerebral spinal fluid (CSF).
15. The method of clause 14, wherein the EZH2 inhibitor is administered to cerebral spinal fluid by an intraspinal, an intracranial, an intrathecal or an intranasal route.
16. The method of any one of clauses 13-15, wherein the EZH2 inhibitor is administered at a dose of between 230 mg/m² and 600 mg/m² twice per day (BID), inclusive of the endpoints.
17. The method of clause 16, wherein the EZH2 inhibitor is administered at a dose of between 230 mg/m² and 305 mg/m² twice per day (BID), inclusive of the endpoints.
18. The method of any one of clauses 13-15, wherein the EZH2 inhibitor is administered at a dose of 240 mg/m² twice per day (BID).
19. The method of any one of clauses 13-15, wherein the EZH2 inhibitor is administered at a dose of 300 mg/m² twice per day (BID).
20. The method of any one of clauses 13-15, wherein the EZH2 inhibitor is administered at a dose of about 60% of the area under the curve (AUC) at steady state (ACUss) following administration of 1600 mg twice a day to an adult subject.
21. The method of clause 13 or 20, wherein the EZH2 inhibitor is administered at a dose of about 600 mg/m² per day.
22. The method of clause 13 or 20, wherein the EZH2 inhibitor is administered at a dose of at least 600 mg/m² per day.
23. The method of any one of clauses 13-15, wherein the EZH2 inhibitor is administered at a dose of about 80% of the area under the curve (AUC) at steady state (ACUss) following administration of 800 mg twice a day to an adult subject.
24. The method of clause 13 or 23, wherein the EZH2 inhibitor is administered at a dose of about 390 mg/m² twice per day (BID).
25. The method of clause 13 or 23, wherein the EZH2 inhibitor is administered at a dose of at least 390 mg/m² twice per day (BID).
26. The method of any one of clauses 13-15, wherein the EZH2 inhibitor is administered at a dose of between 300 mg/m² and 600 mg/m² twice per day (BID).
27. The method of any one of the foregoing clauses, wherein the EZH2 inhibitor is administered twice per day (BID).
28. The method of any one of the foregoing clauses, wherein the subject is a pediatric subj ect.
29. The method of clause 28, wherein the subject is between 6 months and 21 years of age, inclusive of the endpoints.
30. The method of clause 29, wherein the subject is between 1 year and 18 years of age, inclusive of the endpoints.
31. The method of clause 28, wherein the subject is 10 years of age or less.
32. The method of clause 28, wherein the subject is 5 years of age or less.
33. The method of any one of the foregoing clauses, wherein treating comprises preventing and/or inhibiting proliferation of a medulloblastoma cell.
34. A method of treating medulloblastoma in a subject in need thereof comprising administering to the subject a therapeutically-effective amount of tazemetostat,
   wherein the therapeutically effective amount is at least 300 mg/m² twice per day (BID), and
   wherein the subject is between 6 months and 21 years of age, inclusive of the endpoints.

## Claims

1. An enhancer of zeste homolog 2 (EZH2) inhibitor for use in treating a medulloblastoma.

2. An EZH2 inhibitor for use according to claim 1, wherein the EZH2 inhibitor comprises 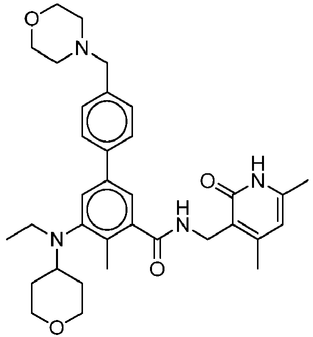 (tazemetostat), or a pharmaceutically-acceptable salt thereof;
or wherein the EZH2 inhibitor comprises 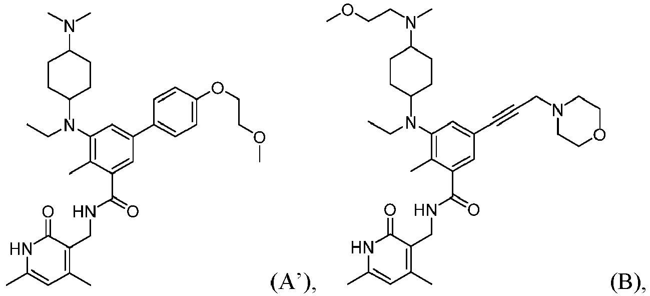 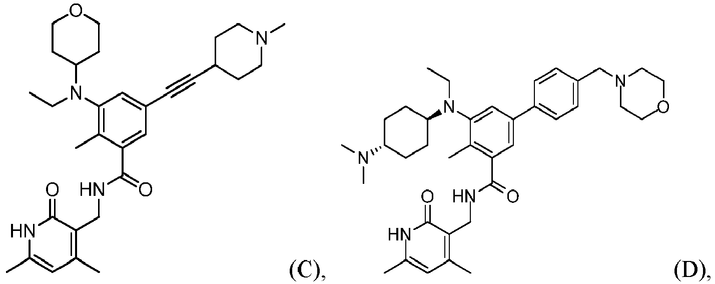
a stereoisomer, a pharmaceutically acceptable salt and/or a solvate thereof;
or wherein the EZH2 inhibitor comprises 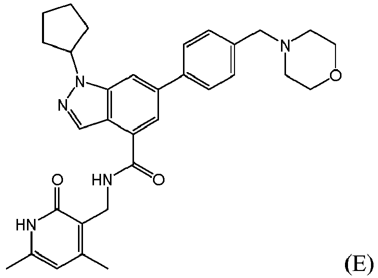
or a pharmaceutically acceptable salt thereof;
or wherein the EZH2 inhibitor comprises 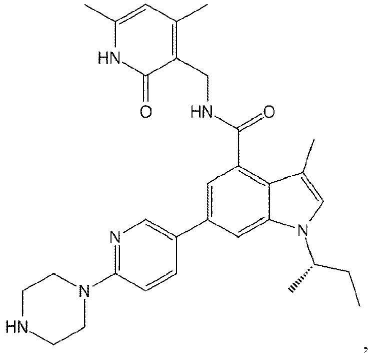
a stereoisomer, a pharmaceutically acceptable salt and/or a solvate thereof;
or wherein the EZH2 inhibitor comprises 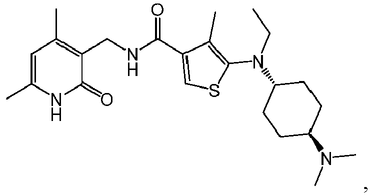
a stereoisomer, a pharmaceutically acceptable salt and/or a solvate thereof;
or wherein the EZH2 inhibitor comprises 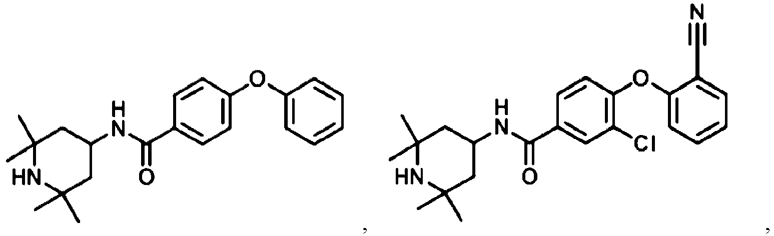 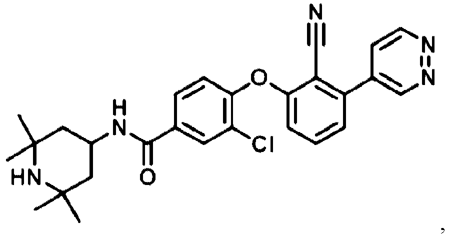
a stereoisomer, a pharmaceutically acceptable salt and/or a solvate thereof.

3. An EZH2 inhibitor for use according to claim 1, wherein the EZH2 inhibitor is administered orally; and or the EZH2 inhibitor is formulated as an oral tablet.

4. An EZH2 inhibitor for use according to any one or more of the foregoing claims, wherein the EZH2 inhibitor is administered at a dose of between 10 mg/kg/day and 1600 mg/kg/day, for example the EZH2 inhibitor is administered at a dose of about 100, 200, 400, 800, or 1600 mg, for example the EZH2 inhibitor is administered at a dose of about 800 mg.

5. An EZH2 inhibitor for use according to claim 1 or claim 2, wherein the EZH2 inhibitor is formulated as an oral suspension, or the EZH2 inhibitor is formulated for administration to cerebral spinal fluid (CSF).

6. An EZH2 inhibitor for use according to claim 5, wherein the EZH2 inhibitor is administered to cerebral spinal fluid by an intraspinal, an intracranial, an intrathecal or an intranasal route.

7. An EZH2 inhibitor for use according to claim 5 or claim 6, wherein the EZH2 inhibitor is administered at a dose of between 230 mg/m² and 600 mg/m² twice per day (BID), inclusive of the endpoints, for example wherein the EZH2 inhibitor is administered at a dose of between 230 mg/m² and 305 mg/m² twice per day (BID), inclusive of the endpoints, for example wherein the EZH2 inhibitor is administered at a dose of 240 mg/m² twice per day (BID), or at a dose of 300 mg/m² twice per day (BID).

8. An EZH2 inhibitor for use according to any one or more of claims 5 to 7, wherein the EZH2 inhibitor is administered at a dose of about 60% of the area under the curve (AUC) at steady state (ACUss) following administration of 1600 mg twice a day to an adult subject.

9. An EZH2 inhibitor for use according to claim 5 or 8, wherein the EZH2 inhibitor is administered at a dose of about 600 mg/m² per day, or at a dose of at least 600 mg/m² per day.

10. An EZH2 inhibitor for use according to claim 5 or claim 6, wherein the EZH2 inhibitor is administered at a dose of about 80% of the area under the curve (AUC) at steady state (ACUss) following administration of 800 mg twice a day to an adult subject.

11. An EZH2 inhibitor for use according to claim 5 or claim 10, wherein the EZH2 inhibitor is administered at a dose of about 390 mg/m² twice per day (BID), or is administered at a dose of at least 390 mg/m² twice per day (BID), or is administered at a dose of between 300 mg/m² and 600 mg/m² twice per day (BID).

12. An EZH2 inhibitor for use according to any one or more of the foregoing claims, wherein the EZH2 inhibitor is administered twice per day (BID).

13. An EZH2 inhibitor for use according to one or more of the foregoing claims, wherein the EZH2 inhibitor is administered to a pediatric subject, for example the pediatric subject is between 6 months and 21 years of age, inclusive of the endpoints, for example wherein the subject is between 1 year and 18 years of age, inclusive of the endpoints, for example wherein the subject is 10 years of age or less, for example wherein the subject is 5 years of age or less.

14. An EZH2 inhibitor for use according to any one or more of the foregoing claims, wherein treating comprises preventing and/or inhibiting proliferation of a medulloblastoma cell.

15. An EZH2 inhibitor for use in treating medulloblastoma in a subject in need thereof comprising administering to the subject a therapeutically-effective amount of tazemetostat,
wherein the therapeutically effective amount is at least 300 mg/m² twice per day (BID), and
wherein the subject is between 6 months and 21 years of age, inclusive of the endpoints.
